# EUROPEAN PATENT APPLICATION

(11) **EP 4 617 265 A1**
(43) Date of publication of application: **17.09.2025**
(21) Application number: 23888089.2
(22) Date of filing: 09.11.2023
(51) Int. Cl.: C07D 401/06, C07D 209/96, C07D 471/10, C07D 221/20, A61K 31/499, A61K 31/519, A61P 17/00, A61P 29/00, A61P 35/00

(54) **NITROGEN-CONTAINING SPIROCYCLIC COMPOUND, PHARMACEUTICAL COMPOSITION AND USE THEREOF**

(30) Priority: 10.11.2022 CN 202211406867
(71) Applicant: Prime Gene Therapeutics Co., Ltd., Beijing 100070 (CN)
(72) Inventor: YANG, Yanqing, Beijing 100070 (CN); ZHU, Li, Beijing 100070 (CN); LI, Yuliang, Beijing 100070 (CN); ZHANG, Hui, Beijing 100070 (CN); DU, Daniel Yunlong, Beijing 100070 (CN)
(74) Representative: Winter, Brandl - Partnerschaft mbB
(86) International application number: PCT/CN2023/130831
(87) International publication number: WO 2024/099404

(57) **Abstract**

The present application relates to a nitrogen-containing spirocyclic compound, a pharmaceutical composition and a use thereof. The nitrogen-containing spirocyclic compound has the structural formula represented by formula (I). The compound of the present invention has TRPV3 inhibitory activity and can be used as a TRPV3 antagoni st or inhibitor

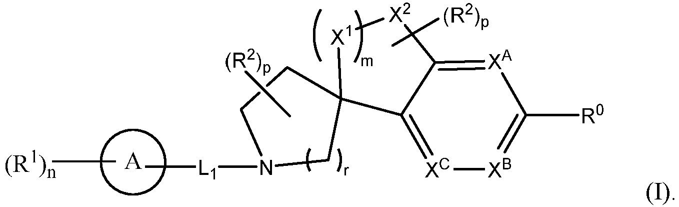

## Description

### Technical Field

The present invention relates to pharmaceutical field, and specifically, to a nitrogen-containing spirocyclic compound; a stereoisomer, a deuterated compound and a pharmaceutical composition comprising the compound; as well as use of the compound and the pharmaceutical composition.

### Background

Transient receptor potential (TRP) is a type of ion channel protein that exists on cell membrane or intracellular organelle membrane, which consists of seven subfamilies: TRPC, TRPV, TRPM, TRPML, TRPP, TRPA, TRPN, etc. Moreover, the transient receptor potential vanilloid (TRPV) subfamily of mammals is in turns composed of TRPV1-6. In recent years, research has found that TRPV3 is mainly expressed in the keratinocytes of human skin, plays an important role in mediating skin sensation, influencing the proliferation and differentiation of epidermal keratinocytes, hair growth, participating in inflammatory responses, and maintaining skin homeostasis and normal function. This ion channel can be regulated by many factors, such as temperature, osmotic pressure, pH value, mechanical force, and intracellular signaling molecules. Research has shown that TRPV3 is a pathogenic gene for Olmsted syndrome, a rare skin disease (Am. J. Hum. Genet. 2012, 90, 558), and TRPV3 inhibitors have potential therapeutic prospects in keratinizing skin diseases, pruritic skin diseases, inflammation, abnormal hair growth, and painful skin diseases. TRPV3 is mainly expressed in skin keratinocytes, as well as in tissues such as tongue, dorsal root ganglion, trigeminal ganglion, spinal cord, and brain, and mainly responds to warm stimulation (32~39°C). The thermal sensitivity of TRPV3 is also regulated by calcium in extracellular fluid. When subjected to repeated thermal stimulation, the channel current continuously increases. Free nerve endings under the skin may sense and transmit thermal stimulation through signaling molecules similar to those present in thermoreceptive neurons. Therefore, TRPV3 has potential therapeutic prospects in painful diseases.

TRPV3 can be activated by monoterpenoid compounds (e.g., camphor, borneol, peppermint, etc.) and research has found that it works by increasing the level of intracellular divalent calcium ions (Ca²⁺). These aromatic compounds have anti-inflammatory, analgesic, and anti itch effects etc., and have been widely used in fields such as medicine and cosmetics. However, these early TRPV3 inhibitors were either mostly natural products with poor specificity and high effective doses, which could lead to serious side effects, or had mediocre molecular backbone activity, and research and development were mostly stalled. The existing TRPV3 inhibitors have the problem of limited structural types and slow development. It is necessary to develop molecules with novel scaffolds and clinical use value.

### Summary of the Invention

The present invention provides a nitrogen-containing spirocyclic compound of formula (I), or stereoisomers, tautomers, solvates, hydrates, oxides, active metabolites, isotopic labels, and pharmaceutically acceptable salts thereof: 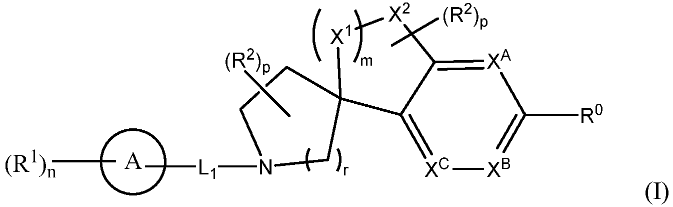
wherein ring A is selected from monocyclic or polycyclic ring systems containing 3-12 ring atoms;
R¹ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, - C(O)N(R^{a})(R^{b}) and -SO₂N(R^{a})(R^{b}), and -SOR^{c}, or two R¹, together with the atoms of ring A to which they are connected, form a 3-10 membered ring structure;
R² is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}) and -SO₂N(R^{a})(R^{b}), and -SOR^{c}, or two R², together with the atoms of the ring to which they are connected, form a 3-10 membered ring structure;
L₁ is selected from the group consisting of a bond, and the following structural formulae: 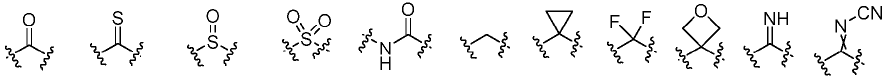 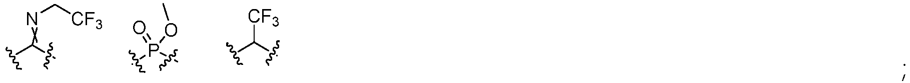
X¹ is each independently selected from the group consisting of C, O, and N at each occurrence;
X² is each independently selected from the group consisting of C, O, B, and N at each occurrence;
X^{A}, X^{B}, X^{C} are each independently CR^{x} or N;
R^{x} is each independently selected from the group consisting of H, halogen, hydroxyl, mercapto, nitro, cyano, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}) and -SO₂N(R^{a})(R^{b}), and -SOR^{c}, or two R^{x}, together with the atoms of the ring to which they are connected, form a 3-10 membered ring structure;
R⁰ is each independently selected from the group consisting of H, halogen and a structural formula of formula (II) 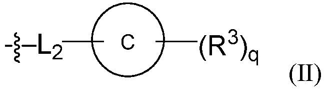
wherein,
L₂ is each independently selected from the group consisting of a bond, -O-, -S-, -N(R²⁰)-, - C(O)-, -C(R²⁰R²¹)-, -S(O)-, and -S(O₂)-;
ring C is each independently selected from monocyclic or polycyclic ring systems containing 3-12 ring atoms;
R³ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}) and -SO₂N(R^{a})(R^{b}), and -SOR^{c}, or two R³, together with the atoms of ring C to which they are connected, form a 3-10 membered ring structure;
R¹¹ is each independently selected from C₁-C₆ alkylene substituted with 0 to 2 R^{f};
R¹² is each independently selected from the group consisting of H, C₁-C₆ alkyl substituted with 0 to 2 R^{f}, C₁-C₆ haloalkyl substituted with 0 to 2 R^{f}, C₃-C₆ cycloalkyl substituted with 0 to 2 R^{f}, and C₃-C₆ halocycloalkyl substituted with 0 to 2 R^{f};
R^{a} and R^{b} are each independently selected from the group consisting of H, C₁-C₆ alkyl substituted with 0 to 2 R^{f}, aryl substituted with 0 to 2 R^{f}, aralkyl substituted with 0 to 2 R^{f}, - C(O)R^{c}, and -C(O)OR^{d};
R^{c} is each independently selected from the group consisting of H, halogen, C₁-C₆ alkyl substituted with 0 to 2 R^{f}, aryl substituted with 0 to 2 R^{f}, and aralkyl substituted with 0 to 2 R^{f},
R^{d} is each independently selected from the group consisting of H, C₁-C₆ alkyl substituted with 0 to 2 R^{f}, aryl substituted with 0 to 2 R^{f}, and aralkyl substituted with 0 to 2 R^{f},
R²⁰ and R²¹ are each independently selected from the group consisting of H, hydroxyl, C₁-C₆ alkyl, aryl, and aralkyl;
R^{f} is each independently selected from the group consisting of halogen, hydroxyl, amino, C₁-C₆ alkyl, C₁-C₆ alkoxy, C₁-C₆ haloalkyl, C₁-C₆ haloalkoxy, C₃-C₆ cycloalkyl, and C₃-C₆ halocycloalkyl;
n is 0, 1, 2 or 3;
p is 0, 1, 2 or 3;
q is 0, 1, 2 or 3;
m is 1, or 2;
r is 1, or 2.

The present invention also relates to a pharmaceutical composition, comprising the compound of the present invention, or stereoisomers, tautomers, solvates, hydrates, active metabolites, isotopic labels, or pharmaceutically acceptable salts thereof, and a pharmaceutically acceptable carrier.

The present invention also relates to use of the compound of the present invention, or stereoisomers, tautomers, solvates, hydrates, active metabolites, isotopic labels, or pharmaceutically acceptable salts thereof, and the pharmaceutical composition of the present invention in manufacture of pharmaceuticals for inhibiting TRPV3 activity.

The present invention also relates to use of the compound of the present invention, or stereoisomers, tautomers, solvates, hydrates, active metabolites, isotopic labels, or pharmaceutically acceptable salts thereof, and the pharmaceutical composition of the present invention in manufacture of pharmaceuticals for treating TRPV3-mediated conditions in a subject.

The present invention futher provides use of the above-mentioned TRPV3 inhibitors in manufacture of reagents for inhibiting TRPV3 ion channels, preferably, the use of TRPV3 inhibitors in manufacture of pharmaceuticals for inhibiting skin itching, pain, hair loss, or inflammation caused by overexpression of TRPV3.

The present invention relates to a method for treating TRPV3 mediated conditions, comprsing administering a therapeutically effective amount of the compound of the present invention, or stereoisomers, tautomers, solvates, hydrates, active metabolites, isotopic labels, or pharmaceutically acceptable salts thereof, or the pharmaceutical composition of the present invention to a patient in need of administration.

The compound of the present invention has the ability to inhibit TRPV3 activity and can be used as a TRPV3 antagonist or inhibitor, which greatly promotes the study on the properties and characteristics of TRPV3 ion channels. Meanwhile, pharmaceuticals for treating diseases associated with enhanced TRPV3 ion channel activity can also be manufactured. Therefore, the TRPV3 antagonist or inhibitor provided by the present invention has great value for scientific and clinical research.

### Detailed Description

The present application will be further explained below in detail through embodiments. Through these descriptions, the features and advantages of the present application will become more clearly defined.

The term "exemplary" used here means "serving as an example, embodiment, or illustration." Any embodiment described here as "exemplary" should not be interpreted as being superior or better than other embodiments.

Furthermore, the technical features in different embodiments of the present application described below can be combined with each other if they do not conflict.

### Definitions

The terms "antagonist" and "inhibitor", which are used interchangeably, refer to pharmaceuticals that reduce or inhibit biological activity, for example inhibit the activity of ion channels such as TRPV3.

In terms of the present invention, for example, an "effective amount" of TRPV3 antagonist relates to the amount of the antagonist in the formulation that brings a desired clinical or functional result, when administered as part of a required dosage regimen. Without being bound by theory, an effective amount of a TRPV3 antagonist used in in the present invention comprises an amount of a TRPV3 antagonist effective to decrease one or more *in vitro* or *in vivo* functions of a TRPV3 channel. Exemplary functions comprise, but are not limited to, intracellular calcium levels, membrane polarization (e.g. an antagonists may promote cell hyperpolarization), Phase I outward current, Phase II outward current, Phase I inward current, and Phase II inward current. Compounds that antagonize TRPV3 function comprise compounds that antagonize an *in vitro* or *in vivo* functional activity of TRPV3. When a particular functional activity is only readily observable in an *in vitro* assay, the ability of a compound to inhibit TRPV3 function is a reasonable alternative for *in vitro* analysis of the activity of this compound. The term "preventing" is recognized in the art, and when used for a condition such as a local recurrence (e.g., pain), a disease such as cancer, a syndrome such as heart failure or any other medical condition, is well understood in the art, and comprises administration of a composition which reduces the frequency of, or delays the onset of, a medical condition in a subject relative to a subject which does not receive the composition. Thus, prevention of cancer comprises, for example, reducing the number of detectable cancerous growths in a population of patients receiving a prophylactic treatment relative to an untreated control population, and/or delaying the appearance of detectable cancerous growth in a treated population relative to an untreated control population, e.g., by a statistically and/or clinically significant amount. Prevention of an infection comprises, for example, reducing the number of diagnoses of the infection in a treated population relative to an untreated control population, and/or delaying the onset of conditions of the infection in a treated population relative to an untreated control population. Prevention of pain comprises, for example, reducing the magnitude of, or alternatively delaying, pain sensations experienced by subjects in a treated population relative to an untreated control population.

The present invention provides compounds in prodrug form. The term "prodrug" is intended to encompass compounds that, under physiological conditions, are converted into the therapeutically active agents of the present invention. A common method for preparing a prodrug comprises revealing the selected moieties of the required molecules after hydrolyzing under physiological conditions. In other embodiments, the prodrug is converted by enzymatic activity of a host animal. Additionally, prodrugs can be converted to the compounds of the present invention by chemical or biochemical methods in an ex vivo environment. For example, prodrugs can be slowly converted to the compounds of the present invention when placed in a transdermal patch reservoir with a suitable enzyme or chemical reagent.

The term 'oxidative metabolites' is intended to encompass compounds obtained by metabolizing parent compounds under normal physiological conditions. Specifically, an oxidative metabolite is formed by the oxidation of a parent compound during metabolism. For example, the oxidation of sulfide groups can generate the corresponding sulfoxides or sulfones.

The term "solvate" as used herein relates to compounds formed by solvation (for example, compounds formed by combining solvent molecules with solute molecules or ions).

The term 'hydrate' as used herein relates to compounds formed by the combination of water with parent compounds.

The term "treating" comprises prophylactic and/or therapeutic treatments. The term "prophylactic or therapeutic" treatment is recognized in the art, and comprises administering one or more compositions of the prssent invention to a host. If it is administered prior to clinical manifestation of a unwanted disease (e.g., diseases or other unwanted states of a host animal), the treatment is prophylactic (i.e., it protects the host against developing a unwanted condition), whereas if it is administered after manifestation of a unwanted condition, the treatment is therapeutic (i.e., it is intended to diminish, ameliorate, or stabilize the existing unwanted condition or side effects thereof).

The terms "TRPV3", "TRPV3 protein", and "TRPV3 channel" are used interchangeably throughout the present application. These terms refer to an ion channel (e.g., a polypeptide) comprising amino acid sequence, for example, the amino acid sequence of a human TRPV3 protein, or an equivalent polypeptide, or a functional bioactive fragment thereof. In certain embodiments, these term refer to those comprising, consisting of, or consisting essentially of, a TRPV3 amino acid sequence set forth, for example, in any patent application cited herein. TRPV3 protein may also comprise orthologs, for example, mouse, rat, horse, or Drosophila TRPV3.

TRPV3 comprises polypeptides that retain a function of TRPV3 and comprise (i) all or a portion of a TRPV3 amino acid sequence; (ii) a TRPV3 amino acid sequence with 1 to about 2, 3, 5, 7, 10, 15, 20, 30, 50, 75 or more conservative amino acid substitutions; (iii) an amino acid sequence that is at least 70%, 75%, 80%, 85%, 90%, 95%, 96%, 97%, 98%, or 99% identical to a TRPV3 amino acid sequence; and (iv) functional fragments thereof. Polypeptides of the present invention also comprise homologs, e.g., orthologs and paralogs, of a human TRPV3 polypeptide. TRPV3 polypeptides and amino acid sequences comprise, for example, the sequences set forth in any patent application cited herein.

The term " TRPV3" further relates to a nucleic acid encoding a polypeptide of the present invention, e.g., a nucleic acid comprising a sequence consisting of, or consisting essentially of, a TRPV3 polynucleotide sequence. A nucleic acid of the present invention may comprise all, or a portion of the following nucleotide sequences: (i) a TRPV3 nucleotide sequence; (ii) a nucleotide sequence that is at least 70%, 75%, 80%, 85%, 90%, 95%, 96%, 97%, 98% or 99% identical to a TRPV3 nucleotide sequence; (iii) a nucleotide sequence that hybridizes under stringent conditions to a TRPV3 nucleotide sequence; (iv) nucleotide sequences encoding polypeptides that are functionally equivalent to polypeptides of the invention; (v) nucleotide sequences encoding polypeptides having at least about 70%, 75%, 80%, 85%, 90%, 95%, 98%, 99% or more homology or identity with a TRPV3 polypeptide sequence; (vi) nucleotide sequences encoding polypeptides having the activity of a polypeptide of the present invention and having at least about 70%, 75%, 80%, 85%, 90%, 95%, 98%, 99% or more homology or identity with a TRPV3 polypeptide sequence; (vii) nucleotide sequences that differ by 1 to about 2, 3, 5, 7, 10, 15, 20, 30, 50, 75 or more nucleotide substitutions, additions or deletions of a TRPV3 nucleotide sequence, such as allelic variants; (viii) nucleic acids derived from and evolutionarily related to a TRPV3 nucleotide sequence; and (ix) complementary sequences for all of the aforementioned nucleic acids and other nucleic acids of the present invention, and nucleotide sequences resulting from the degeneracy of the genetic code. Nucleic acids of the invention also comprise homologs, e.g., orthologs and paralogs, of a TRPV3 nucleic acid sequence, and also variants which have been codon optimized for expression in a particular organism (e.g., host cell). TRPV3 nucleic acid sequences comprise, for example, the sequences set forth in any patent application cited herein. Where not explicitly stated, one of skill in the art can readily assess whether TRPV3 relates to a nucleic acid or a protein.

Herein, the term "aliphatic group" relates to a straight-chain, straight-chain, or cyclic aliphatic hydrocarbon group and comprises saturated and unsaturated aliphatic groups, such as an alkyl, an alkenyl, and an alkynyl.

The terms "alkenyl" and "alkynyl" refer to unsaturated aliphatic groups that are similar in length to the alkyls described above and may be substituted, but contain at least one double or triple bond respectively.

The terms "alkoxy" as used herein relates to an alkyl as defined below, to which an oxygen radical is attached. Representative alkoxyls comprise methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as can be represented by one of -O-alkyl, -O-alkenyl, -O-alkynyl, -O-(CH₂)ₜ-R₈, where R₈ is selected from hydrogen, halogen, lower alkyl, lower alkoxy, amino, or-NHSO₂NH₂ with t being an integer from 0 to 6.

The term "alkyl" relates to the radical of saturated aliphatic groups, comprising straight-chain alkyls and branched-chain alkyls. In preferred embodiments, a straight chain or branched chain alkyl has 30 or less, and more preferably 20 or less, and most preferably 10 or less carbon atoms in its backbone (e.g., C₁-C₃₀ for straight chains, C₃-C₃₀ for branched chains).

The term "cycloalkyl" comprises saturated and locally unsaturated cyclic alkyls with 3 to 12 carbons, preferably 3 to 8 carbons, and more preferably 3 to 6 carbons, wherein optionally, the cycloalkyl is additionally substituted. The preferred cycloalkyl has 3 to12 carbon atoms, and more preferably 5, 6, 7, or 8 carbon atoms in its ring structure. Preferred cycloalkyls comprise, but are not limited to, cyclopropyl, cyclobutyl, cyclopentyl, cyclopentenyl, cyclohexyl, cyclohexenyl, cycloheptyl, and cyclooctyl. The term "cycloalkyl" also comprises bridged ring groups, comprising but not limited to bicyclic [2.2.2] octane, bicyclic [1.1.1] pentane, bicyclic [3.2.1] octane, and bicyclic [2.1.1] hexane.

Moreover, the term "alkyl" (or "lower alkyl") as used throughout the specification, Example s, and claims is intended to comprise both "unsubstituted alkyls" and "substituted alkyls", the latter of which relates to alkyl moieties having substituents replacing hydrogens on one or more carbons of the hydrocarbon backbone. Such substituents can comprise, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxyl, a phosphoryl, a phosphate, a phosphonate, a phosphonite, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or aromatic or heteroaromatic moiety. It will be understood by those skilled in the art that the moieties substituted on the hydrocarbon chain can themselves be substituted, if appropriate. For instance, the substituents of a substituted alkyl may comprise substituted and unsubstituted forms of amino, azido, imino, amido, phosphoryl (comprsing phosphonate and phosphonite), sulfonyl (comprsing sulfate, sulfonamido, sulfamoyl and sulfonate), and silyl, as well as ethers, alkylthios, carbonyls (comprsing ketones, aldehydes, carboxylates, and esters), -CF₃, -CN and the like. Exemplary substituted alkyls are described below. A cycloalkyl can be further substituted with alkyl, alkenyl, alkoxy, alkylthio, aminoalkyl, carbonyl-substituted alkyl, -CF₃, -CN, and the like.

Analogous substitutions can be made to alkenyl and alkynyls to produce, for example, aminoalkenyls, aminoalkynyls, amidoalkenyls, amidoalkynyls, iminoalkenyls, iminoalkynyls, thioalkenyls, thioalkynyls, carbonyl-substituted alkenyls or alkynyls.

Unless the number of carbons is otherwise specified, "lower alkyl" as used herein means an alkyl as defined above, but having 1 to 10 carbons, more preferably 1 to 6 carbon atoms in its backbone structure. Likewise, "lower alkenyl" and "lower alkynyl" have similar chain lengths. Throughout the application, preferred alkyls are lower alkyls. In preferred embodiments, a substituent known as alkyl herein is a lower alkyl.

The term "alkylthio" relates to an alkyl as defined above, to which a sulfur radical is attached. In preferred embodiments, the "alkylthio" moiety is represented by one of -S-alkyl, - S-alkenyl, -S-alkynyl, and -S-(CH₂)ₜ-R₈ wherein t and R₈ are defined as above. Representative alkylthios comprise methylthio, ethylthio, and the like.

The term "aralkyl", as used herein, relates to an alkyl substituted with an aryl (e.g., an aromatic or heteroaromatic group).

The term "aryl" as used herein comprises 5-, 6-, and 7-membered single-ring aromatic groups that may comprise 0 to 4 heteroatoms, for example, benzene, pyrrole, furan, thiophene, imidazole, oxazole, thiazole, triazole, pyrazole, pyridine, pyrazine, pyridazine and pyrimidine, and the like. Those aryls having heteroatoms in the ring structure may also be referred to as "aryl heterocycles" or "heteroaromatics." The aromatic ring can be substituted at one or more ring positions with such substituents as described above, for example, halogen, azide, alkyl, aralkyl, alkenyl, alkynyl, cycloalkyl, hydroxyl, alkoxyl, amino, nitro, sulfhydryl, imino, amido, phosphate, phosphonate, phosphonite, carbonyl, carboxyl, silyl, ether, alkylthio, sulfonyl, sulfonamido, ketone, aldehyde, ester, heterocyclyl, aromatic or heteroaromatic moieties, -CF₃, - CN, or the like. The term "aryl" also comprises polycyclic ring systems having two or more rings in which two or more carbons are common to two adjoining rings (the rings are "fused rings"), wherein at least one of the rings is aromatic, e.g., the other cyclic rings can be cycloalkyls, cycloalkenyls, cycloalkynyls, aryls and/or heterocyclyls.

The term "carbocycle" as used herein relates to an aromatic or non-aromatic ring, in which each atom of the ring is carbon.

The term "electron withdrawing group" relates to a chemical group that attracts electron density from the atoms or the atomic groups bound with electron withdrawing groups. Attracting electron density comprises attraction through induction effect or delocalization/resonance effect. Example s of electron withdrawing groups connected to aromatic rings comprise perfluoroalkyls such as trifluoromethyl, halogens, azides, carbonyl-containing groups such as acyl, cyano, and imine-containing groups.

The term "heteroatom" as used herein means an atom of any element other than carbon or hydrogen. Preferred heteroatoms are boron, nitrogen, oxygen, phosphorus, sulfur and selenium.

The terms "heterocyclyl" or "heterocyclic group" refer to 3- to 10-membered ring structures, more preferably 3- to 7-membered rings, whose ring structures comprise 1 to 4 heteroatoms. Heterocycles can also be polycycles. Heterocyclyls comprise, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring can be substituted at one or more positions with such substituents as described above, for example, halogen, alkyl, aralkyl, alkenyl, alkynyl, cycloalkyl, hydroxyl, amino, nitro, sulfhydryl, imino, amido, phosphate, phosphonate, phosphonite, carbonyl, carboxyl, silyl, ether, alkylthio, sulfonyl, ketone, aldehyde, ester, heterocyclyl, aromatic or heteroaromatic moiety, -CF₃, -CN, or the like.

The term "nitro" as used herein means -NO₂; the term "halogen" means -F, -Cl, -Br or -I; the term "sulfhydryl" means -SH; the term "hydroxyl" means -OH; and the term "sulfonyl" means -SO₂-.

The terms "polycyclyl", "polycyclic group" or "polycyclic system" refer to two or more rings (e.g., cycloalkyl, cycloalkenyl, cycloalkynyl, aryls and/or heterocyclyl), in which one, two or more carbons are common to two adjoining rings, for example, the rings are "fused rings" or " spiro ring". Rings that are joined through non-adjacent atoms are termed "bridged" rings, for example C₅-C₁₂ bridged carbon ring, comprsing but not limited to bicyclic [2.2.2] octane, bicyclic [1.1.1] pentane, bicyclic [3.2.1] octane, and bicyclic [2.1.1] hexane. Each ring in the polycycle can be substituted with such substituents as described above, for example, halogen, alkyl, aralkyl, alkenyl, alkynyl, cycloalkyl, hydroxyl, amino, nitro, sulfhydryl, imino, amido, phosphate, phosphonate, phosphonite, carbonyl, carboxyl, silyl, ether, alkylthio, sulfonyl, ketone, aldehyde, ester, heterocyclyl, aromatic or heteroaromatic moiety, -CF₃, -CN, or the like.

The term "protecting group" as used herein means temporary substituents which protect a potentially reactive functional group from undesired chemical transformations. Examples of such protecting groups comprise esters of carboxylic acids, silyl ethers of alcohols, and acetals and ketals of aldehydes and ketones, respectively. The protecting groups in chemistry field have been reviewed (Greene, T.W.; Wuts, P.G.M. Protective Groups in Organic Synthesis, 2nd ed.; Wiley: New York, 1991).

The term "substituted" as used herein is contemplated to comprise all permissible substituents of organic compounds. In a broad aspect, the permissible substituents comprise acyclic and cyclic, branched and unbranched, carbocyclic and heterocyclic, aromatic and nonaromatic substituents of organic compounds (e.g., alkyl, alkenyl, alkynyl, cycloalkyl, heterocyclyl, aryl, heteroaryl, cycloalkylalkyl, heterocyclylalkyl, aralkyl, or heteroaralkyl, wherein any substituent itself may be further substituted), as well as halogen, carbonyl (e.g., ester, carboxyl, or formyl), thiocarbonyl (e.g., thioester, thiocarboxylate, or thioformate), ketone, aldehyde, amino, acylamino, amido, amidino, cyano, nitro, azido, sulfonyl, sulfoxido, sulfate, sulfonate, sulfamoyl, sulfonamido, and phosphoryl. Illustrative substituents comprise, for example, those described above. The permissible substituents can be one or more and the same or different for appropriate organic compounds. For purposes of the present invention, a heteroatom such as nitrogen may have hydrogen substituents and/or any permissible substituents of organic compounds described herein, which satisfy the valence of the heteroatom. The present invention is not intended to be limited in any manner by the permissible substituents of organic compounds.

It will be understood that "substitution" or "substituted with" comprises the implicit proviso that such substitution is in accordance with permitted valence of the substituted atom and the substituent, and that the substitution results in a stable compound, e.g., which does not spontaneously undergo transformation such as by rearrangement, cyclization, elimination, etc. The phrase "substituted with 0 to 2 R^{f}" means that the corresponding group carry 0, 1, or 2 R^{f}.

As used herein, the definition for each expression, when it occurs more than once in any structure, is intended to represent an independent definition unless it is in the same structure.

The abbreviations Me, Et, Ph, Tf, Nf, Ts, Ms represent methyl, ethyl, phenyl, trifluoromethanesulfonyl, nonafluorobutanesulfonyl, *p*-toluenesulfonyl and methanesulfonyl, respectively. A more comprehensive list of the abbreviations utilized by an ordinary skill in the field of organic chemistry appears in the first issue of each volume of the *Journal of Organic Chemistry;* this list is typically presented in a table entitled Standard List of Abbreviations. The abbreviations contained in said list, and all abbreviations utilized by an ordinary skill in the field of organic chemistry are hereby incorporated by reference.

Certain compounds of the present invention may exist in particular geometric or stereoisomeric forms. The present invention contemplates that all such compounds, including cis- and trans-isomers, *R*- and *S*-enantiomers, diastereomers, (D)-isomers, (L)-isomers, the racemic mixtures thereof, and other mixtures thereof, fall within the scope of the present invention. Additional asymmetric carbon atoms may be present in a substituent such as an alkyl. All such isomers, as well as mixtures thereof, are intended to be comprised in the present invention.

Methods for preparing substantially isomerically pure compounds are known in the art. If, for instance, a particular enantiomer of the compound of the present invention is desired, it may be prepared by asymmetric synthesis, or by derivation with a chiral auxiliary, where the resulting diastereomeric mixture is separated and the auxiliary group is removed in order to provide the desired pure enantioniers. Alternatively, where the molecule contains a basic functional group, such as amino, or an acidic functional group, such as carboxyl, diastereomeric salts may be formed with an appropriate optically active acid or base, and then the resulting diastereomer is separated by well-known fractional crystallization or chromatographic methods in the field, and the pure enantiomer is subsequently recovered. Alternatively, enantiomerically enriched mixtures and pure enantiomeric compounds can be prepared by using synthetic intermediates that are enantiomerically pure in combination with reactions that either maintain the stereochemistry at a chiral center unchanged or result in its complete inversion. Techniques for inverting a particular stereocenter or maintaining it unchanged, and those for seprating mixtures of stereoisomers are well known in the art, and it is well within the ability of one of skill in the art to choose an appropriate method for a particular situation. See, generally, Furniss et al. (eds.), Vogel's Encyclopedia of Practical Organic Chemistry 5th Ed., Longman Scientific and Technical Ltd., Essex, 1991, pp. 809-816; and Heller, Acc. Chem. Res. 23: 128 (1990).

Contemplated equivalents of the compounds described above comprise the compounds which otherwise correspond thereto, and which have the same general properties thereof (e.g., the ability to inhibit TRPV3 activity), wherein it is ensured that one or more simple variants of substituents do not adversely affect the efficacy of the compound. In general, the compounds of the present invention are prepared by, for example, the methods illustrated in the general reaction schemes described below, or by modifications thereof, using readily available starting materials, reagents and conventional synthesis procedures. In these reactions, it is also possible to make use of variants that are already known, but are not mentioned here.

For purposes of the present invention, the chemical elements are identified in accordance with the Periodic Table of the Elements. Also for purposes of the present invention, the term "hydrocarbon" is contemplated to comprise all permissible compounds having at least one hydrogen and one carbon atom. In a broad aspect, the permissible hydrocarbons comprise acyclic and cyclic, branched and unbranched, carbocyclic and heterocyclic, aromatic and nonaromatic organic compounds which can be substituted or unsubstituted.

The compounds of the present invention may also contain unnatural proportions of atomic isotopes at one or more atoms that constitute such compounds. For example, the compounds may be radiolabeled with radioactive isotopes, such as deuterium (²H), tritium (³H), iodine-125 (¹²⁵I) or carbon-14 (¹⁴C). All isotopic variants of the compounds of the present invention, whether radioactive or not, are intended to be encompassed within the scope of the present invention. In the application, when not recited simultaneously with protium (¹H), deuterium (²H), and tritium (³H), "hydrogen" or "H" can comprise all isotopes of hydrogen, protium (¹H), deuterium (²H), and tritium (³H).

The symbol " 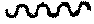 " , whether utilized as a bond or shown perpendicular to a bond, indicates the point at which the displayed moiety is attached to the remainder of the molecule, solid support, etc.

Certain compounds of the present invention can exist in unsolvated forms as well as solvated forms, comprsing hydrated forms. In general, the solvated forms are equivalent to unsolvated forms and are encompassed within the scope of the present invention. Certain compounds of the present invention may exist in polycrystalline or amorphous forms. In general, all physical forms are equivalent for the uses contemplated by the present invention and are intended to be within the scope of the present invention.

Substituent groups are specified by their conventional chemical formulae, written from left to right, and they equally encompass the chemically identical substituents, which would result from writing the structure from right to left, for example, -CH₂O- is also intended to represent -OCH₂-; -NHS(O)₂- is also intended to represent -S(O)₂HN-; etc.

The term "pharmaceutically acceptable salts" comprises salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either purely or in a suitable inert solvent. Examples of pharmaceutically acceptable base addition salts comprise sodium, potassium, calcium, ammonium, organic amino, or magnesium salts, or similar salts. When compounds of the present invention contain relatively basic functionalities, acid addition salts can be obtained by contacting the neutral form of such compounds with a sufficient amount of the desired acid, either purely or in a suitable inert solvent. Example s of pharmaceutically acceptable acid addition salts comprise those derived from inorganic acids such as hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids such as acetic, trifluoroacetic, propionic, isobutyric, maleic, malonic, benzoic, succinic, suberic, fumaric, lactic, mandelic, phthalic, benzensulfonic, p-tolylsulfonic, citric, tartaric, methanesulfonic, and the like. Also comprised are the salts of amino acids such as arginate and the like, and salts of organic acids such as glucuronic or galactunoric acids and the like (see, for example, Berge et al., "Pharmaceutical Salts", Journal of Pharmaceutical Science, 1977, 66, 1-19). Certain specific compounds of the present invention contain both basic and acidic functionalities, so that the compounds can be converted into either base or acid addition salts.

The neutral forms of the compounds are preferably regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

The term "low enough pyrogen activity", with reference to a pharmaceutical preparation, means that the content of a pyrogen in a preparation would not lead to a side effect (e.g., irritation, fever, inflammation, diarrhea, respiratory distress, endotoxic shock, etc.) in a subject to which the preparation has been administered. For example, the term encompasses preparations that are free of, or substantially free of, an endotoxin, for example, a lipopolysaccharide (LPS).

### Diseases, Disorders, or Conditions Related to TRPV3 Function

In an embodiment of the methods for preventing or treating a disease or disorder or condition, the pharmaceutical being administered is one that modulates the level and/or activity of a TRPV3 protein. In certain embodiments, the compound inhibits the expression and/or activity of a TRPV3 protein. In other embodiments, the compound selectively inhibits the expression of a TRPV3 protein. In other words, in certain embodiment, compared to one or more other ion channels, the compound preferentially inhibits the activity of a TRPV3 protein.

In particular embodiments of the methods for preventing or treating diseases and disorders provided herein, the disease or disorder can be, for example, a pain or sensitivity to touch such as pain related to a disease or disorder, e.g., cancer pain, a dermatological disease or disorder, e.g., psoriasis as well as basal cell carcinomas and squamous cell cariconomas, a neurodegenerative disease or disorder, e.g., Alzheimer's disease (AD), Parkinson's disease, Huntington's disease, amyotrophic lateral sclerosis (ALS), and other brain diseases caused by trauma or other insults including aging, an inflammatory disease (e.g., asthma, chronic obstructive pulmonary disease, rheumatoid arthritis, osteoarthritis, inflammatory bowel disease, glomerulonephritis, neuroinflammatory diseases, multiple sclerosis, and disorders of the immune system), cancer or other proliferative disease, kidney disease and liver disease, metabolic disorder such as diabetes. Further diseases and conditions comprise post-surgical pain, post herpetic neuraligia, fibromyalgia, and shingles.

Because of the important role that calcium regulation plays in many cellular processes including cellular activation, gene expression, cellular trafficking and apoptotic cell death, calcium dyshomeostasis is implicated in the many diseases and disorders involving such cellular activities. These diseases and disorders comprise dermatological diseases and disorders; neurological and neurodegenerative diseases and disorders; fever associated with various diseases, disorders or conditions; incontinence; inflammatory diseases and disorders such as inflammatory bowel disease and Crohn's disease; respiratory diseases and disorders such as chronic cough, asthma and chronic obstructive pulmonary disease (COPD); digestive disorders such as ulcers and acid reflux; metabolic diseases and disorders including obesity and diabetes; liver and kidney diseases and disorders; malignancies including cancers; aging-related disorders; and sensitivity to pain and touch.

Other diseases or conditions that can be treated comprise ATP related diseases or disorders, including epilepsy, cognition, vomiting, pain (such as migraine), asthma, peripheral vascular diseases, hypertension, immune and inflammatory conditions, irritable bowel syndrome, cystitis, depression, age-related degenerative diseases, urinary incontinence, premature ejaculation, cystic fibrosis, diabetes, birth control and infertility, and wound healing (for example, see Foresta et al. (1992) J. Biol. Chem. 257: 19443-19447; Wang et al. (1990)Biochim.Biophys.Res. Commun. 166: 251-258; Burnstock and Williams, (2000)J.Pharmacol.Exp.Ther.295: 862-869; and Burnstock, Pharmacol Rev(2006)58: 58-86).

The TRPV3 inhibitors described herein can be used to treat any of the aforementioned and subsequent diseases or conditions, including the treatment of pain associated with any of the aforementioned or subsequent diseases or conditions. When used in treatment methods, inhibitors can be selected and formulated based on predetermined routes of administration.

Compositions and methods provided herein can be used in the prevention or treatment of pain or sensitivity to pain and touch. Pain or sensitivity to pain and touch may be indicated in a variety of diseases, disorders or conditions, comprising, but not limited to, diabetic neuropathy, breast pain, psoriasis, eczema, dermatitis, bum, post-herpetic neuralgia (shingles), nociceptive pain, peripheral neuropathic and central neuropathic pain, chronic pain, cancer and tumor pain, spinal cord injury, crush injury and trauma induced pain, migraine, cerebrovascular and vascular pain, Sickle cell disease pain, rheumatoid arthritis pain, musculoskeletal pain including treating signs and symptoms of osteoarthritis and rheumatoid arthritis, orofacial and facial pain including dental- and cancer-related lower back or pelvic pain, surgical incision related pain, inflammatory and non-inflammatory pain, visceral pain, psychogenic pain and soft tissue inflammatory pain, fibromyalgia-related pain, and reflex sympathetic dystrophy. The compounds and methods of the present invention can be used in the treatment of chronic as well as acute pain. Chronic or acute pain may be the result of injury, age, or disease.

Other ion channels have been implicated in reception or transmission of pain. For example, the involvement of N-type calcium channels in the synaptic transmissions that convey pain signals from sensory afferent nerve cells to the central nervous system has been recognized. Certain naturally occurring peptide neurotoxins that specifically block N-type calcium channel have been shown to act as extremely potent and efficient analgesics in a wide range of animal pain models, which comprise models of inflammatory and neuropathic pain. The available evidence suggests that N-type calcium channel blockers are at least as effective as opiates, are devoid of a number of the typical opiate side effects (e.g. respiratory depression) and that the analgesic effect is not subject to tolerance development.

TRPV3, as well as TRPV1 and TRPV4, are expressed in a pattern consistent with involvement in pain. TRPV3 is expressed in pain sensitive neurons, and this expression is upregulated following injury. In addition, TRPV3 is robustly expressed in skin. Accordingly, methods for treating pain comprise administration of (i) antagonists of a TRPV3 function; (ii) combinations of selective antagonists of a TRPV3 and TRPV1 and/or TRPV4 function; or (iii) a pan-TRP inhibitor that inhibits functions of TRPV3, TRPV1, and TRPV4.

In addition to TRPV family members, other TRP channels have been implicated in pain reception and/or sensation. For example, certain TRPM channels including TRPM8 have been implicated in the reception and/or sensation of pain. Accordingly, in certain embodiments, the methods of the present invention comprise treating pain by administering (i) a combination of a selective TRPV3 antagonist and a selective TRPM8 antagonist; (ii) a combination of a selective TRPV3 antagonist, a selective TRPM8 antagonist, and one or more of a selective TRPV1 and/or TRPV4 antagonist; (iii) a cross-TRP inhibitor that antagonizes a function of TRPV3 and TRPM8; or (iv) a pan inhibitor that antagonizes a function of TRPV3, TRPM8, and one or more of TRPV1 and TRPV4.

Influx of calcium across plasma membrane of skin cells is a critical signaling element involved in cellular differentiation in the skin epidermis (Dotto, 1999 Crit Rev Oral Biol Med 10:442-457). Regulating or modulating the calcium entry pathway, and thus a critical control point for skin cell growth, can treat or prevent skin diseases or disorders that are characterized by epidermal hyperplasia, i.e., a condition in which skin cells both proliferate too rapidly and differentiate poorly. Such diseases comprise psoriasis, basal cell carcinomas and squamous cell carcinomas. Psoriasis, estimated to affect up to 7 million Americans, afflicts sufferers with mild to extreme discomfort, enhanced susceptibility to secondary infections, and psychological impact due to disfigurement of the affected areas (Lebwohl and Ali, 2001J Am Acad Dermatol 45:487-498). Basal cell carcinomas (BCC) and squamous cell carcinomas (SCC) of the skin represent at least one-third of all cancers diagnosed in the United States each year. More than 1 million new cases are reported annually and incidence is increasing. Despite being relatively non-aggressive, slow-growing cancers, BCCs can result in significant local tissue destruction and disfigurement. SCCs are more aggressive and thus present even greater complications. Moreover, given that 80% of lesions are on the head and neck with another 15% on shoulders, back or chest, BCCs and SCCs of the skin can have a significant impact on the appearance and quality of life of the afflicted patient.

Many dermatological disorders are accompanied by itch (pruritus). Pruritus and pain share many mechanistic similarities. Both are associated with activation of C-fibers, both are potentiated by increases in temperature and inflammatory mediators and both can be quelled with opiates. Decreasing neuronal excitability, particularly C-fiber excitability can alleviate pruritus associated with dialysis, dermatitis, pregnancy, poison ivy, allergy, dry skin, chemotherapy and eczema.

Acne is a dermatological disorder of complex etiology. Among other factors, secretion of oils from the sebaceous glands contributes to the development of acne. Since TRPV3 is also expressed in the sebaceous gland and has been shown to be able to regulate secretion in other skin cells, antagonizing TRPV3 function may reduce the signs and symptoms of acne.

In certain preferred embodiments, the TRPV3 antagonist is administered to prevent, treat, or improve signs and symptoms of acute pain, chronic pain, touch sensitivity, itch sensitivity, or as part of the treatment of burns, such as post-surgical pain, cancer pain, or neuropathic pain.

In certain preferred embodiments, the TRPV3 antagonist is administered to prevent, treat, or improve signs and symptoms of migraine.

In certain preferred embodiments, the TRPV3 antagonist is administered to prevent, treat, or improve signs and symptoms of disorders or conditions which are selected from diabetic neuropathy, inflammation, psoriasis, eczema, dermatitis, post-herpetic neuralgia (shingles), incontinence, bladder incontinence, fever, hot flashes, and cough.

In certain preferred embodiments, the TRPV3 antagonist is administered to prevent, treat, or improve signs and symptoms of osteoarthritis.

In certain preferred embodiments, the TRPV3 antagonist is administered to prevent, treat, or improve signs and symptoms of rheumatoid arthritis.

In certain preferred embodiments, the TRPV3 antagonist is administered to prevent, treat, or improve signs and symptoms of Oral Mucositis.

In certain preferred embodiments, the TRPV3 antagonist is administered to promote hair loss or inhibit hair growth in patients.

Another aspect of the present invention relates to the use of TRPV3 antagonists in manufacture of pharmaceuticals that prevent, treat, or improve symptoms of diseases, disorders or conditions in patients, wherein the symptoms involve the activation of TRPV3, or for the symptoms, a reduced TRPV3 activity can reduce severity.

The present invention relates to a method for treating TRPV3 mediated conditions, comprising administering a therapeutically effective amount of the compound of the present invention, or stereoisomers, tautomers, solvates, hydrates, active metabolites, isotopic labels, or pharmaceutically acceptable salts thereof, or the pharmaceutical composition of the present invention to a patient in need of administration.

### Pharmaceutical Composition

While the compound of the present invention can be administered alone, it is preferable to administer the compound as a pharmaceutical formulation (composition). The compounds according to the invention can be formulated for administration in any convenient way for medicinal use in human or veterinary. In certain embodiments, the compound comprised in the pharmaceutical formulation can be active itself, or can be a prodrug which, for example, is capable of being converted to an active compound in a physiological setting.

Regardless of the route of administration selected, the compounds of the present invention, which is used in a suitable hydrated form, and/or the pharmaceutical compositions of the present invention, may be formulated into the following pharmaceutically acceptable dosage forms by other conventional methods known to those of skill in the art.

Thus, another aspect of the present invention provides a pharmaceutically acceptable composition comprising a therapeutically effective amount of one or more compounds described above, formulated together with one or more pharmaceutically acceptable carriers (additives) and/or diluents into a formulation. As described in detail below, the pharmaceutical composition of the present invention can be specially formulated for administration in solid or liquid form, comprsing those adapted for the following: (1) oral administration, for example, drenches (aqueous or non-aqueous solutions or suspensions); tablets; boluses; powders; granules; pastes for application to the tongue, teeth, lips, gums; mouth washes; gels; (2) parenteral administration, for example, as a sterile solution or suspension, by subcutaneous, intramuscular or intravenous injection; (3) topical application, for example, as a cream, ointment or spray applied to skin; (4) intravaginally or intrarectally, for example, as a vaginal suppository, cream or foam; or (5) for inhalation. However, in certain embodiments, the compounds of the present invention can be easily dissolved or suspended in sterile water. In certain embodiments, the pharmaceutical formulation is non-pyrogenic, i.e., does not elevate the body temperature of a patient.

The TRPV3 antagonist can be administered alone or in combination with other therapeutic agents. For example, the TRPV3 antagonist is administered in combination with one or more of the following therapeutic agents: anti-inflammatory agents, anti-acne agents, anti-wrinkle agents, anti-scarring agent, anti-psoriatic agents, anti-proliferative agents, anti-fungal agents, anti-viral agents, preservatives, anti-migraine agents, keratolytic agents, or hair growth inhibitors.

The TRPV3 antagonist can be administered locally, orally, percutaneously, rectally, vaginally, parenterally, intranasally, intraocularly, intravenously, intramuscularly, intraarterially, intrathecally, intracapsularly, intraorbitally, intracardiacly, intradermally, intraperitoneally, transtracheally, subcutaneously, subcuticularly, intraarticularly, subcapsularly, subarachnoidly, intraspinally, and intrasternally, or by inhalation.

In certain preferred embodiments, the TRPV3 antagonist is administered locally.

In certain preferred embodiments, the TRPV3 antagonist is administered orally.

In certain preferred embodiments, the TRPV3 antagonist is administered parenterally.

The term "therapeutically effective amount" as used herein means that amount of a compound, material, or composition comprising a compound of the present invention which is effective for producing some desired therapeutic effect by inhibiting TRPV3 function in at least a sub-population of animal cells and thereby blocking the biological consequences of that function in the treated cells, at a reasonable benefit/rish ratio applicable to any medical treatment.

The terms "systemic administration," "administered systemically," "peripheral administration" and "administered peripherally" as used herein mean that a compound, pharmaceutical or other material are not administered directly into the central nervous system, so that it enters the patient's system, and thus, is subject to metabolism and other processes, for example, subcutaneous administration.

The term "pharmaceutically acceptable" is employed herein to refer to those compounds, materials, compositions, and/or dosage forms which are, within the reasonable scope of medical evaluation, suitable for use in contact with the tissues of human beings and animals without excessive toxicity, irritation, allergic response, or other problem or complication, and commensurate with a reasonable benefit/risk ratio.

The term "pharmaceutically acceptable carrier" as used herein means a pharmaceutically acceptable material, composition or vehicle, such as a liquid or solid filler, diluent, excipient, solvent or encapsulating material, which participate in carrying or transporting the antagonist of the present invention from one organ or part of the body to another. Each carrier must be "acceptable" in the sense of being compatible with the other ingredients of the formulation and not injurious to the patient. Some Example s of materials which can serve as pharmaceutically acceptable carriers comprise: (1) sugars, such as lactose, glucose and sucrose; (2) starches, such as corn starch and potato starch; (3) cellulose and derivatives thereof, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethanol; (20) phosphate buffer solutions; and (21) other non-toxic compatible substances employed in pharmaceutical formulations.

As mentioned above, certain embodiments of the present compounds can contain a basic functional group, such as amino or alkylamino, and thus, are capable of forming pharmaceutically acceptable salts with pharmaceutically acceptable acids. In this respect, the term "pharmaceutically acceptable salts" relates to the relatively non-toxic, inorganic and organic acid addition salts of the compounds of the present invention. These salts can be prepared *in situ* during the final separation and purification of the compounds of the invention, or by separately reacting the purified compound of the present invention in its free base form with a suitable organic or inorganic acid, and seprating the salt thus formed. Representative salts comprise the hydrobronide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like (See, for example, Berge et al. (1977) "Pharmaceutical Salts", J. Pharm. Sci. 66:1-19).

The pharmaceutically acceptable salts of the compounds of the present invention comprise the conventional nontoxic salts or quaternary ammonium salts of the compounds, e.g., from non-toxic organic or inorganic acids. For example, such conventional nontoxic salts comprise those derived from inorganic acids such as hydrochloride, hydrobromic, sulfuric, sulfamic, phosphoric, nitric, and the like; and the salts prepared from organic acids such as acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

In other cases, the compounds of the present invention can contain one or more acidic functional groups, and thus, are capable of forming pharmaceutically acceptable salts with pharmaceutically acceptable bases. The term "pharmaceutically acceptable salts" in these instances relates to the relatively non-toxic, inorganic and organic base addition salts of the compounds of the present invention. These salts can likewise be prepared *in situ* during the final separation and purification of the compounds, or by separately reacting the purified compound in its free acid form with a suitable base, such as the hydroxide, carbonate or bicarbonate of a pharmaceutically acceptable metal cation; with ammonia; or with a pharmaceutically acceptable organic primary, secondary or tertiary amine. Representative alkali or alkaline earth salts comprise lithium, sodium, potassium, calcium, magnesium, and aluminum salts and the like. Representative organic amines useful for the formation of base addition salts comprise ethylamine, diethylamine, ethylenediamine, ethanolamine, diethanolamine, piperazine and the like (see for example, Berge et al., as above).

Example s of pharmaceutically acceptable antioxidants comprise: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention comprise those suitable for oral, nasal, local (including buccal and sublingual), rectal, vaginal and/or parenteral administration. Oral formulations comprise those delivered to and maintained in the mouth without swallowing, as well as formulations that are swallowed as part of or following use. The formulations can conveniently be presented in unit dosage form and can be prepared by any methods well known in the art of pharmacy. The amount of active ingredient which can be combined with a carrier material to produce a single dosage form will vary depending upon the host being treated, the particular mode of administration. The amount of active ingredient that can be combined with a carrier material to produce a single dosage form will generally be that amount of the compound which produces a therapeutic effect. Generally, based on 100%, this amount will range from about 1% to about 99% of active ingredient, preferably from about 5% to about 70%, most preferably from about 10% to about 30%.

Methods of preparing these formulations or compositions comprise the step of combining a compound of the present invention with the carrier and optionally one or more accessory ingredients. In general, the formulations are prepared by uniformly and intimately combining a compound of the present invention with liquid carriers, or finely divided solid carriers, or both, and then, if necessary, shaping the product.

Formulations of the present invention suitable for oral administration can be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of the compound of the present invention as active ingredient. The compound of the present invention can also be administered as a bolus, electuary or paste.

The present invention provides a nitrogen-containing spirocyclic compound of formula (I), or stereoisomers, tautomers, solvates, hydrates, oxides, active metabolites, isotopic labels, and pharmaceutically acceptable salts thereof: 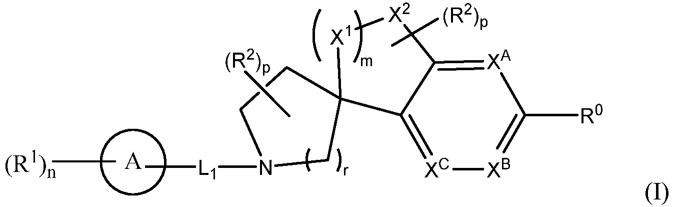
wherein ring A is selected from monocyclic or polycyclic ring systems containing 3-12 ring atoms;
R¹ is each independently selected from the group consisting of H, halogen, hydroxyl, mercapto, nitro, cyano, oxo, C₁-C₆ alkyl substituted with 1 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, - C(O)N(R^{a})(R^{b}) and -SO₂N(R^{a})(R^{b}), and -SOR^{c}, or two R¹, together with the atoms of ring A to which they are connected, form a 3-10 membered ring structure;
R² is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}) and -SO₂N(R^{a})(R^{b}), and -SOR^{c}, or two R², together with the atoms of the ring to which they are connected, form a 3-10 membered ring structure;
L₁ is selected from the group consisting of a bond, and the following structural formulae: 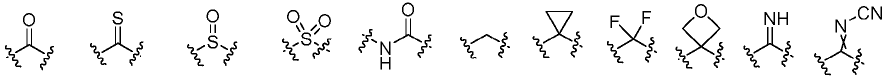 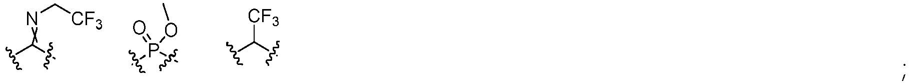
X¹ is each independently selected from the group consisting of C, O, and N at each occurrence;
X² is each independently selected from the group consisting of C, O, B, and N at each occurrence;
X^{A}, X^{B}, X^{C} are each independently CR^{x} or N;
R^{x} is each independently selected from the group consisting of H, halogen, hydroxyl, mercapto, nitro, cyano, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}) and -SO₂N(R^{a})(R^{b}), and -SOR^{c}, or two R^{x}, together with the atoms of the ring to which they are connected, form a 3-10 membered ring structure;
R⁰ is each independently selected from the group consisting of H, halogen or a structural formula of formula (II) 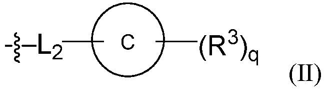
wherein,
L₂ is each independently selected from the group consisting of a bond, -O-, -S-, -N(R²⁰)-, - C(O)-, -C(R²⁰R²¹)-, -S(O)-, and -S(O₂)-;
ring C is each independently selected from monocyclic or polycyclic ring systems containing 3-12 ring atoms;
R³ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}) and -SO₂N(R^{a})(R^{b}), and -SOR^{c}, or two R³, together with the atoms of ring C to which they are connected, form a 3-10 membered ring structure;
R¹¹ is each independently selected from C₁-C₆ alkylene substituted with 0 to 2 R^{f};
R¹² is each independently selected from the group consisting of H, C₁-C₆ alkyl substituted with 0 to 2 R^{f}, C₁-C₆ haloalkyl substituted with 0 to 2 R^{f}, C₃-C₆ cycloalkyl substituted with 0 to 2 R^{f}, and C₃-C₆ halocycloalkyl substituted with 0 to 2 R^{f};
R^{a} and R^{b} are each independently selected from the group consisting of H, C₁-C₆ alkyl substituted with 0 to 2 R^{f}, aryl substituted with 0 to 2 R^{f}, aralkyl substituted with 0 to 2 R^{f}, - C(O)R^{c}, and -C(O)OR^{d};
R^{c} is each independently selected from the group consisting of H, halogen, C₁-C₆ alkyl substituted with 0 to 2 R^{f}, aryl substituted with 0 to 2 R^{f}, and aralkyl substituted with 0 to 2 R^{f};
R^{d} is each independently selected from the group consisting of H, C₁-C₆ alkyl substituted with 0 to 2 R^{f}, aryl substituted with 0 to 2 R^{f}, and aralkyl substituted with 0 to 2 R^{f};
R²⁰ and R²¹ are each independently selected from the group consisting of H, hydroxyl, C₁-C₆ alkyl, aryl, and aralkyl;
R^{f} is each independently selected from the group consisting of halogen, hydroxyl, amino, C₁-C₆ alkyl, C₁-C₆ alkoxy, C₁-C₆ haloalkyl, C₁-C₆ haloalkoxy, C₃-C₆ cycloalkyl, and C₃-C₆ halocycloalkyl;
n is 0, 1, 2 or 3;
p is 0, 1, 2 or 3;
q is 0, 1, 2 or 3;
m is 1, or 2;
r is 1, or 2.

In one embodiment, ring A is selected from the group consisting of benzene ring, pyridine ring, quinoline ring, piperidine ring, C₃-C₆ cycloalkyl ring, isoquinoline ring, pyrazine ring, pyrimidine ring, pyridazine ring, thiazole ring, thiophene ring, pyrrole ring, pyrazole ring, imidazole ring, isothiazole ring, indole ring, benzimidazole ring, furan ring, oxazole ring, oxadiazole ring, quinoxaline ring, and purine ring.

In one embodiment, ring A is selected from the following structural formulae:

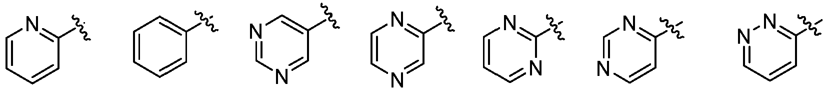

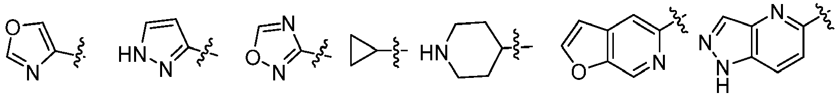

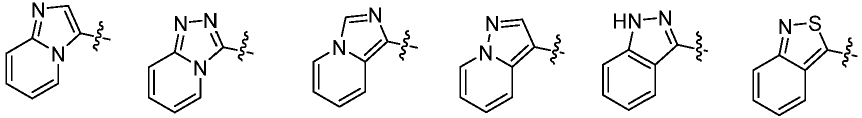

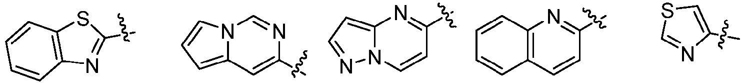

In one embodiment, R¹ is each independently selected from the group consisting of H, halogen, cyano, hydroxyl, C₁-C₆ alkyl, C₁-C₆ haloalkyl, C₁-C₆ alkoxy, -N(R^{a})(R^{b}) and -R¹¹OR¹², wherein R^{a} and R^{b} are each independently selected from the group consisting of H and C₁-C₆ alkyl; R¹¹ is each independently selected from C₁-C₆ alkylene; R¹² is each independently selected from the group consisting of H, and C₁-C₆ alkyl.

In one embodiment, R¹ is each independently selected from the group consisting of H, Cl, F, -CF₃, -CN, -CH₃, -OH, -OCH₃, and -CH₂OCH₃.

In one embodiment, R² is each independently selected from the group consisting of H, cyano, hydroxyl, mercapto, oxo, C₁-C₆ alkyl, C₁-C₆ haloalkyl, C₃-C₆ cycloalkyl substituted with 0 to 2 R^{f}, C₁-C₆ alkoxy, C₁-C₆ haloalkoxy, -R¹¹OR¹², -R¹¹SR¹², -CH(O), -C(O)OR^{d} and - C(O)N(R^{a})(R^{b}); wherein R¹¹ is each independently selected from C₁-C₆ alkylene; R¹² is each independently selected from the group consisting of H, C₁-C₆ alkyl, C₁-C₆ haloalkyl, C₃-C₆ cycloalkyl, and C₃-C₆ halocycloalkyl; R^{a} and R^{b} are each independently selected from the group consisting of H and C₁-C₆ alkyl; R^{d} is each independently selected from the group consisting of H and C₁-C₆ alkyl; R^{f} is each independently selected from the group consisting of halogen, hydroxyl, amino, and C₁-C₆ alkyl.

In one embodiment, R² is each independently selected from the group consisting of protium, deuterium, tritium, -SH, -OH, -OCF₃, -CH₃, -NH₂, -CN, -CONH₂, -CH₂OH, -CH(O), -CHF₂, - COOH, -COOCH₃, oxo, and

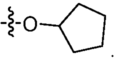

In one embodiment, L₂ is each independently selected from the group consisting of a bond, -O-, -S-, -N-, -C(O)-, -CH₂-, -CF₂-, -C(OH)-, -S(O)-, and -S(O₂)-.

In one embodiment, ring C is selected from the group consisting of C₃-C₆ cycloalkane ring, benzene ring, benzo-C₃-C₆ cycloalkane rings, pyridine ring, quinoline ring, isoquinoline ring, pyrazine ring, pyrimidine ring, pyridazine ring, thiazole ring, thiophene ring, pyrrole ring, pyrazole ring, imidazole ring, isothiazole ring, indole ring, benzimidazole ring, furan ring, oxazole ring, quinoxaline ring, and purine ring.

In one embodiment, ring C is selected from the following structural formulae:

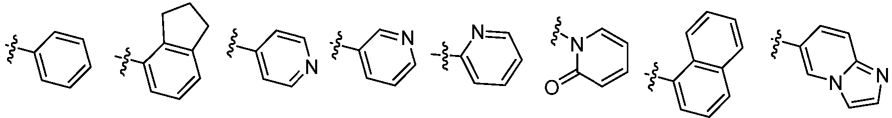

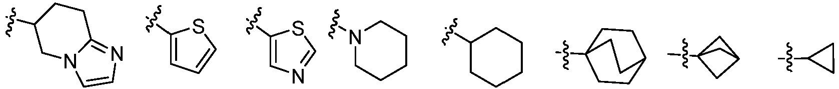

In one embodiment, R³ is each independently selected from the group consisting of H, cyano, hydroxyl, halogen, C₁-C₆ alkyl, C₁-C₆ haloalkyl, C₁-C₆ alkoxy, C₁-C₆ haloalkoxy, C₃-C₆ cycloalkyl, -R¹¹OR¹², -R¹¹SR¹², -C(O)R^{c}, -C(O)OR^{d}, and -C(O)N(R^{a})(R^{b}), or two R³, together with the atoms of ring C to which they are connected, form a 3-10 membered ring structure;
wherein R¹¹ is each independently selected from C₁-C₆ alkylene; R¹² is each independently selected from the group consisting of H, C₁-C₆ alkyl, C₁-C₆ haloalkyl, C₃-C₆ cycloalkyl, and C₃-C₆ halocycloalkyl; R^{a} and R^{b} are each independently selected from the group consisting of H and C₁-C₆ alkyl; R^{c} is independently selected from the group consisting of H, halogen, and C₁-C₆ alkyl; R^{d} is each independently selected from the group consisting of H and C₁-C₆ alkyl.

In one embodiment, R³ is each independently selected from the group consisting of H, cyclopropyl, isopropyl, tert-butyl, F, Cl, CN, ethyl, methyl, trifluoromethoxy, methylcarbonyl, methoxymethyl, and -C(CH₃)₂OH.

In one embodiment, 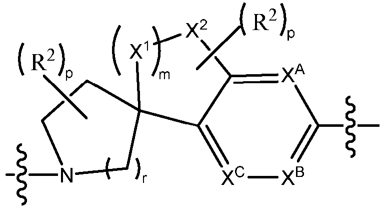 in formula (I) is selected from one of the following structural formulae: 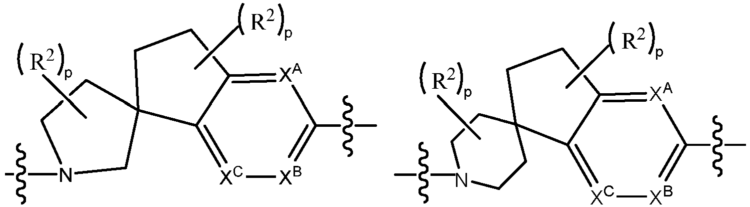 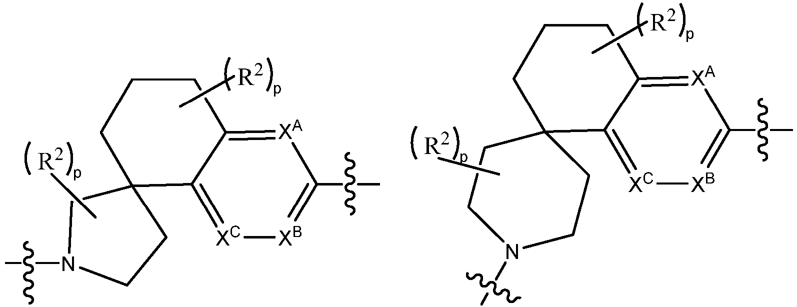 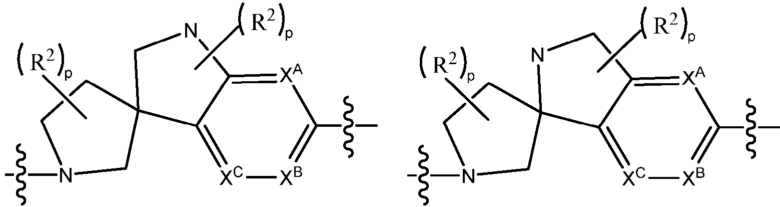 or, 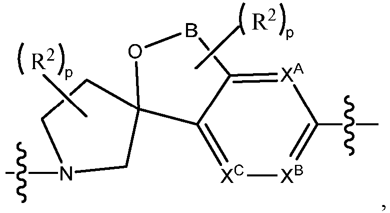 wherein R², X^{A}, X^{B}, X^{C}, and p are defined as in formula (I).

In one embodiment, 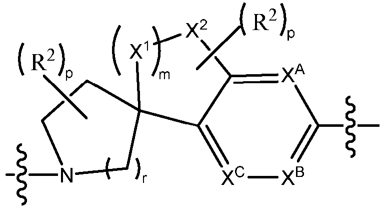 in formula (I) is selected from one of the following structural formulae: 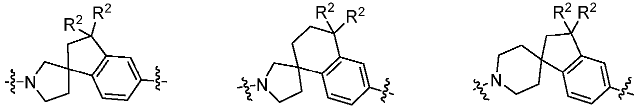 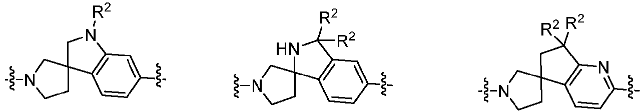 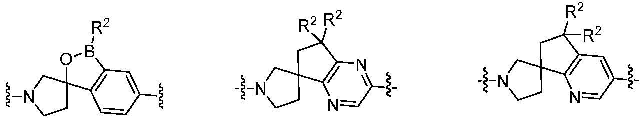 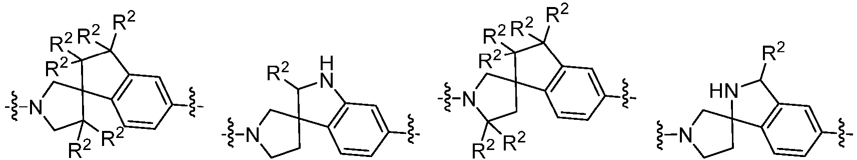 wherein R² is each independently selected from the group consisting of protium, deuterium, tritium, halogen, oxo, -SH, -OH, -CH₃, -CN, -CONH₂, -COOH, -COH, -COOCH₃, -CF₃, -OCH₃, -CH₂OH, -OCF₃, -CHF₂, and -NH₂.

In these structural formulae, p of R² groups can still be optionally substituted in one or two ring parts of the spirocyclic structure, respectively. The definition of R² group can be as described above. In one embodiment, at least one R² is - OH.

In one embodiment, 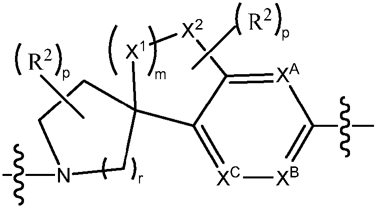 in formula (I) is selected from one of the following structural formulae: 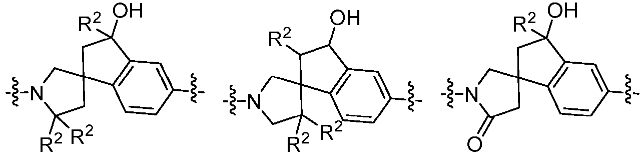 wherein R² is each independently selected from the group consisting of protium, deuterium, tritium, halogen, -CH₃ and -CF₃.

In one embodiment, 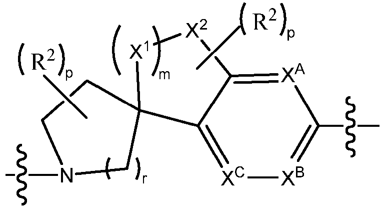 in formula (I) is selected from one of the following structural formulae: 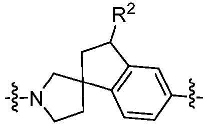 wherein R² is independently selected from the group consisting of oxo, -SH, -CN, -CONH₂, -COOH, -COH, -COOCH₃, -OCH₃, -CH₂OH, -CHF₂, -OCF₃ and -NH₂.

In one embodiment, 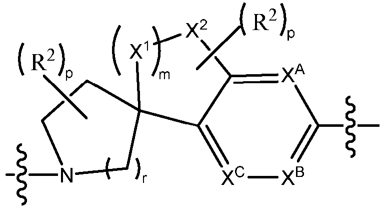 in formula (I) is selected from one of the following structural formulae:

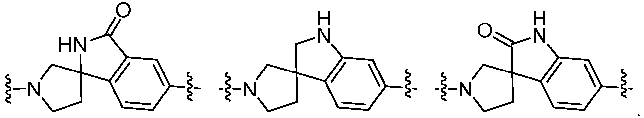

In one embodiment, L₁ is

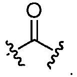

In one embodiment, ring A is 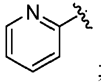 ; or
ring A is 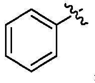 ; or
ring A is

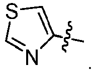

In one embodiment, the compound is selected from the following compounds:

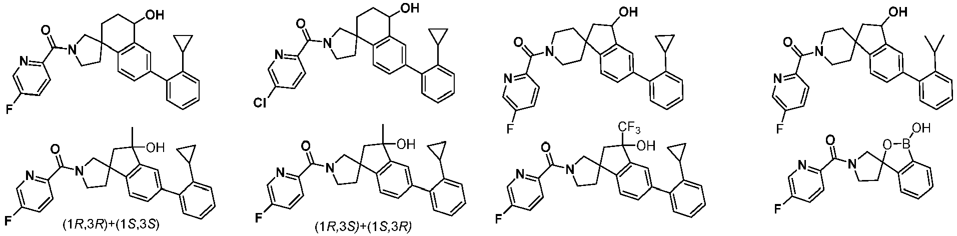

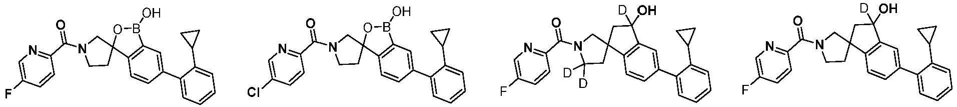

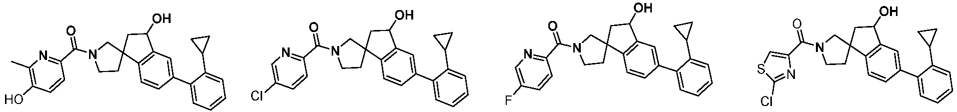

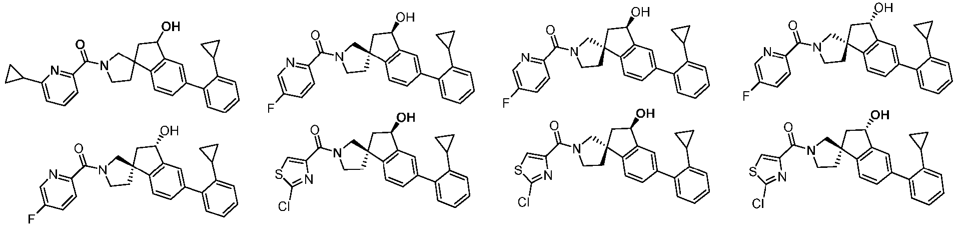

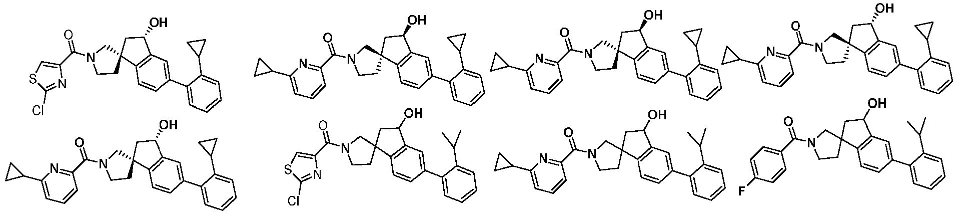

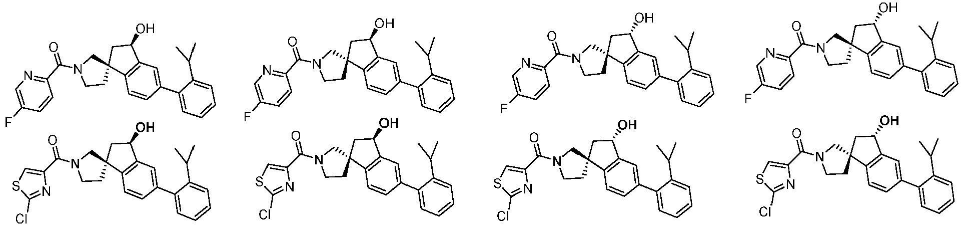

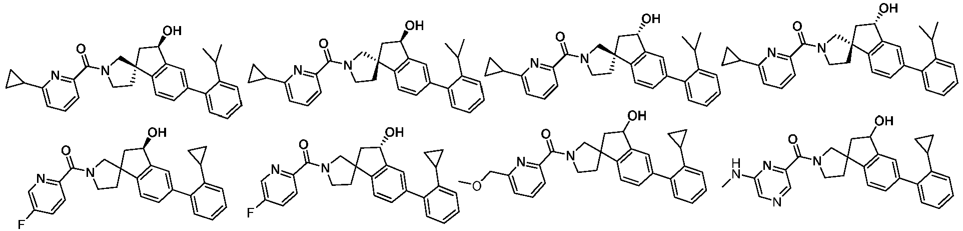

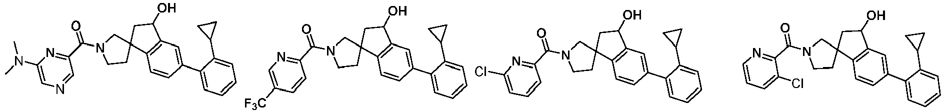

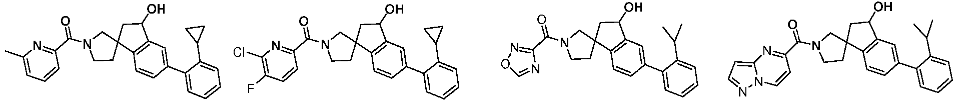

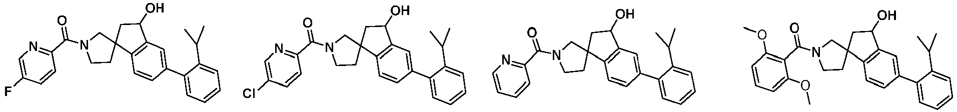

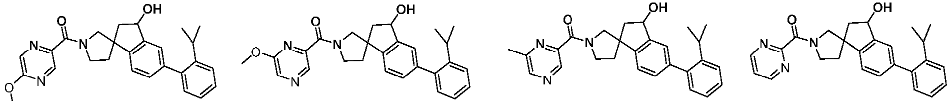

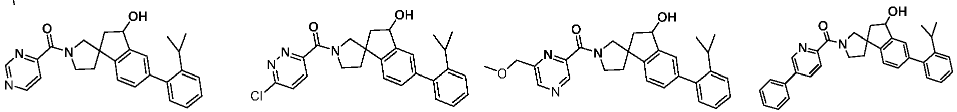

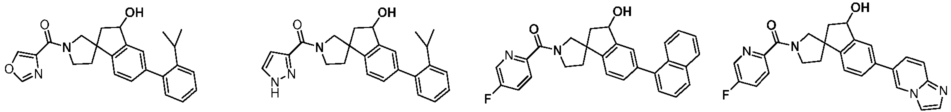

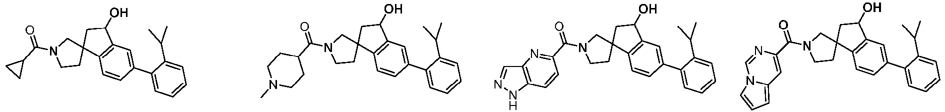

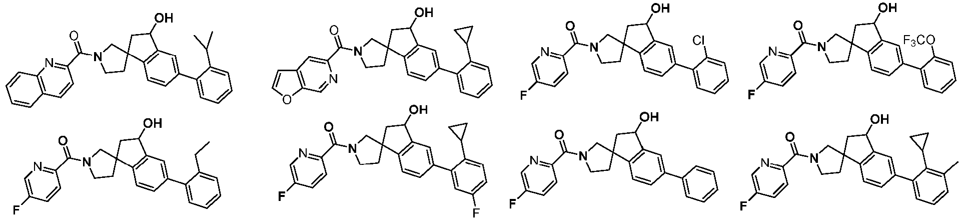

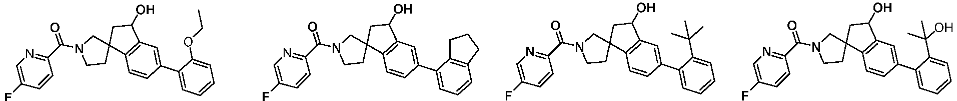

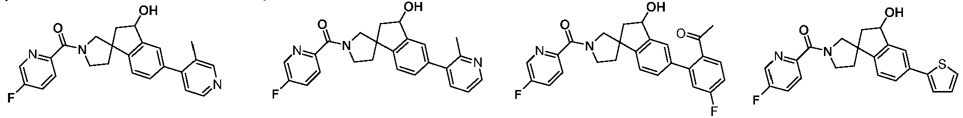

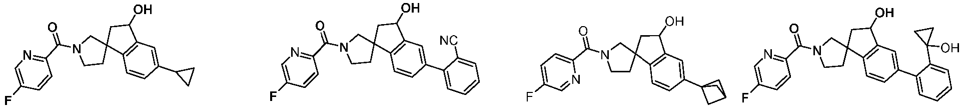

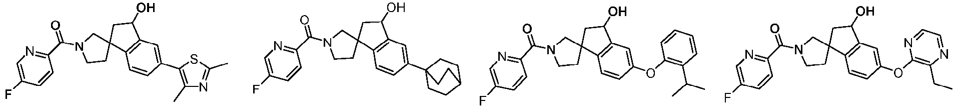

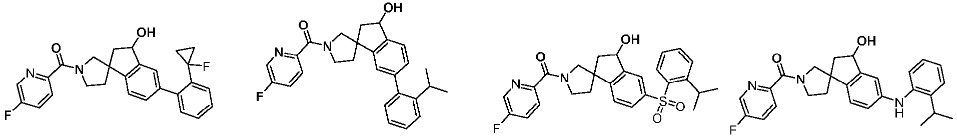

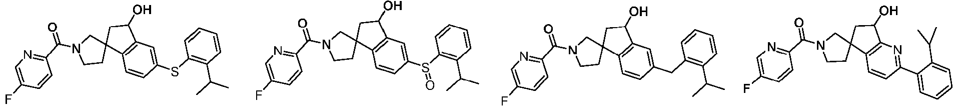

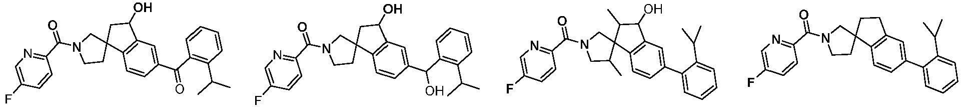

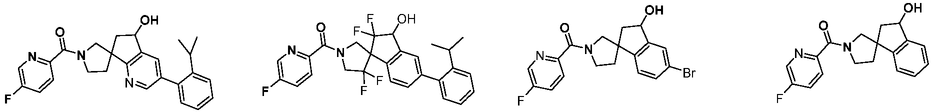

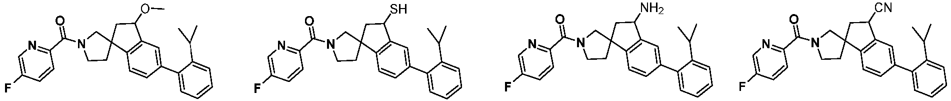

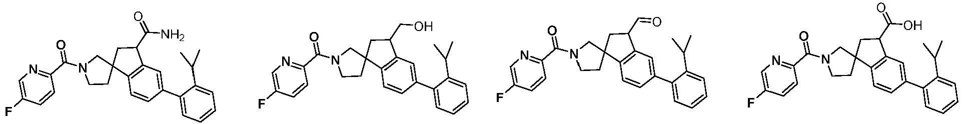

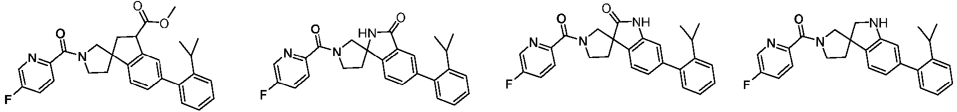

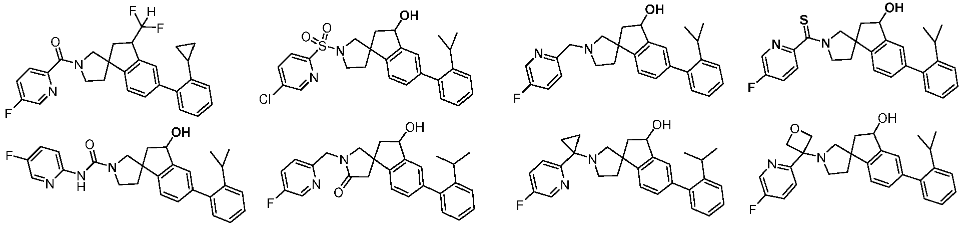

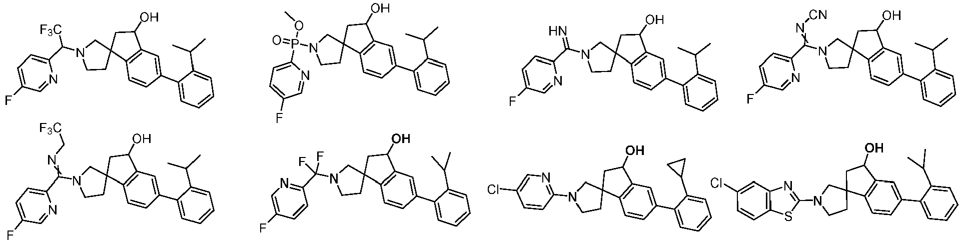

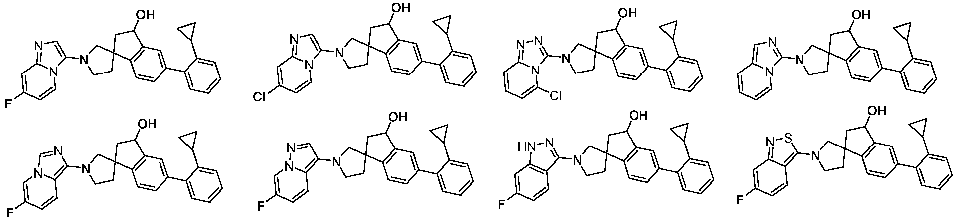

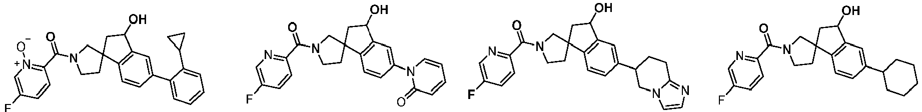

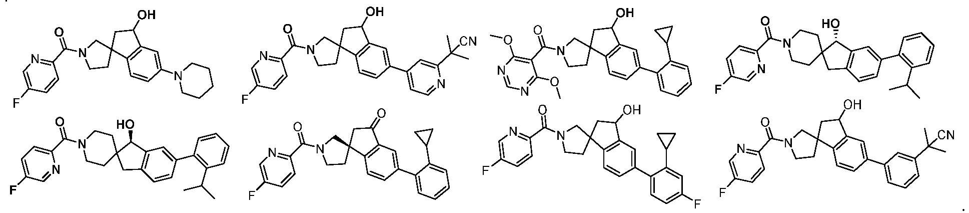

The present invention relates to a pharmaceutical composition, comprising the compound of the present invention, or stereoisomers, tautomers, solvates, hydrates, active metabolites, isotopic labels, or pharmaceutically acceptable salts thereof, and a pharmaceutically acceptable carrier.

The present invention also relates to use of the compound of the present invention, or stereoisomers, tautomers, solvates, hydrates, active metabolites, isotopic labels, or pharmaceutically acceptable salts thereof, and the pharmaceutical composition of the present invention in manufacture of pharmaceuticals for inhibiting TRPV3 activity.

The present invention also relates to use of the compound of the present invention, or stereoisomers, tautomers, solvates, hydrates, active metabolites, isotopic labels, or pharmaceutically acceptable salts thereof, and the pharmaceutical composition of the present invention in manufacture of pharmaceuticals for treating TRPV3-mediated conditions in a subject.

In one embodiment, the conditions are selected from the group consisting of pain, itching, skin conditions, inflammation, abnormal hair growth, incontinence, fever, hot flashes, cystitis, irritable bowel syndrome, and/or cough symptoms.

In one embodiment, the pain is cancerous pain and skin pain.

In one embodiment, it is used for manufacturing pharmaceuticals that inhibit proliferation, and thereby prevent, treat, or alleviat cancer symptoms.

In one embodiment, the cancer is liposarcoma.

In one embodiment, the abnormal hair growth is hair loss.

In one embodiment, the skin conditions are selected from the group consisting of skin keratosis, ichthyosis, and pruritus.

In one embodiment, the skin keratosis is olmsted syndrome.

In one embodiment, the ichthyosis is harlequin ichtyosis.

### General Synthesis Scheme

The compounds of the present invention can be prepared using the methods illustrated in the general synthesis scheme and experimental procedures detailed below. These general synthesis schemes and experimental procedures are presented for illustrative purposes and are not intended to be limiting. The raw materials used for preparing the compounds of the present invention are commercially available or can be prepared using conventional methods known in the art.

The representative procedures for preparing the compounds of the present invention are summarized in Scheme 1, Scheme 2, Scheme 3, Scheme 4 and Scheme 5. The raw materials of p-halogenated phenylacetonitrile and ethyl bromoacetate can be commercially available or prepared using methods known in the art, with representative procedures providing intermediates. Scheme 1 highlights the fully detailed synthesis of 1-(3-hydroxy-2,3-dihydrospiro[indene-1,3'-pyrrolidine]-1-one. The synthesis of diethyl 3-(4-halogenophenyl)-3-cyanopentanedioate 2 can be achieved by the reaction of p-halogenated phenylacetonitrile and ethyl bromoacetate in a solvent such as tetrahydrofuran. The cyano and ester groups of 2 generate cyclized 3 under reducing agent. Ester 3 hydrolyzes to carboxylic acid 4 under alkaline conditions, and 4 undergoes cyclization under acidic conditions to generate 5. 5 reacts with the desired boric acid under Suzuki conditions to form coupling in order to obtain the intermediate 6, which undergoes reduction under reducing agent conditions to generate intermediate 7, and then, it reacts with carboxylic acid under condensing agent conditions to generate 8, which is separated into 8a, 8b, 8c, and 8d by supercritical separation.

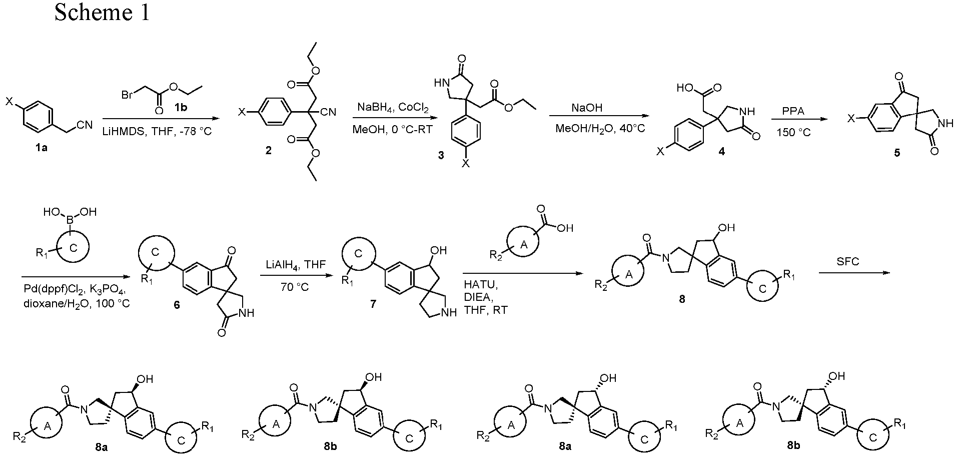

Scheme 2 shows the synthesis of the desired compound, in which 8 is generated through Suzuki reaction in the final step. The synthesis of intermediate 5 is described in scheme 1. Intermediate 5a is generated through intermediate 5 under reducing agent conditions, and 5a reacts with carboxylic acid to genenrate 5b. 5b reacts with the desired boric acid under Suzuki conditions to form coupling in order to generate intermediate 8, which is further separated into 8a, 8b, 8c, and 8d by supercritical separation.

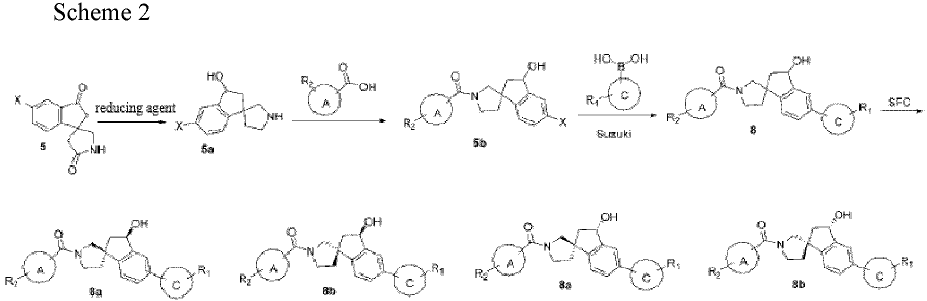

Scheme 3 shows the synthesis of the desired compound, which belongs to semi-chiral synthesis. The synthesis of intermediate 6 is described in Scheme 1. In the presence of a chiral ligand, the intermediate undergoes catalytic reduction to obtain chiral alcohol 6a. Amide is then reduced to generate 6b. 6b is condensed with the desired carboxylic acid to obtain 8, which is further separated into 8a and 8b by supercritical separation.

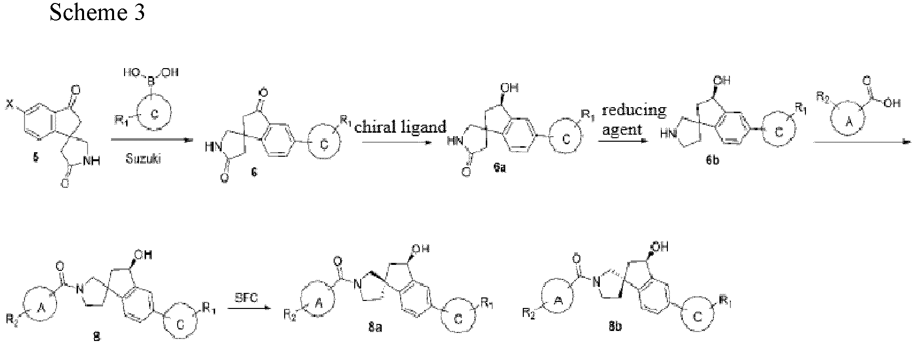

Scheme 4 shows the synthesis of the desired compound, which also belongs to semi-chiral synthesis. The synthesis of intermediate 5 is described in Scheme 1. Intermediate 5 is catalytically reduced by a chiral ligand to obtain 5a, which then reacts with the desired boric acid under Suzuki conditions to form coupling in order to generate 5b. 5b is reduced to generate 5c under reducing agent conditions. 5c is condensed with the desired carboxylic acid to obtain 8, which is further separated into 8a and 8b by supercritical separation.

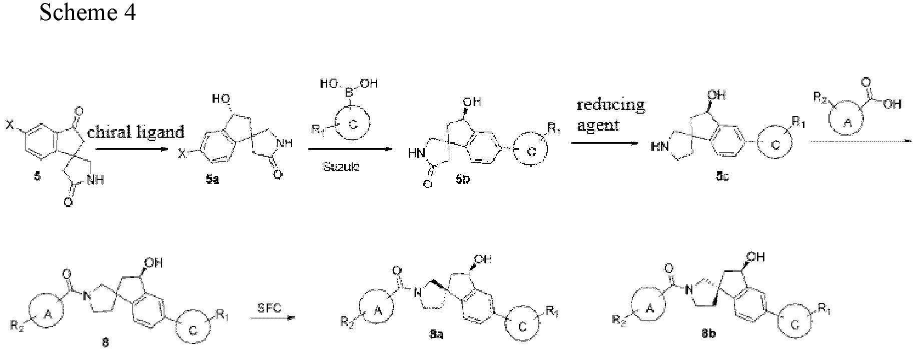

Scheme 5 shows the synthesis of the desired compound, which also belongs to semi-chiral synthesis. The synthesis of intermediate 5 is described in Scheme 1. Intermediate 5 is catalytically reduced by a chiral ligand to obtain 5a. Lactam is further reduced to generate intermediate 5b. 5b is condensed with the desired carboxylic acid to obtain 5c. 5c reacts then with the desired boric acid under Suzuki conditions to form coupling in order to generate 8, which is further separated into 8a and 8b by supercritical separation.

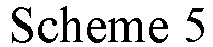

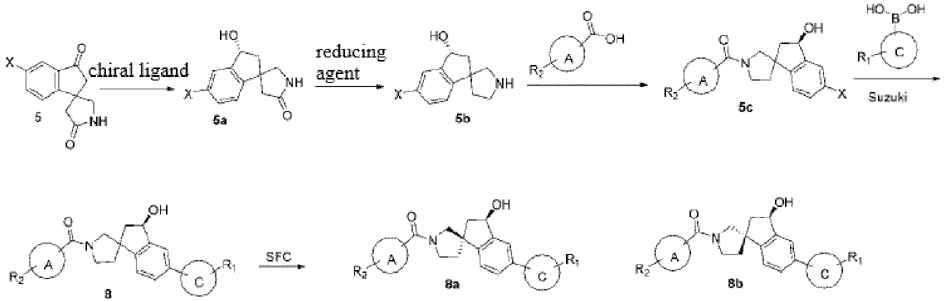

### Detailed Description

### Preparation of intermediates

### Preparation Example 1: 5-bromospiro[indene-1,3'-pyrrolidine]-3,5'(2H)-dione (compound I1)

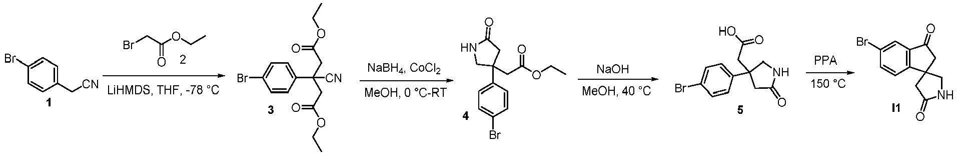

### Step A: diethyl 3-(4-bromophenyl)-3-cyanopentanedioate

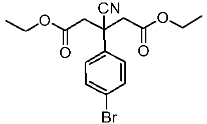

20.0 g (102 mmol, 1.0 eq) 2-(4-bromophenyl)acetonitrile was dissolved in 100mL tetrahydrofuran, and nitrogen replacement was carried out for three times. The temperature was reduced to -60 °C using dry ice. 224 mL (224 mmol, 2.2 eq) lithium bis(trimethylsilyl)amine (lithium hexamethyldisilazide) was added dropwise at -60 °C, and stirring was carried out at room temperature for 3 h. Subsequently, 34.1 g (204 mmol, 2.0 eq) ethyl bromoacetate was added dropwise below -60 °C, and stirring was carried out overnight at room temperature. After confirming that the raw materials had reacted completely through LCMS, 200 mL water was added for quenching, and then extraction was carried out with ethyl acetate for three times. The organic layer was washed with 100 mL saturated saline solution, dried over anhydrous sodium sulfate and evaporated under reduced pressure. The residue was subjected to column chromatography (PE/EA = 0~10:1) to obtain the product (37.4 g, yield=99%).

¹H NMR (400 MHz, CDCl₃) δ 7.54 (dd, *J =* 8.7, 2.7 Hz, 2H), 7.39 (dd, J=8.7, 2.7 Hz, 2H), 4.12 - 4.10 (m, 4H), 3.25 (d, *J =* 16.3 Hz, 2H), 3.08 (d, *J* = 16.3 Hz, 2H), 1.19 (t, *J* = 7.1 Hz, 6H).

### Step B: ethyl 2-(3-(4-bromophenyl)-5-oxopyrrolidin-3-yl) acetate

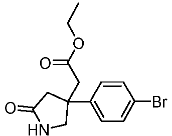

5.00 g (13.8 mmol, 1.0 eq) diethyl 3-(4-bromophenyl)-3-cyanopentanedioate was dissolved in 50 mL methanol, and 3.70 g (28.5 mmol, 2.1 eq) anhydrous cobalt chloride was added. Then, 5.39 g (142 mmol, 10.5 eq) sodium borohydride was slowly added at 0 °C, and stirring was carried out at room temperature for 1 h. After confirming that the product had been generated through LCMS, 50 mL hydrochloric acid solution (2mol/L) was used to dilute, and then, extraction was carried out with dichloromethane for three times. The organic layer was washed with 50 mL saturated saline solution, dried over anhydrous sodium sulfate and evaporated under reduced pressure. The residue was subjected to column chromatography (DCM/MeOH = 0~15:1) to obtain the product (3.29 g, yield=59%).

LC-MS: (M+H)⁺; m/z = 326.05, 327.17;

### Step C: 2-(3-(4-bromophenyl)-5-oxopyrrolidin-3-yl) acetic acid

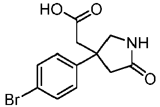

1.0 g (3.07 mmol, 1.0 eq) ethyl 2-(3-(4-bromophenyl)-5-oxopyrrolidin-3-yl) acetate was dissolved in 20 mL methanol, and 12.5 mL sodium hydroxide solution (1.0mol/L) was slowly added at 0 °C, and stirring was carried out at 40 °C for 1 h. After confirming that the product had been generated through LCMS, the reaction solution was diluted with hydrochloric acid solution (1.0 mol/L) to acidity, and then extracted three times with dichloromethane. The organic layer was washed with 50 mL saturated saline solution, dried over anhydrous sodium sulfate and evaporated under reduced pressure to obtain a crude product (0.75 g, yield =82%).

LC-MS: (M-H)⁻; m/z = 297.66, 298.25;

### Step D: 5-bromospiro[indene-1,3'-pyrrolidine]-3,5'(2H)-dione

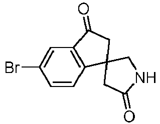

7.5 g polyphosphoric acid was heated to 150 °C, and then 0.75 g (2.52 mmol, 1.0 eq) 2-(3-(4-bromophenyl)-5-oxopyrrolin-3-yl) acetic acid was added. Then, stirring was carried out at 150 °C for 1 h. After confirming that the product had been generated through LCMS, the mixture was slowly poured into ice water while it was still hot, and stirred while adding, and then extracted three times with dichloromethane. The organic layer was washed with 30 mL saturated saline solution, dried over anhydrous sodium sulfate and evaporated under reduced pressure. The residue was subjected to column chromatography (DCM/MeOH = 0~20:1) to obtain the product (0.65 g, yield=92%).

LC-MS: (M+H)⁺; m/z = 280.03, 282.04.

### Preparation Example 2: 5-(2-cyclopropylphenyl)spiro[indene-1,3'-pyrrolidine]-3,5'(2H) - dione (compound I2)

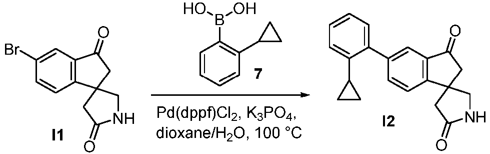

550 mg (1.96 mmol, 1.0 eq) 5-bromospiro[indene-1,3'-pyrrolidine]-3,5'(2*H*)-dione and 381 mg (2.35 mmol, 1.2 eq) (2-cyclopropylphenyl)boric acid were dissolved in 50mL dioxane/water mixed solution (V:V = 4: 1), and then 116 mg (0.20 mmol, 0.1 eq) [1,1'-bis(diphenylphosphine)ferrocene] dichloride palladium and 1.25 g (5.89 mmol, 3.0 eq) tripotassium phosphate were added. Nitrogen replacement was carried out for three times, and stirring was carried out at 100 °C for 1 h. After confirming that the product had been generated through LCMS, 50 mL water was added thereto, and then extraction was carried out with ethyl acetate for three times. The organic layer was washed with 30 mL saturated saline solution, dried over anhydrous sodium sulfate and evaporated under reduced pressure. The residue was subjected to column chromatography (PE/EA = 0~10:1) to obtain the product (620 mg, yield=99%).

LC-MS: (M+H)⁺; m/z = 318.20.

### Preparation Example 3: 5-(2-cyclopropylphenyl)-2,3-dihydrospiro[indene-1,3'-pyrrolidine] -3-ol (compound I3)

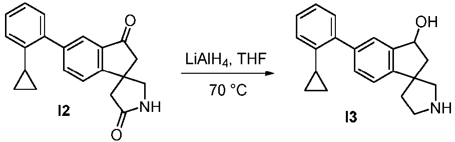

650 mg (2.05 mmol, 1.0 eq) 5-bromospiro[indene-1,3'-pyrrolidine]-3,5'(2*H*)-dione was dissolved in 50mL anhydrous tetrahydrofuran. 1.16 g (30.7 mmol, 15.0 eq) lithium aluminum hydride was slowly added at 0 °C, and stirring was carried out at 70 °C for 2 h. After confirming that the raw materials had been completely converted and the product had been generated through LCMS, the reaction solution was filtered through diatomite and washed with tetrahydrofuran. After drying over anhydrous sodium sulfate and evaporating under reduced pressure, a crude product was obtained (420 mg, yield=67%).

LC-MS: (M+H)⁺; m/z = 306.26;

### Preparation Example 4: 5-(2-isopropylphenyl)-2,3-dihydrospiro[indene-1,3'-pyrrolidine]-3-ol (compound I4)

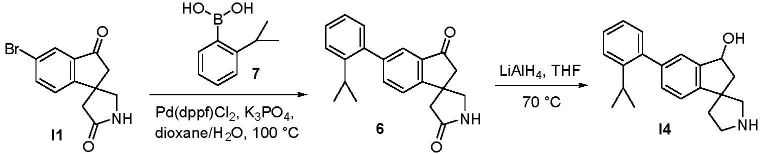

### Step A: 5-(2-isopropylphenyl)spiro[indene-1,3'-pyrrolidine]-3,5'(2H)-dione

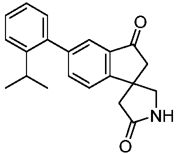

2.60 g (9.28 mmol, 1.0 eq) 5-bromospiro[indene-1,3'-pyrrolidine]-3,5'(2*H*)-dione, 1.83 g (11.1 mmol, 1.2 eq) (2-isopropylphenyl) boric acid, 7.88 g (37.1 mmol, 4.0 eq) potassium phosphate, 0.07 g (0.093 mmol, 0.01 eq) [1,1'-bis(diphenylphosphine)ferrocene] palladium dichloride dichloromethane complex, and 40 mL (1,4-dioxane: water=4:1) mixed solvent were added in a 100 mL reaction flask. Nitrogen replacement was carried out for three times, and then the reaction was carried out at 100°C for 4 h. A complete reaction of the raw materials was confirmed through TLC Plate (DCM : MeOH=20:1). 50 mL water was added in the reaction solution, and then extraction was carried out with ethyl acetate (50 mL) for three times. The organic phases were combined, dried, concentrateed, and sampled. Purification by silica gel column chromatography (DCM : MeOH=20:1) was carried out to obtain 2.56g yellow oily product (yield=83%).
LC-MS: (M+H)⁺; m/z = 320.16;
1H NMR (400 MHz, DMSO-d₆) δ 8.08 (s, 1H), 7.91-7.93 (m, 1H), 7.63-7.68 (m, 1H), 7.37-7.52 (m, 2H), 7.23-7.31 (m, 2H), 7.09-7.16 (m, 1H), 3.40-3.63(m, 2H), 3.17-3.25 (m, 2H), 2.69-2.75(m, 3H), 1.19 (d, *J* = 4 Hz, 3H), 1.14 (d, *J* = 8 Hz, 3H).

### Step B: 5-(2-isopropylphenyl)-2,3-dihydrospiro[indene-1,3'-pyrrolidine]-3-ol

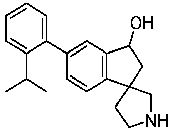

1.50 g (4.69 mmol, 1.0 eq) 5-(2-isopropylphenyl)spiro[indene-1,3'-pyrrolidine]-3,5'(2*H*)-dione and 20 mL tetrahydrofuran were added in a 100 mL reaction flask. The reaction solution was cooled to 0 °C under nitrogen protection. 2.67 g (70.4 mmol, 15.0 eq) lithium aluminium hydride was slowly added. After stirring at 0 °C for 10 min, the reaction solution was refluxed for 2 h. A complete reaction of the raw materials was confirmed through TLC Plate (DCM : MeOH=20:1). 2mL water was added to the reaction solution to quench the reaction, and then filtration was carried out through diatomite. The filter cake had been washed with tetrahydrofuran (200mL) until the product was completely dissolved. The filtrate was concentrated and dried to obtain 1.20 g yellow oily liquid crude product.

LC-MS: (M+H)⁺; m/z = 308.14.

### Preparation Example 5: (5-bromo-3-hydroxy-2,3-dihydrospiro[indene-1,3'-pyrrolidine]-1'-yl) (5-fluoropyridin-2-yl)ketone (compound I5)

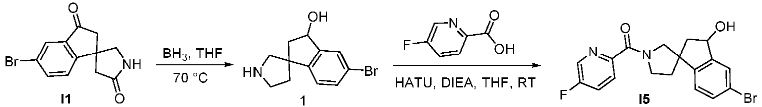

### Step A: 5-bromo-2,3-dihydrospiro[indene-1,3'-pyrrolidine]-3-ol

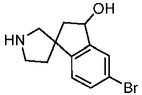

In a 50mL Shrek tube, 300 mg (1.07 mmol, 1.0 eq) 5-bromospiro[indene-1,3'-pyrrolidine]-3,5'(2*H*)-dione was dissolved in 6 mL tetrahydrofuran. 1.1 mL (10.7 mmol, 10.0 eq) solution of borane in dimethyl sulfide was slowly added by using a syringe. After the addition, the system was heated to 70 °C and reacted for 18 h. After confirming that the target product had been generated through LCMS, the reaction solution was cooled to -10 °C, and the resction was quenched by slowly adding 2 mL water dropwise. 20 mL tetrahydrofuran was added again to the reation system. Filtration was carried out through diatomite. The filter cake had been washed with tetrahydrofuran (20mL x 3) until the product was completely dissolved. The filtrate was concentrated and dried to obtain 350 mg yellow oily liquid crude product. The subsequent reaction proceeded directly.

LC-MS: (M+H)⁺; m/z = 268.01;

### Step B: (5-bromo-3-hydroxy-2,3-dihydrospiro[indene-1,3'-pyrrolidine]-1'-yl)(5-fluoropyridin-2-yl) ketone

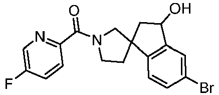

145 mg (1.03 mmol, 1.2 eq) 5-fluoropyridin-2-carboxylic acid, 391 mg (1.03 mmol, 1.2 eq) 2-(7-azobenzotriazole)-*N,N,N',N'*-tetramethylurea hexafluorophosphate, 665 mg (5.15 mmol, 6.0 eq) N,N-diisopropylethylamine, and 10 mL tetrahydrofuran was added in a 50 mL reaction flask. The reaction solution was stired at room temperature for 10 min under nitrogen protection, and then a solution of 230 mg (0.86 mmol, 1.0 eq) 5-bromo-2,3-dihydrospiro[indene-1,3'-pyrrolidine]-3-ol dissolved in 6 mL tetrahydrofuran was added in the reaction solution. Stirring was carried out at room temparature for 2 h. After confirming that the target product had been generated through LCMS, the reaction solution is quenched by adding 15 mL water, and extracted three times with ethyl acetate (25mL). The organic phases were combined, dried, and concentrated. The crude product was purified by column chromatography (PE/EA=50:1~5/1) to obtain 203 mg oily product (yield=70%).

LC-MS: (M+H)⁺; m/z = 391.01.

### Example 1: (6-(2-Cyclopropylphenyl)-4-hydroxy-3,4-dihydro-2H-spiro[naphthalene-1,3'-pyrrolidine]-1'-yl) (5-fluoropyridin-2-yl) ketone

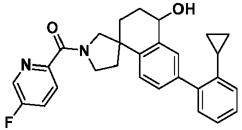

### Step A: 2-(4-bromophenyl)-4-(1,3-dioxolan-2-yl) nitrile

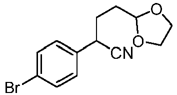

Under nitrogen protection, 2-(4-bromophenyl)acetonitrile (20.0 g, 102 mmol, 1.0 eq) was dissolved in 100 mL tetrahydrofuran, and then LiHMDS (122mL, 1.0mol/L, 1.2eq) was slowly dripped at -78 °C. After the dropwise addition, the temperature was heated to room temperature, and stirring was carried out for 3 h. Then, the temperature was reduced to -78 °C. Subsequently, 2- (2-bromoethyl)-1,3-dioxane (22.0 g, 122 mmol, 1.2 eq) was slowly added dropwise. After the dropwise addition, the temperature slowly rised to room temperature, and stirring was carried out overnight. After confirming that the raw materials had reacted completely through TLC, 200mL ice water was slowly added dropwise thereto in order to quench the reaction, and then extraction was carried out with ethyl acetate (200mL x 3). Subsequently, the organic layer was washed with 200 mL saturated saline solution. The organic phase was dried over anhydrous sodium sulfate, and evaporated under reduced pressure to obtain a crude product. The crude product was purified by silica gel column chromatography (PE/EA = 10/1) to obtain the target product (13.0 g, yield =43%).

¹H NMR (400 MHz, CDCl₃) δ 7.52 - 7.48 (m, 2H), 7.23 - 7.19 (m, 2H), 4.89 (t, *J =* 4.0 Hz, 1H), 3.99 - 3.80 (m, 5H), 2.07 - 1.93 (m, 2H), 1.85 - 1.78 (m, 2H).

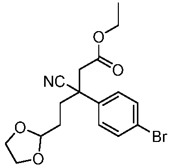

### Step B: ethyl 3-(4-bromophenyl)-3-cyano-5-(1,3-dioxolan-2-yl) valerate

2-(4-bromophenyl)-4-(1,3-dioxolan-2-yl)nitrile (4.0 g, 13.5 mmol, 1.0 eq) was dissolved in 40 mL tetrahydrofuran, and then sodium hydride (1.62 g, 40.5 mmol, 60% in oil, 3 eq) was added in batches under ice bath. After the addition, the temperature rised to 70 °C, and heating and stirring were carried out for 2 h. Then, ethyl bromoacetate (2.70 g, 16.2 mmol, 1.2 eq) was slowly added dropwise. After the dropwise addition, heating and stirring were proceededed at 70 °C overnight. The generation of a new product was confirmed by TLC, with some remaining raw materials. After cooling down, 100mL ice water was slowly added dropwise to quench the reaction, and then extraction was carried out with ethyl acetate for three times (100mL x 3). The organic layer was washed with 100 mL saturated saline solution. The organic phase was dried over anhydrous sodium sulfate, and evaporated under reduced pressure to obtain a crude product. The crude product was subjected to silica gel column chromatography (PE/EA = 10:1~5:1) to obtain the target product (1.6 g, yield =31%).

¹H NMR (400 MHz, CDCl₃) δ 7.54 - 7.49 (m, 2H), 7.33 - 7.30 (m, 2H), 4.82 (t, *J* = 4.0 Hz, 1H), 4.03 (q, *J* = 6.8 Hz, 2H), 3.92 - 3.78 (m, 4H), 3.03 - 2.88 (m, 2H), 2.24 - 2.16 (m, 1H), 2.06 - 1.99 (m, 1H), 1.48 - 1.39 (m, 1H), 1.36 - 1.24 (m, 1H), 1.12 (t, *J* = 7.2 Hz, 3H).

### Step C: ethyl 3-cyano-3-(2'-cyclopropyl-[1,1'-biphenyl]-4-yl)-5-(1,3-dioxane-2-yl) valerate

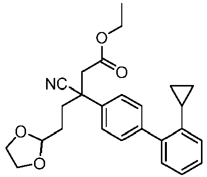

1.60 g (4.18 mmol, 1.0 eq) ethyl 3-(4-bromophenyl)-3-cyano-5-(1,3-dioxolan-2-yl) valerate and 813 mg (5.02 mmol, 1.2 eq) (2-cyclopropylphenyl) boric acid were dissolved in 50mL mixed solution of dioxane/water (V:V = 4: 1), and then 307 mg (0.42 mmol, 0.1 eq) [1,1'-bis(diphenylphosphine)ferrocene] dichloride palladium and 2.66 g (12.5 mmol, 3.0 eq) tripotassium phosphate were added. Nitrogen replacement was carried out for three times, and stirring was carried out at 100 °C for 2 h. After cooling, 50 mL water was added thereto, and then extraction was carried out with ethyl acetate for three times. The organic layer was washed with 30 mL saturated saline solution, dried over anhydrous sodium sulfate and evaporated under reduced pressure. The residue was subjected to column chromatography (PE/EA = 0~5:1) to obtain the product (1.10 g, yield=63%).

¹H NMR (400 MHz, CDCl₃) δ 7.50 - 7.44 (m, 4H), 7.30 - 7.26 (m, 1H), 7.23 - 7.19 (m, 2H), 6.94 (d, *J* = 7.8 Hz, 1H), 4.87 (t, *J* = 4.4 Hz, 1H), 4.10 - 4.04 (m, 2H), 3.97 - 3.87 (m, 2H), 3.86 - 3.80 (m, 2H), 3.09 - 2.96 (m, 2H), 2.33 - 2.26 (m, 1H), 2.17 - 2.10 (m, 1H), 1.95 - 1.79 (m, 2H), 1.71 - 1.62 (m, 1H), 1.12 (t, *J* = 7.2, 3H), 0.89 - 0.79 (m, 2H), 0.70 - 0.66 (m, 2H).

### Step D: 4-(2-(1,3-dioxan-2-yl)ethyl)-4-(2'-cyclopropyl- [1,1'- biphenyl]-4-yl)pyrrolidin-2-one

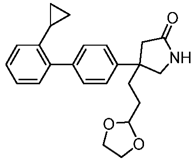

1.10 g (2.62 mmol, 1.0 eq) ethyl 3-cyano-3-(2'-cyclopropyl-[1,1'-biphenyl]-4-yl)-5-(1,3-dioxane-2-yl) valerate was dissolved in 25 mL methanol, and then 714 mg (5.50 mmol, 2.1 eq) anhydrous cobalt chloride was added. Then, 1.04 g (27.5 mmol, 10.5 eq) sodium borohydride was slowly added at 0 °C, and stirring was carried out at room temperature for 1 h. After confirming that the product had been generated through LCMS, 50 mL ice water was used to quench the reaction, and then, extraction was carried out with dichloromethane for three times. The organic layer was washed with 50 mL saturated saline solution, dried over anhydrous sodium sulfate and evaporated under reduced pressure. The residue was subjected to column chromatography (DCM/MeOH = 0~15:1) to obtain the product (0.80 g, yield=81%).
LC-MS: (M+H)⁺; m/z = 378.26;
¹H NMR (400 MHz, CDCl₃) δ 7.43 - 7.41 (m, 2H), 7.29 - 7.24 (m, 1H), 7.22 - 7.17 (m, 4H), 6.93 (d, *J* = 8.0 Hz, 1H), 6.02 (s, 1H), 4.76 (t, *J* = 4.4 Hz, 1H), 3.93 - 3.78 (m, 4H), 3.74 - 3.61 (m, 2H), 2.84 - 2.58 (m, 2H), 2.01 - 1.93 (m, 2H), 1.90 - 1.83 (m, 1H), 1.55 - 1.49 (m, 2H), 0.89 - 0.80 (m, 2H), 0.71 - 0.64 (m, 2H).

### Step E: 3-(3-(2-(2'-cyclopropyl-[1,1'-biphenyl]-4-yl)-5-oxopyrrolidin-3-yl) propanal

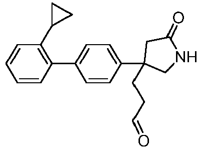

0.80 g (2.12 mmol, 1.0 eq) 4-(2-(1,3-dioxane-2-yl)ethyl)-4-(2'-cyclopropyl-[1,1'-biphenyl]-4-yl)pyrrolidin-2-one was dissolved in 5 mL 1,4-dioxane, and then, 10.64 mL HCl/1,4-dioxane solution (4.24 mmol, 20 eq, 4M) was added under ice bath. After reacting overnight with stirring, a complete conversion was detected through LCMS. Sodium bicarbonate solution with ice was added to adjust the pH value to around 7-8 under ice bath, and then, extraction was carried out with ethyl acetate for three times. The organic layer was washed with 50 mL saturated saline solution, dried over anhydrous sodium sulfate, and concentrated by vacuum distillation to obtain the crude product of the target product (0.50 g, yield =71%), which was used directly in the subsequent reaction without purification.
LC-MS: (M+H)⁺; m/z = 334.11;
¹H NMR (400 MHz, CDCl₃) δ 9.63 (s, 1H), 7.46 - 7.44 (m, 2H), 7.23 - 7.17 (m, 5H), 6.96 - 6.93 (m, 2H), 3.79 - 3.62 (m, 4H), 2.42 - 2.27 (m, 2H), 2.21 - 2.17 (m, 1H), 1.91 - 1.82 (m, 2H), 0.88 - 0.81 (m, 2H), 0.72 - 0.68 (m, 2H).

### Step F: 3-(3-(2-(2'-cyclopropyl-[1,1'-biphenyl]-4-yl)-5-oxopyrrolidin-3-yl) propionic acid

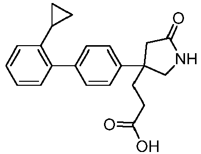

3-(3-(2-(2'-cyclopropyl-[1,1'-biphenyl]-4-yl)-5-oxopyrrolidin-3-yl) propanal (0.50 g, 1.50 mmol, 1.0 eq) was dissolved in a mixture of tert butanol (10mL) and 2-methyl-2-butene (1.05 g, 15.0 mmol, 10 eq). The obtained solution was stirred and cooled to 0 °C. A mixture of NaClO₂ (80% mixture with NaCl, 237 mg, 2.1 mmol, 1.4 eq) and NaH₂PO₄ (180 mg, 1.50 mmol, 1.0 eq) was dissolved in a minimum volume of water, and then added to the reaction. The reaction solution had been monitored through TLC until a complete consumption of aldehyde was observed. The reaction solution was acidified with NaHSO₄ (aq) to pH=3, and then extracted three times with ethyl acetate. The organic layer was washed with 50 mL saturated saline solution, dried over anhydrous sodium sulfate and evaporated under reduced pressure to obtain the crude product of the target product. The crude product was subjected to column chromatography (DCM/MeOH = 0~15:1) to obtain the product (300 mg, yield =57%).
LC-MS: (M+H)⁺; m/z = 350.21;

### Step G: 6-(2-cyclopropylphenyl)-2,3-dihydro-4H-spiro[naphthalene-1,3'-pyrrolidine]-4,5'-dione

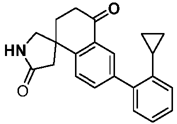

6.0 g polyphosphoric acid was heated to 150 °C, and then 300 mg (0.85 mmol, 1.0 eq) 3-(3-(2-(2'-cyclopropyl-[1,1'-biphenyl]-4-yl)-5-oxopyrrolidin-3-yl) propionic acid was added while stirring. Then, stirring was carried out at 150 °C for 1 h. After confirming that the product had been generated through LCMS, the mixture was slowly poured into ice water while it was still hot, and stirred while adding, and then extracted three times with dichloromethane. The organic layer was washed with 30 mL saturated saline solution, dried over anhydrous sodium sulfate and evaporated under reduced pressure to obtain the crude product of the target product (120 mg, yield=42%), which was used directly in the subsequent reaction.
LC-MS: (M+H)⁺; m/z = 332.16;

### Step H: 6-(2-cyclopropylphenyl)-3,4-dihydro-2H-spiro[naphthalene-1,3'-pyrrolidine]-4-ol

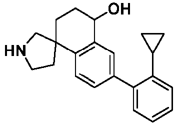

120 mg (0.36 mmol, 1.0 eq) 6-(2-cyclopropylphenyl)-2,3-dihydro-4*H*-spiro[naphthalene-1,3'-pyrrolidine]-4,5'-dione was dissolved in 5 mL anhydrous tetrahydrofuran. 205 mg (5.40 mmol, 15.0 eq) lithium aluminum hydride was slowly added at 0 °C, and stirring was carried out at 70 °C for 2 h. After confirming that the product had been generated through LCMS, the reaction solution was filtered through diatomite and washed with tetrahydrofuran. After drying over anhydrous sodium sulfate and evaporating under reduced pressure, a crude product was obtained (50 mg, yield =43%), which was used directly in the subsequent reaction.
LC-MS: (M+H)⁺; m/z = 320.22;

### Step I: (6-(2-cyclopropylphenyl)-4-hydroxy-3,4-dihydro-2H-spiro[naphthalene-1,3'-pyrrolidine]-1'-yl)(5-fluoropyridin-2yl) ketone

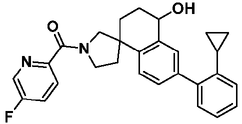

33.0 mg (0.234 mmol, 1.5 eq) 5-fluoropyridin-2-carboxylic acid, 60.5 mg (0.468 mmol, 3.0 eq) N,N-diisopropylethylamine and 89.0 mg (0.234 mmol, 1.5 eq) 2-(7-azobenzotriazole)-*N,N,N',N'*-tetramethylurea hexafluorophosphate were dissolved in 5 mL tetrahydrofuran. Stirring was carried out at room temperature for 0.5 h. 50 mg (0.156 mmol, 1.0 eq) 6-(2-cyclopropylphenyl)-3,4-dihydro-2*H*-spiro[naphthalene-1,3'-pyrrolidine]-4-ol was added, and stirring was carried out at room temparature for 1.5 h. After confirming that the product had been generated through LCMS, 50mL saline solution was added thereto, and then, extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min), and freeze-dring, a white solid was obtained (4.15 mg, yield =6%).
LC-MS: (M+Na)⁺; m/z = 465.11;
¹H NMR (400 MHz, CDCl₃) δ 8.45 - 8.35 (m, 1H), 8.02 (dd, *J* = 8.8, 4.4 Hz, 1H), 7.59 - 7.49 (m, 4H), 7.36 - 7.26 (m, 4H), 4.10 - 3.86 (m, 4H), 2.91- 2.86 (m, 2H), 2.44 - 2.37 (m, 1H), 2.24 - 2.14 (m, 3H), 2.10 - 2.00 (m, 2H), 1.90 - 1.83 (m, 1H), 0.63 - 0.50 (m, 4H).

### Example 2: (5-chloropyridin-2-yl)(6-(2-cyclopropylphenyl)-4-hydroxy-3,4-dihydro-2H-spiro[naphthalene-1,3'-pyrrolidine]-1'-yl) ketone

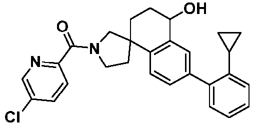

Experimental procedure referred to Step I in Example 1, but wherein the reagent 5-fluoropyridine-2-carboxylic acid was replaced with 5-chloropyridin-2-carboxylic acid to synthesize the compound of Example 2.
LC-MS: (M+Na)⁺; m/z = 481.07;
¹H NMR (400 MHz, CDCl₃) δ 8.48 - 8.39 (m, 1H), 8.05 (dd, *J* = 8.8, 4.4 Hz, 1H), 7.61 - 7.50 (m, 4H), 7.37 - 7.27 (m, 4H), 4.12 - 3.88 (m, 4H), 2.93- 2.88 (m, 2H), 2.45 - 2.39 (m, 1H), 2.25 - 2.16 (m, 3H), 2.13 - 2.00 (m, 2H), 1.92 - 1.86 (m, 1H), 0.64 - 0.52 (m, 4H).

### Example 3: (5-(2-cyclopropylphenyl)-3-hydroxy-2,3-dihydrospiro[indene-1,4'-piperidine]-1'-yl)(5-fluoropyridin-2-yl) ketone

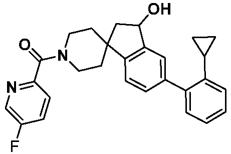

### Step A: 5-bromo-1H-indene

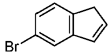

2.00 g (9.38 mmol, 1.0 eq) 6-bromo-2,3-dihydro-1*H*-indene-1-ol was dissolved in 20 mL toluene, and then 180 mg (0.94 mmol, 0.1 eq) p-toluenesulfonic acid monohydrate was added to the rection system. The reaction was carried out under nitrogen protection at 65 °C for 3 hours with stirring. The temperature was reduced to room temperature, and then 30 mL water and 80 mL ethyl acetate were added. The organic phase was separated. The aqueous phase was extracted twice with ethyl acetate (2 x 60mL). The organic phases were combined, washed with salt water, dried over anhydrous sodium sulfate and concentrated to obtain a crude product. The crude product was subjected to column chromatography (petroleum ether) to obtain an oily substance (1.50g, yield =82%).

¹H NMR (400 MHz, CDCl₃) δ 7.52 (d, *J* = 4.0 Hz, 1H), 7.33 - 7.27 (m, 2H), 6.83 - 6.80 (m, 1H), 6.61 - 6.58 (m, 1H), 3.35 - 3.34 (m, 2H).

### Step B: tert-butyl 5-bromospiro[indene-1,4'-piperidine]-1'-carboxylate

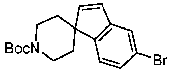

1.50 g (7.69 mmol, 1.0 eq) 5-bromo-1*H*-indene was dissolved in 20 mL tetrahydrofuran. The temperature was reduced to 0 °C. 19.4 mL (19.2 mmol, 2.5 eq) lithium bis(trimethylsilyl)amine was added dropwise to the above reaction system under nitrogen protection. After the dropwise addition, the reaction system was stirred at 0 °C for 1 h. Then, a solution of 2.23 g (9.23 mmol, 1.2 eq) tert butyl bis(2-chloroethyl)carbamate dissolved in 20 mL tetrahydrofuran was added dropwise to the above reaction solution. After the dropwise addition, the temperature naturally rised to room temperature, and the reaction was carried out for 16 h. After confirming that the raw materials had reacted completely through LC-MS results, 40 mL water and 90 mL ethyl acetate were added. The organic phase was separated. The aqueous phase was extracted twice with ethyl acetate (2 x 80mL). The organic phases were combined, washed with salt water, dried over anhydrous sodium sulfate and concentrated to obtain a crude product. The crude product was subjected to column chromatography (petroleum ether: ethyl acetate=100:1~20:1) to obtain an oily substance (2.40 g, yield =86%).
LC-MS: (M-Boc)⁺; m/z = 264.12;

### Step C: tert-butyl 5-bromo-3-hydroxy-2,3-dihydrospiro[indene-1,4'-piperidine]-1'-carboxylate

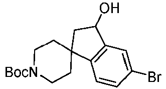

100 mg (0.28 mmol, 1.0 eq) tert-butyl 5-bromospiro[indene-1,4'-piperidine]-1'-carboxylate was dissolved in 5 mL anhydrous tetrahydrofuran, and then 1.1 mL (0.56 mmol, 2.0 eq) 9-borobicyclo[3.3.1]nonane was added to the above reaction system. The reaction system was reacted in a sealed tube at 70 °C for 17 h. The reaction solution was cooled to room temperature, and 0.55 mL 1M sodium hydroxide solution was added, and then 0.1 mL (30% mw) hydrogen peroxide solution was added, and stirring was proceeded for 1 h. Extraction was carried out twice with ethyl acetate (50mL x 2). The organic phases were washed with salt water, dried over anhydrous sodium sulfate, and concentrated to obtain a light yellow oily substance (80.0 mg, yield=80%).
LC-MS: (M-Boc)⁺; m/z = 282.00;

### Step D: 5-bromo-2,3-dihydrospiro[indene-1,4'-piperidine]-3-ol

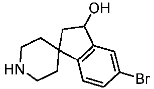

80.0 mg (0.21 mmol, 1.0 eq) tert-butyl 5-bromo-3-hydroxy-2,3-dihydrospiro[indene-1,4'-piperidine]-1'- carboxylate was dissolved in 6 mL dichloromethane, and then 1 mL (4.31 mmol, 30 eq) solution of hydrogen chloride in dioxane was added in the above reaction system. The reaction was carried out under nitrogen protection at 40 °C for 1 h. After confirming that the target product had been generated through LC-MS results, the reaction solution was concentrated directly to dryness, and a light yellow oily substance was obtained (58.0 mg, yield =98%).
LC-MS: (M+H)⁺; m/z = 283.95;

### Step E: (5-bromo-3-hydroxy-2,3-dihydrospiro[indene-1,4'-piperidine]-1'-yl)(5-fluoropyridin-2-yl) ketone

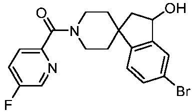

48.0 mg (0.34 mmol, 1.2 eq) 5-fluoropyridinecarboxylic acid was dissolved in 6 mL tetrahydrofuran, and then 129 mg (0.34 mmol, 1.0 eq) 2-(7-azobenzotriazole)-*N,N,N',N'-*tetramethylurea hexafluorophosphate and 0.28 mL (1.70 mmol, 6.0 eq) N,N-diisopropylethylamine was added in the reaction system. Stirring was carried out under nitrogen protection for 10 min. A solution of 80.0 mg (0.28 mmol, 1.0 eq) 5-bromo-2,3-dihydrospiro[indene-1,4'-piperidine]-3-ol dissolved in 3 mL tetrahydrofuran was added dropwise in the above reaction system. After the dropwise addition, stirring was carried out at room temparature for 2 h. After confirming that the target product had been generated through LC-MS results, the reaction solution is cooled to room temperature and quenched by adding water, and then extracted twice with ethyl acetate. The organic phases were washed with salt water, dried over anhydrous sodium sulfate, and concentrated to dryness, and then subjected to column chromatography (dichloromethane/methanol=80/1~30/1) to obtain a light yellow solid (50.0 mg, yield=44%).
LC-MS: (M+H)⁺; m/z = 406.89;

### Step F: (5-(2-cyclopropylphenyl)-3-hydroxy-2,3-dihydrospiro[indene-1,4'-piperidine]-1'-yl)(5-fluoropyridin-2-yl) ketone

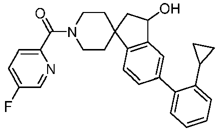

50.0 mg (0.12 mmol, 1.0 eq) (5-bromo-3-hydroxy-2,3-dihydrospiro[indene-1,4'-piperidine]-1'-yl)(5-fluoropyridin-2-yl) ketone was dissolved in 6 mL dioxane and 2mL water, and then, 23.9 mg (0.15 mmol, 1.2 eq) (2-cyclopropylphenyl) boric acid and 78.2 mg (0.15 mmol, 3.0 eq) potassium phosphate were added to the above reaction system. 18.0 mg (0.02 mmol, 0.1 eq) [1,1'-bis(diphenylphosphine)ferrocene] dichloride palladium was added under nitrogen protection, and the reaction was carried out at 100 °C for 3 h. After confirming that the target product had been generated through LC-MS results, the reaction solution is cooled to room temperature and quenched by adding water, and then extracted twice with ethyl acetate. The organic phases were washed with salt water, dried over anhydrous sodium sulfate, and concentrated to dryness. The sample was sent for preparation and purification (mobile phase A: 0.1% formic acid; mobile phase B: acetonitrile; gradient: 30% -70% B, 55 min; flow rate: 70mL/min), and then freeze-dried to obtain the product as a white solid (10.2 mg, yield=18%).
LC-MS: (M+H)⁺; m/z = 443.42;
¹H NMR (400 MHz, CDCl₃) δ 8.48 - 8.46 (m, 1H), 7.78 (dd, *J* = 8.6, 4.5 Hz, 1H), 7.56 - 7.51 (m, 2H), 7.45 (d, *J =* 7.9 Hz, 1H), 7.31 - 7.27 (m, 2H), 7.26 - 7.19 (m, 2H), 6.94 (d, *J* = 7.8 Hz, 1H), 5.38 - 5.37 (m, 1H), 4.81 - 4.80 (m, 1H), 4.09 - 4.07 (m, 1H), 3.40 - 3.38 (m, 1H), 3.13 - 3.11 (m, 1H), 2.70 - 2.59 (m, 1H), 2.21 - 1.97 (m, 3H), 1.95 - 1.87 (m, 3H), 1.77 - 1.67 (m, 1H), 0.90 - 0.85 (m, 2H), 0.76 - 0.72 (m, 2H).
¹⁹F NMR (376 MHz, CDCl₃) δ -123.84 (d, *J* = 3.9 Hz).

### Example 4: (5-fluoropyridin-2-yl)(3-hydroxy-5-(2-isopropylphenyl)-2,3-dihydrospiro[indene-1,4'-piperidine]-1'-yl) ketone

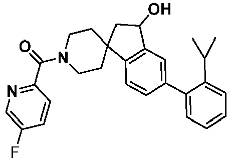

Experimental procedure referred to Step F in Example 3, but wherein (2-cyclopropylphenyl) boric acid was replaced with 2-isopropylphenyl boronic acid to synthesize the compound of Example 4.

LC-MS: (M+H)⁺; m/z = 445.17.

### Examples 5 and 6 : (5-(2-cyclopropylphenyl)-3-hydroxy-3-methyl-2,3-dihydrospiro[indene-1,3'-pyrrolidine]-1'-yl)(5-fluoropyridin-2-yl) ketone (P1 and P2)

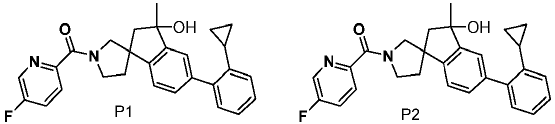

### Step A: 5-(2-cyclopropylphenyl)-3-hydroxy-3-methyl-2,3-dihydrospiro[indene-1,3'-pyrrolidine]-2'-one

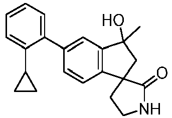

0.76 g (2.4 mmol, 1.0 eq) 5-(2-cyclopropylphenyl)spiro[indene-1,3'-pyrrolidine]-3,5'(2H)-dione was dissolved in 25 mL anhydrous tetrahydrofuran. Nitrogen replacement was carried out for three times. 4 mL (12.0 mmol, 5.0 eq, 3 M) methyl magnesium chloride was added dropwise at 0 °C, and stirring was carried out at room temperature for 2 h. After confirming that the raw materials had been completely converted and the product had been generated through LCMS, 30 mL saturated ammonium chloride solution was added thereto in order to quench the reaction, and then, extraction was carried out with dichloromethane for three times. The organic layer was washed with 30 mL saturated saline solution, dried over anhydrous sodium sulfate and evaporated under reduced pressure. The residue was subjected to column chromatography (DCM/MeOH = 0~20:1) to obtain the product (0.60 g, yield =75%).
LC-MS: (M+H)⁺; m/z = 334.21;

### Step B: 5-(2-cyclopropylphenyl)-3-methyl-2,3-dihydrospiro[indene-1,3'-pyrrolidine]-3-ol

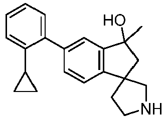

550 mg (1.65 mmol, 1.0 eq) 5-(2-cyclopropylphenyl)-3-hydroxy-3-methyl-2,3-dihydrospiro[indene-1,3'-pyrrolidine]-2'-one was dissolved in 50 mL anhydrous tetrahydrofuran, and 1.25 g (32.99 mmol, 20.0 eq) lithium aluminum hydride was slowly added dropwise at 0 °C. Stirring was carried out at 70 °C for 2 h. After confirming that the raw materials had been completely converted and the product had been generated through LCMS, the reaction solution was filtered through diatomite and washed with tetrahydrofuran. After drying over anhydrous sodium sulfate, concentrated by vacuum distillation, a reaction solution containing product was obtained.
LC-MS: (M+H)⁺; m/z=320.23;

### Step C: (5-(2-cyclopropylphenyl)-3-hydroxy-3-methyl-2,3-dihydrospiro[indene-1,3'-pyrrolidine]-1'-yl)(5-fluoropyridin-2-yl) ketone

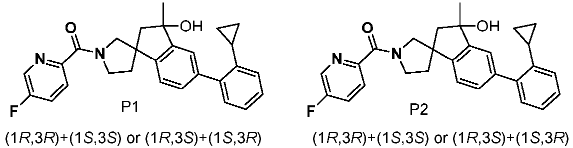

0.40 g (2.82 mmol, 1.5 eq) 5-fluoropyridin-2-carboxylic acid, 0.93 mL *N,N-*diisopropylethylamine and 1.07 g (2.82 mmol, 1.5 eq) 2-(7-azobenzotriazole)-*N,N,N',N'-*tetramethylurea hexafluorophosphate were dissolved in 50 mL tetrahydrofuran, and then stirring was carried out at room temperature for 0.5 h. The reaction solution containing 5-(2-cyclopropylphenyl)-3-methyl-2,3-dihydrospiro[indene-1,3'-pyrrolidine]-3-ol was added, and stirring was carried out at room temperature for 2 h. After confirming that the product had been generated through LCMS, 50 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min), and freeze-dring, a white solid product P1 (118 mg, yield =14%) and a white solid product P2 were obtained (135 mg, yield =16%).
Example 5 (P1) LC-MS: (M+H)⁺; m/z=443.18; ¹H NMR (400 MHz, CDCl₃) δ 8.50 - 8.40 (m, 1H), 8.11 - 8.06 (m, 1H), 7.58 - 7.44 (m, 3H), 7.34 - 7.30 (m, 2H), 7.26 - 7.22 (m, 2H), 6.96 - 6.94 (m, 1H), 4.14 - 3.87 (m, 4H), 2.44 - 2.22 (m, 5H), 1.93 - 1.83 (m, 1H), 1.29 (s, 3H), 0.88 - 0.85 (m, 2H), 0.79 - 0.75 (m, 2H).
Example 6 (P2) LC-MS: (M-OH+H)⁺; m/z=425.18 ; ¹H NMR (400 MHz, CDCl₃) δ 8.49 - 8.38 (m, 1H), 8.09 - 8.02 (m, 1H), 7.57 - 7.45 (m, 3H), 7.33 - 7.29 (m, 2H), 7.26 - 7.20 (m, 2H), 6.97 - 6.94 (m, 1H), 4.14 - 3.85 (m, 4H), 2.42 - 2.04 (m, 4H), 1.85 - 1.68 (m, 2H), 1.61 (s, 3H), 0.90 - 0.84 (m, 2H), 0.78 - 0.73 (m, 2H).

### Example 7 : (5-(2-cyclopropylphenyl)-3-hydroxy-3-(trifluoromethyl)-2,3-dihydrospiro[indene-1,3'-pyrrolidine]-1'-yl)(5-fluoropyridin-2-yl) ketone

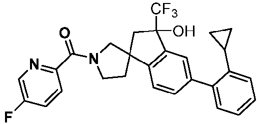

### Step A: 5-(2-cyclopropylphenyl)-3-hydroxy-3-(trifluoromethyl)-2,3-dihydrospiro[indene-1,3'-pyrrolidine]-5'-one

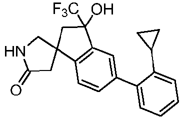

100 mg (0.31 mmol, 1.0 eq) 5-(2-cyclopropylphenyl)-3-hydroxy-3-(trifluoromethyl)-2,3-dihydrospiro[indene-1,3'-pyrrolidine]-5'-one and 270 mg (1.86 mmol, 6.0 eq) (trifluoromethyl) trimethylsilane was dissolved in 5 mL anhydrous tetrahydrofuran. Nitrogen replacement was carried out for three times. 0.3 mL tetrabutylammonium fluoride was added dropwise at 0 °C, and stirring was carried out overnight at room temperature. After confirming that the product had been generated through LCMS, the reaction solution was added dropwise to 30 mL saturated sodium bicarbonate solution for quenching, and then extraction was carried out with ethyl acetate for three times. The organic layer was washed with 30 mL saturated saline solution, dried over anhydrous sodium sulfate and evaporated under reduced pressure to obtain a crude product (103 mg, yield =86%).
LC-MS: (M+H)⁺; m/z = 388.11;

### Step B: 5-(2-cyclopropylphenyl)-3-(trifluoromethyl)-2,3-dihydrospiro[indene-1,3'-pyrrolidine]-3-ol

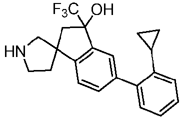

103 mg (0.27 mmol, 1.0 eq) 5-(2-cyclopropylphenyl)-3-hydroxy-3-(trifluoromethyl)-2,3-dihydrospiro[indene-1,3'-pyrrolidine]-5'-one was dissolved in 5 mL anhydrous tetrahydrofuran, and 99.0 mg (2.66 mmol, 10.0 eq) lithium aluminum hydride was added slowly at 0 °C. Stirring was carried out at 70 °C for 3 h. After confirming that the product had been generated through LCMS, the reaction solution was filtered through diatomite and washed with tetrahydrofuran. After drying over anhydrous sodium sulfate and evaporating under reduced pressure, a crude product was obtained (100 mg, yield =100%).
LC-MS: (M+H)⁺; m/z = 373.97;

### Step C: (5-(2-cyclopropylphenyl)-3-hydroxy-3-(trifluoromethyl)-2,3-dihydrospiro[indene-1,3'-pyrrolidine]-1'-yl)(5-fluoropyridin-2-yl) ketone

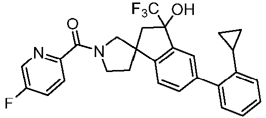

57.0 mg (0.40 mmol, 1.5 eq) 5-fluoropyridin-2-carboxylic acid, 0.08 mL N,N-diisopropylethylamine and 153 mg (0.40 mmol, 1.5 eq) 2-(7-azobenzotriazole)-N,N,N',N'-tetramethylurea hexafluorophosphate were dissolved in 10 mL tetrahydrofuran, and stirring was carried out at room temperature for 0.5 h. 100 mg (0.27 mmol, 1.0eq) 5-(2-cyclopropylphenyl)-3-(trifluoromethyl)-2,3-dihydrospiro[indene-1,3'-pyrrolidine]-3-ol was added, and stirring was carried out at room temperature for 3 h. After confirming that the product had been generated through LCMS, 5 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min), and freeze-dring, a white solid was obtained (7.00 mg, yield =5%)
LC-MS: (M+H)⁺; m/z = 497.29;
¹H NMR (400 MHz, CDCl₃) δ 8.40 - 8.38 (m, 1H), 8.10 - 8.02(m, 1H), 7.64 - 7.62 (m, 1H), 7.57 - 7.52 (m, 2H), 7.39 - 7.29 (m, 2H), 7.23 - 7.20 (m, 2H), 7.00 - 6.95 (m, 1H), 4.24 - 3.89 (m, 4H), 2.65 - 2.35 (m, 5H), 1.80 - 1.78 (m, 1H), 0.88 - 0.80 (m, 2H), 0.75 - 0.71 (m, 2H).
¹⁹F NMR (376 MHz, CDCl₃) δ -79.51, -122.46.

### Example 8: (5-fluoropyridin-2-yl) (1-hydroxy-1H-spiro[benzo[c][1,2]oxaborolane-3,3'-pyrrolidine]-1'-yl) ketone

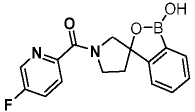

### Step A: tert-butyl 3-(2-bromophenyl)-3-hydroxypyrrolidine-1-carboxylate

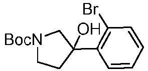

3.00 g (10.6 mmol, 1.0 eq) o-bromoiodobenzene was dissolved in 20 mL anhydrous tetrahydrofuran, and then 0.50 g (12.7 mmol, 1.2 eq) lithium chloride was added. After replacing with nitrogen, the system was cooled to -78 °C, and 6.4 mL (12.7 mmol, 1.2 eq) isopropyl magnesium chloride (2M) was slowly added dropwise to the former, while maintaining the system at -78 °C during the process. After the addition, the reaction proceeded at -78 °C for 1 h. Subsequently, 2.00 g (10.6 mmol, 1.0 eq) solution of 1-tert-butoxycarbonyl-3-pyrolidone in anhydrous tetrahydrofuran (10mL) was slowly added to the reaction system. Finally, it was naturally returned to room temperature, and the reaction proceeded for 18 h. After confirming the complete reaction through LC-MS results, 10mL saturated ammonium chloride solution was used for quenching, and then extraction was carried out with 50mL ethyl acetate for three times. The organic phases were taken, washed with sodium chloride solution, and then dried over anhydrous sodium sulfate, and evaporated under reduced pressure. The residue was subjected to column chromatography (PE/EA = 0~1:6) to obtain tert-butyl 3-(2-bromophenyl)-3-hydroxypyrrolidine-1-carboxylate (1.00 g, yield =28%).
LC-MS: (M+H-Boc)⁺; m/z = 242, 244;

### Step B: tert-butyl 1-hydroxy-1H-spiro[benzo[c][1,2]oxaborolane-3,3'-pyrrolidine]-1'-carboxylate

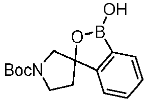

66.0 mg (0.2 mmol, 1.0 eq) tert-butyl 3-(2-bromophenyl)-3-hydroxypyrrolidine-1-carboxylate was dissolved in 5 mL 1,4-dioxane, and then 108.9 mg (0.5 mmol, 2.5 eq) bis(neopentylglycolato) diboron, 14.1 mg (0.02 mmol, 0.1 eq) [1,1'-bis(diphenylphosphine)ferrocene] dichloride palladium and 37.9 mg (0.4 mmol, 2.0 eq) potassium acetate were added to the reaction system. After replacing with nitrogen, it was heated to 80 °C, and reacted for 3 h. After confirming the complete reaction through LC-MS results, the system was returned to room temperature, and used directly for the next reaction step.
LC-MS: (M+Na)⁺; m/z = 312;

### Step C: 1H-spiro[benzo[c][1,2]oxaborolane-3,3'-pyrrolidine]-1-ol hydrochloride

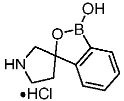

After return the tert-butyl 1-hydroxy- 1*H*-spiro[benzo[c][1,2]oxaborolane-3,3'-pyrrolidine]-1'-carboxylate reaction system to room temperature, 1mL (12 mmol, 60.0 eq) hydrochloric acid dioxane solution (4M) was added, and then the reaction was carried out under nitrogen protection at room temperature for 1 h. After confirming the complete reaction through LC-MS results, the solvent in the system was evaporated under reduced pressure to obtain 1*H-*spiro[benzo[c][1,2]oxaborolane-3,3'-pyrrolidine]-1-ol hydrochloride (21.2mg, two-step comprehensive yield =58%)
LC-MS: (M+H)⁺; m/z = 190;

### Step D: (5-fluoropyridin-2-yl)(1-hydroxy-1H-spiro[benzo[c][1,2]oxaborolane-3,3'-pyrrolidine]-1'-yl) ketone

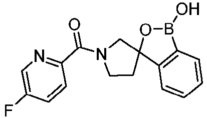

15.8mg (0.10mmol, 1.0eq) 5-fluoro-2-pyridinecarboxylic acid was dissolved in 1 mL N,N-dimethylformamide. After replacing with nitrogen, 46.9 mg (0.10 mmol, 1.1 eq) 2-(7-azobenzotriazole)-N,N,N',N'-tetramethylurea hexafluorophosphate and 29.0 mg (0.20 mmol, 2.0 eq) N,N-diisopropylethylamine were added in sequence. After stirring at room temperature for 1 min, 21.2 mg (0.10 mmol, 1.0 eq) 1*H*-spiro[benzo[c][1,2]oxaborolane-3,3'-pyrrolidine]-1-ol was added, and stirring was proceeded for 30 min. After confirming the complete reaction through LC-MS results, 10 mL saturated sodium chloride solution was used to dilute, and extraction was carried out with 10 mL ethyl acetate for three times. The organic phases were taken, dried over anhydrous sodium sulfate, and evaporated under reduced pressure. The residue was purified through column chromatography (MeOH/DCM = 0~1:30) and preparative chromatography (mobile phase A: 0.1% formic acid; mobile phase B: acetonitrile; gradient: 30% -70% B, 55 min; flow rate: 70mL/min) in sequence to obtain (5-fluoropyridin-2-yl)(1-hydroxy-1*H*-spiro[benzo[c][1,2]oxaborolane-3,3'-pyrrolidine]-1'-yl) ketone as a white solid (15.9 mg, yield =45%).
LC-MS: (M+H)⁺; m/z = 429;
¹H NMR (400 MHz, CDCl₃) δ 8.40 (dd, *J* = 56.3, 2.9 Hz, 1H), 8.07 (ddd, *J* = 10.9, 8.7, 4.6 Hz, 1H), 7.74 (t, *J* = 7.3 Hz, 1H), 7.56 - 7.46 (m, 2H), 7.45 - 7.34 (m, 2H), 5.32 (d, *J* = 43.4 Hz, 1H), 4.35 - 3.96 (m, 4H), 2.50 - 2.39 (m, 1H), 2.15 - 2.07 (m, 1H).

### Example 9: (6-(2-cyclopropylphenyl)-1-hydroxy-1H-spiro[benzo[c][1,2]oxaborolane-3,3'-pyrrolidine]-1'-yl)(5-fluoropyridin-2-yl) ketone

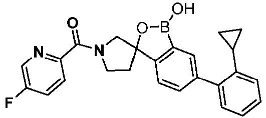

### Step A: 3-bromo-2'-cyclopropyl-[1,1'-biphenyl]-4-amine

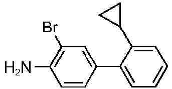

1.50 g (5.00 mmol, 1.0 eq) 2-bromo-4-iodoaniline was dissolved in 12.5 mL 1,4-dioxane, and then, 0.80 g (5.00 mmol, 1.0 eq) 2-cyclopropylphenylboronic acid, 0.30 g (0.30 mmol, 0.05 eq) tetrakis(triphenylphosphine) palladium, 1.40 g (10.1 mmol, 2.0 eq) potassium carbonate and 2.5mL water were added to the reaction system. After replacing with nitrogen, it was heated to 80 °C, reacted for 2 h. After confirming the complete reaction through LC-MS results, the system was returned to room temperature, and diluted with 20mL saturated sodium chloride solution. Then, it was extracted three times with 30mL ethyl acetate. The organic phases were taken, dried over anhydrous sodium sulfate, and evaporated under reduced pressure. The residue was subjected to column chromatography (EA/PE = 0~1:10) to obtain 3-bromo-2'-cyclopropyl-[1,1'-biphenyl]-4-amine (791 mg, yield =55%).
LC-MS: (M+H)⁺; m/z = 288; 290;

### Step B: 3'-bromo-2-cyclopropyl-4'-iodo-1,1'-biphenyl

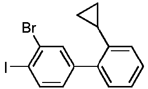

733 mg (2.50 mmol, 1.0 eq) 3-bromo-2'-cyclopropyl-[1,1'-biphenyl]-4-amine was dissolved in 4 mL anhydrous tetrahydrofuran, and then 8mL concentrated hydrochloric acid was added while stirring. After cooling the system to 0 °C, 263 mg (3.80 mmol, 1.5 eq) sodium nitrite aqueous solution (0.9 mL) was slowly added dropwise to the former, while maintaining the system at 0-5 °C during the process. After the addition, the reaction proceeded at 0 °C for 30 min. Subsequently, 1.69 g (10.2 mmol, 4.0 eq) potassium iodide was slowly added to the reaction system. Finally, the reaction was carried out at 0 °C for 30 min. After confirming the complete reaction through LC-MS results, 10mL 10 % sodium sulfite aqueous solution was used for quenching, and then extraction was carried out with 50 mL ethyl acetate for three times. The organic phases were taken, washed with saturated sodium carbonate solution and saturated sodium chloride solution in sequence, and then dried over anhydrous sodium sulfate, and evaporated under reduced pressure. The residue was subjected to column chromatography (PE) to obtain 3'-bromo-2-cyclopropyl-4'-iodo-1,1'-biphenyl (641 mg, yield =63%).

¹H NMR (400 MHz, CDCl₃) δ 7.79 (dd, *J* = 7.1, 3.1 Hz, 1H), 7.64 (dd, *J* = 7.1, 2.2 Hz, 1H), 7.22 - 7.07 (m, 3H), 7.01 - 6.99 (m, 1H), 6.87 - 6.86 (m, 1H), 1.73 - 1.71 (m, 1H), 0.80 - 0.74 (m, 2H), 0.64 - 0.55 (m, 2H).

### Step C: tert-butyl 3-(3-bromo-2'-cyclopropyl-[1,1'-biphenyl]-4-yl)-3-hydroxypyrrolidine-1-carboxylate

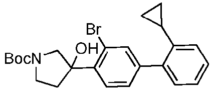

389 mg (1.00 mmol, 1.0 eq) 3'-bromo-2-cyclopropyl-4'-iodo-1,1'-biphenyl was dissolved in 6 mL anhydrous tetrahydrofuran, and then 53.7 mg (1.30 mmol, 1.3 eq) lithium chloride was added. After replacing with nitrogen, the system was cooled to -78 °C, and 0.6 mL (1.30 mmol, 1.3 eq) isopropyl magnesium chloride (2M) was slowly added dropwise to the former, while maintaining the system at -78 °C during the process. After the addition, the reaction proceeded at -78 °C for 1 h. Subsequently, 253 mg (1.40 mmol, 1.4 eq) solution of 1-tert-butoxycarbonyl-3-pyrolidone in anhydrous tetrahydrofuran (4mL) was slowly added to the reaction system. Finally, it was naturally returned to room temperature, and the reaction proceeded for 18 h. After confirming the complete reaction through LC-MS results, 5 mL saturated ammonium chloride solution was used for quenching, and then extraction was carried out with 50 mL ethyl acetate for three times. The organic phases were taken, washed with sodium chloride solution, and then dried over anhydrous sodium sulfate, and evaporated under reduced pressure. The residue was subjected to column chromatography (PE/EA = 0~1:6) to obtain tert-butyl 3-(3-bromo-2'-cyclopropyl-[1,1'-biphenyl]-4-yl)-3-hydroxypyrrolidine-1-carboxylate (107 mg, yield =24%).
LC-MS: (M+H-Boc-OH)⁺; m/z = 340; 342;

### Step D: tert-butyl 6-(2-cyclopropylphenyl)-1-hydroxy-1H-spiro[benzo[c][1,2]oxaborolane-3,3'-pyrrolidine]-1'-carboxylate

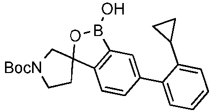

107 mg (0.20 mmol, 1.0 eq) tert-butyl 3-(3-bromo-2'-cyclopropyl-[1,1'-biphenyl]-4-yl)-3-hydroxypyrrolidine-1-carboxylate was dissolved in 6 mL 1,4-dioxane, and then 132 mg (0.60 mmol, 2.5 eq) bis(neopentylglycolato) diboron, 17.1 mg (0.02 mmol, 0.1 eq) [1,1'-bis(diphenylphosphine)ferrocene] dichloride palladium and 45.7 mg (0.50 mmol, 2.eq) potassium acetate were added to the reaction system. After replacing with nitrogen, it was heated to 80 °C, and reacted for 3 h. After confirming the complete reaction through LC-MS results, the system was returned to room temperature, and used directly for the next reaction step.
LC-MS: (M+Na)⁺; m/z = 428;

### Step E: 6-(2-cyclopropylphenyl)-1H-spiro[benzo[c][1,2]oxaborolane-3,3'-pyrrolidine]-1-ol

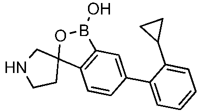

After return the tert-butyl 6-(2-cyclopropylphenyl)-1-hydroxy-1*H-*spiro[benzo[c][1,2]oxaborolane-3,3'-pyrrolidine]-1'-carboxylate reaction system to room temperature, 3 mL (12 mmol, 60.0 eq) hydrochloric acid dioxane solution (4M) was added, and then the reaction was carried out under nitrogen protection at room temperature for 2 h. After confirming the complete reaction through LC-MS results, the system solvent was evaporated under reduced pressure to obtain 6-(2-cyclopropylphenyl)-1*H*-spiro[benzo[c][1,2]oxaborolane-3,3'-pyrrolidine]-1-ol (62.2mg, two-step comprehensive yield =88%)
LC-MS: (M+H)⁺; m/z = 306;

### Step F: (6-(2-cyclopropylphenyl)-1-hydroxy-1H-spiro[benzo[c][1,2]oxaborolane-3,3'-pyrrolidine]-1'-yl)(5-fluoropyridin-2-yl) ketone

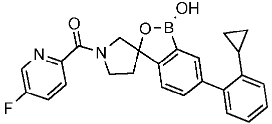

28.8 mg (0.20 mmol, 1.0 eq) 5-fluoro-2-pyridinecarboxylic acid was dissolved in 1 mL N,N-dimethylformamide. After replacing with nitrogen, 85.3 mg (0.20 mmol, 1.1 eq) 2-(7-azobenzotriazole)-*N*,*N*,*N*',*N*'-tetramethylurea hexafluorophosphate and 52.7 mg (0.40 mmol, 2.0 eq) *N,N*-diisopropylethylamine were added in sequence. After stirring at room temperature for 1 min, 62.2 mg (0.20 mmol, 1.0 eq) 6-(2-cyclopropylphenyl)-1*H*-spiro[benzo[c][1,2]oxaborolane-3,3'-pyrrolidine]-1-ol was added, and stirring was proceeded for 30 min. After confirming the complete reaction through LC-MS results, 10 mL saturated sodium chloride solution was used to dilute, and extraction was carried out with 10 mL ethyl acetate for three times. The organic phases were taken, dried over anhydrous sodium sulfate, and evaporated under reduced pressure. The residue was purified through column chromatography (MeOH/DCM = 0~1:50) and preparative chromatography (mobile phase A: 0.1% trifluoroacetic acid; mobile phase B: acetonitrile; gradient: 30% -70% B, 55 min; flow rate: 70mL/min) in sequence to obtain (6-(2-cyclopropylphenyl)-1-hydroxy-1*H*-spiro[benzo[c][1,2]oxaborolane-3,3'-pyrrolidine]-1'-yl)(5-fluoropyridin-2-yl) ketone as a white solid (11.9 mg, yield =14%).
LC-MS: (M+H)⁺; m/z = 429 ;
¹H NMR (400 MHz, CDCl₃) δ 8.54 - 8.27 (m, 1H), 8.07 (dd, *J* = 9.4, 4.6 Hz, 1H), 7.79 (d, *J* = 7.4 Hz, 1H), 7.64 (dd, *J* = 7.9, 1.6 Hz, 1H), 7.55 - 7.47 (m, 1H), 7.42 (dd, *J* = 14.9, 7.8 Hz, 1H), 7.29 (s, 1H), 7.23 - 7.19 (m, 2H), 6.98 - 6.92 (m, 1H), 4.40 - 4.01 (m, 4H), 2.59 - 2.44 (m, 1H), 2.27 - 2.13 (m, 1H), 2.02 - 2.00 (m, 1H), 0.88 - 0.83 (m, 2H), 0.72 - 0.71 (m, 2H).

### Example 10 : (5-chloropyridin-2-yl)(6-(2-cyclopropylphenyl)-1-hydroxy-1H-spiro[benzo[c][1,2]oxaborolane-3,3'-pyrrolidine]-1'-yl) ketone

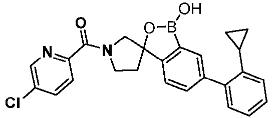

Experimental procedure referred to Step F in Example 9, but wherein 5-fluoro-2-pyridinecarboxylic acid was replaced with 5-chloro-2-pyridine carboxylic acid to synthesize the compound of Example 10.

LC-MS: (M+H)⁺; m/z = 445.09.

### Example 11 : (5-(2-cyclopropylphenyl)-3-hydroxy-2,3-dihydrospiro[indene-1,3'-pyrrolidine]-1'-yl-3,5',5'-d₃)(5-fluoropyridin-2-yl) ketone

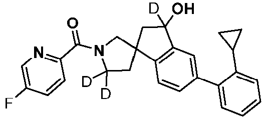

### Step A: 5-(2-cyclopropylphenyl)-2,3-dihydrospiro[indene-1,3'-pyrrolidine]-3,5',5'-d₃-3-ol

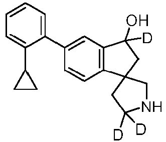

100 mg (0.31 mmol, 1.0 eq) 5-(2-cyclopropylphenyl)-2,3-dihydrospiro[indene-1,3'-pyrrolidine]-3-ol was dissolved in 5 mL anhydrous tetrahydrofuran, and then, 120 mg (3.70 mmol, 12.0 eq) lithium aluminum deuteride was slowly added at 0 °C. Stirring was carried out at 70 °C for 3 h. After confirming that the product had been generated through LCMS, the reaction solution was filtered through diatomite and washed with tetrahydrofuran. After drying over anhydrous sodium sulfate and evaporating under reduced pressure, a crude product was obtained (100 mg, yield =104%).
LC-MS: (M+H)⁺; m/z = 309.19;

### Step B: (5-(2-cyclopropylphenyl)-3-hydroxy-2,3-dihydrospiro[indene-1,3'-pyrrolidine]-1'-yl-3,5',5'-d₃)(5-fluoropyridin-2-yl) ketone

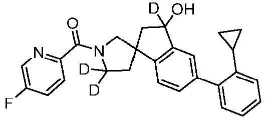

77.0 mg (0.48 mmol, 1.5 eq) 5-chloropyridin-2-carboxylic acid, 0.16 mL N,N-diisopropylethylamine and 187 mg (0.48 mmol, 1.5 eq) 2-(7-azobenzotriazole)-*N*,*N*,*N'*,*N*'-tetramethylurea hexafluorophosphate was dissolved in 10 mL tetrahydrofuran, and then stirring was carried out at room temperature for 0.5 h. 100 mg (0.32 mmol, 1.0 eq) 5-(2-cyclopropylphenyl)-2,3-dihydrospiro[indene-1,3'-pyrrolidine]-3,5',5'-*d₃*-3-ol was added, and then stirring was carried out at room temperature for 3 h. After confirming that the product had been generated through LCMS, 5 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min), a white solid was obtained (14.0 mg, yield =10%).
LC-MS: (M+H)⁺; m/z = 432.19;
¹H NMR (400 MHz, CDCl₃) δ 8.47 - 8.30 (m, 1H), 8.05 - 8.01 (m, 1H), 7.54 - 7.42 (m, 3H), 7.32 - 7.25 (m, 2H), 7.21 - 7.14 (m, 2H), 6.93 - 6.85 (m, 1H), 4.14 - 3.77 (m, 2H), 2.66 - 2.00 (m, 5H), 1.92 - 1.81 (m, 1H), 0.87 - 0.82 (m, 2H), 0.74 - 0.70 (m, 2H).
¹⁹F NMR (376 MHz, CDCl₃) δ -123.19 (d, *J* = 24.8 Hz).

### Example 12 : (5-(2-cyclopropylphenyl)-3-hydroxy-2,3-dihydrospiro[indene-1,3'-pyrrolidine]-1'-yl-3-d)(5-fluoropyridin-2-yl) ketone

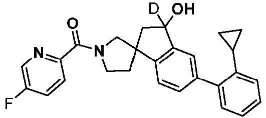

### Step A: 5-(2-cyclopropylphenyl)-3-hydroxy-2,3-dihydrospiro[indene-1,3'-pyrrolidine]-5'-one-3-d

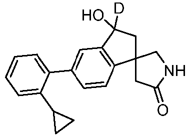

150 mg (0.47 mmol, 1.0 eq) 5-bromospiro[indene-1,3'-pyrrolidine]-3,5'(2*H*)-dione was dissolved in 5 mL anhydrous tetrahydrofuran, and then, 110 mg (0.57 mmol, 1.2 eq.) sodium borodeuteride was slowly added at 0 °C. Stirring was carried out at room temperature for 2 h. After confirming that the product had been generated through LCMS, the reaction solution was quenched using water, dried over anhydrous sodium sulfate and evaporated under reduced pressure to obtain a crude product (148 mg, yield =97%).
LC-MS: (M+H)⁺; m/z = 321.10;

### Step B: 5-(2-cyclopropylphenyl)-2,3-dihydrospiro[indene-1,3'-pyrrolidine]-3-d-3-ol

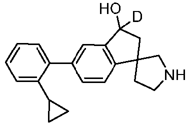

148 mg (0.46 mmol, 1.0 eq) 5-bromospiro[indene-1,3'-pyrrolidine]-3,5'(2*H*)-dione was dissolved in 5 mL anhydrous tetrahydrofuran, and then, 263 mg (6.92 mmol, 15.0 eq) lithium aluminum hydride was slowly added at 0 °C. Stirring was carried out at 70 °C for 3 h. After confirming that the product had been generated through LCMS, the reaction solution was filtered through diatomite and washed with tetrahydrofuran. After drying over anhydrous sodium sulfate and evaporating under reduced pressure, a crude product was obtained (142 mg, yield =100%).
LC-MS: (M+H)⁺; m/z = 307.11;

### Step C: (5-(2-cyclopropylphenyl)-3-hydroxy-2,3-dihydrospiro[indene-1,3'-pyrrolidine]-1'-yl-3-d)(5-fluoropyridin-2-yl) ketone

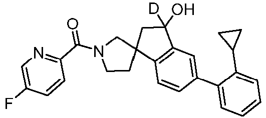

103 mg (0.73 mmol, 1.5 eq) 5-fluoropyridin-2-carboxylic acid, 0.24 mL *N,N-*diisopropylethylamine and 279 mg (0.73mmol, 1.5eq) 2-(7-azobenzotriazole)-*N*,*N*,*N*',*N*'-tetramethylurea hexafluorophosphate was dissolved in 5 mL tetrahydrofuran, and then stirring was carried out at room temperature for 0.5 h. 142 mg (0.43mmol, 1.0eq) 5-(2-cyclopropylphenyl)-2,3-dihydrospiro[indene-1,3'-pyrrolidine]-3-ol was added, and then stirring was carried out at room temperature for 1.5 h. After confirming that the product had been generated through LCMS, 10 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min), a white solid was obtained (12.0 mg, yield =5.7%).
LC-MS: (M+H)⁺; m/z = 430.06;
¹H NMR (400 MHz, CDCl₃) δ 8.43 (d, *J* = 38.7 Hz, 1H), 8.06 - 8.03 (m, 1H), 7.56 - 7.43 (m, 3H), 7.32 - 7.26 (m, 2H), 7.24 - 7.19 (m, 2H), 6.92 (dd, *J* = 7.9, 4.2 Hz, 1H), 4.24 - 3.71 (m, 4H), 2.67 - 2.53 (m, 1H), 2.31 - 2.01 (m, 4H), 1.92 - 1.82 (m, 1H), 0.89 - 0.82 (m, 2H), 0.74 - 0.70 (m, 2H).
¹⁹F NMR (376 MHz, CDCl₃) δ -122.82 (d, *J* = 10.5 Hz).

### Example 13 : (5-(2-cyclopropylphenyl)-3-hydroxy-2,3-dihydrospiro[indene-1,3'-pyrrolidine]-1'-yl)(5-hydroxy-6-methylpyridin-2-yl) ketone

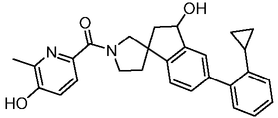

### Step A: Synthesis of 5-hydroxy-6-methylpyridinic acid

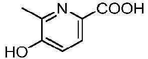

0.97 g (5.16 mmol, 1.0 eq) 6-bromo-2-methylpyridin-3-ol was dissolved in 30 mL N, N-dimethylformamide, and then, under an ice water bath, 2.37 g (51.6 mmol, 10.0 eq) formic acid, 1.57 g (15.5 mmol, 3.0 eq) triethylamine, 23.2 mg (0.10 mmol, 0.02 eq) palladium acetate and 0.03 g (0.15 mmol, 0.03 eq) 4,5-diphenylphosphine-9,9-dimethyloxanthracene were added. Carbon monoxide replacement was carried out for three times, and stirring was carried out overnight at 80 °C. After extraction, most of the product was in the aqueous phase. After preparation and purification (mobile phase A: 0.1% formic acid; mobile phase B: acetonitrile; gradient: 30%-70% B, 55 min; flow rate: 70mL/min) and freeze-dring, the product was obtained (0.78 g, yield =99%).
LC-MS: (M-H)⁻; m/z = 151.87;

### Step B: (5-(2-cyclopropylphenyl)-3-hydroxy-2,3-dihydrospiro[indene-1,3'-pyrrolidine]-1'-yl)(5-hydroxy-6-methylpyridin-2-yl) ketone

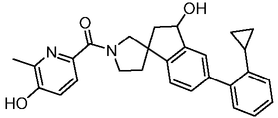

150 mg (0.98 mmol, 3.0 eq) 5-hydroxy-6-methylpyridinic acid, 0.16 mL *N,N-*diisopropylethylamine and 126 mg (0.98 mmol, 3.0 eq) 2-(7-azobenzotriazole)-*N*,*N*,*N*'*,N*'-tetramethylurea hexafluorophosphate were dissolved in 10 mL tetrahydrofuran, and then stirring was carried out at room temperature for 0.5 h. 100 mg (0.33 mmol, 1.0 eq) 5-(2-cyclopropylphenyl)-2,3-dihydrospiro[indene-1,3'-pyrrolidine]-3-ol was added, and then stirring was carried out at room temperature for 3 h. After confirming that the product had been generated through LCMS, 5 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min) and freeze-dring, a white solid was obtained (5.0 mg, yield =3.4%).
LC-MS: (M+H)⁺; m/z = 441.21;
¹H NMR (400 MHz, CDCl₃) δ 7.67 (dd, *J* = 11.2, 8.3 Hz, 1H), 7.52 (d, *J =* 6.2 Hz, 1H), 7.46 (dd, *J* = 7.9, 1.7 Hz, 1H), 7.35 - 7.30 (m, 2H), 7.25 - 7.21 (m, 2H), 7.11 (dd, *J* = 14.0, 8.3 Hz, 1H), 6.96 - 6.93 (m, 1H), 5.40 - 5.36 (m, 2H), 4.17 - 3.80 (m, 4H), 2.59 - 2.01 (m, 8H), 1.93 - 1.87 (m, 1H), 0.90 - 0.85 (m, 2H), 0.76 - 0.72 (m, 2H).

### Example 14 : (5-chloropyridin-2-yl)(5-(2-cyclopropylphenyl)-3-hydroxy-2,3-dihydrospiro[indene-1,3'-pyrrolidine]-1'-yl) ketone

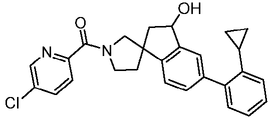

77 mg (0.49 mmol, 1.5 eq) 5-fluoropyridin-2-carboxylic acid, 0.16 mL *N,N-*diisopropylethylamine and 187 mg (0.49 mmol, 1.5 eq) 2-(7-azobenzotriazole)-*N*,*N*,*N*'*,N*'-tetramethylurea hexafluorophosphate were dissolved in 10 mL anhydrous tetrahydrofuran, and then stirring was carried out at room temperature for 0.5 h. 100 mg (0.33 mmol, 1.0 eq) 5-(2-cyclopropylphenyl)-2,3-dihydrospiro[indene-1,3'-pyrrolidine]-3-ol was added, and then stirring was carried out at room temperature for 3 h. After confirming that the product had been generated through LCMS, 20 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min) and freeze-dring, a white solid was obtained (32.0 mg, yield =22%).
LC-MS: (M+H)⁺; m/z = 445.08 ;
¹H NMR (400 MHz, CDCl₃) δ 8.61 - 8.50 (m, 1H), 7.99 - 7.95 (m, 1H), 7.84 - 7.78 (m, 1H), 7.55 - 7.51 (m, 1H), 7.47 - 7.44 (m, 1H), 7.34 - 7.29 (m, 2H), 7.26 - 7.19 (m, 2H), 6.94 (dd, *J=* 7.7, 4.1 Hz, 1H), 5.41 - 5.35 (m, 1H), 4.26 - 3.80 (m, 4H), 2.30 - 2.20 (m, 5H), 1.93 - 1.85 (m, 1H), 0.89 - 0.84 (m, 2H), 0.76 - 0.72 (m, 2H).

### Example 15 : (5-(2-cyclopropylphenyl)-3-hydroxy-2,3-dihydrospiro[indene-1,3'-pyrrolidine]-1'-yl)(5-fluoropyridin-2-yl) ketone

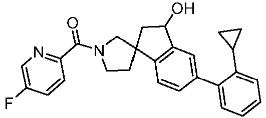

291 mg (2.06 mmol, 1.5 eq) 5-fluoropyridin-2-carboxylic acid, 0.68 mL *N,N-*diisopropylethylamine and 784 mg (2.06 mmol, 1.5 eq) 2-(7-azobenzotriazole)-*N*,*N*,*N*',*N*'-tetramethylurea hexafluorophosphate were dissolved in 65 mL tetrahydrofuran, and then stirring was carried out at room temperature for 0.5 h. 420 mg (1.37 mmol, 1.0 eq) 5-(2-cyclopropylphenyl)-2,3-dihydrospiro[indene-1,3'-pyrrolidine]-3-ol was added, and then stirring was carried out at room temperature for 1.5 h. After confirming that the product had been generated through LCMS, 50 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min) and freeze-dring, a white solid was obtained (83.0 mg, yield =15%).
LC-MS: (M+H)⁺; m/z = 429.20;
¹H NMR (400 MHz, CDCl₃) δ 8.49 - 8.38 (m, 1H), 8.09 - 8.04 (m, 1H), 7.56 - 7.44 (m, 3H), 7.34 - 7.30 (m, 2H), 7.24 - 7.21 (m, 2H), 6.95 (dd, *J* = 7.8, 4.2 Hz, 1H), 5.41 - 5.30 (m, 1H), 4.17 - 3.83 (m, 4H), 2.29 - 2.03 (m, 5H), 1.94-1.85 (m, 1H), 0.90 - 0.84 (m, 2H), 0.76 - 0.72 (m, 2H).

### Example 16 : (5-fluoropyridin-2-yl)(3-hydroxy-5-(2-isopropylphenyl)-2,3-dihydrospiro[indene-1,3'-pyrrolidine]-1'-yl) ketone

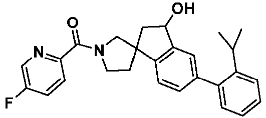

23.0 mg (0.16 mmol, 1.0 eq) 5-fluoropyridin-2-carboxylic acid, 74.2 mg (0.41 mmol, 1.2 eq) 2-(7-azobenzotriazole)-N,N,N',N'-tetramethylurea hexafluorophosphate, 63.0 mg (0.48 mmol, 3.0 eq) N,N-diisopropylethylamine and 3mL tetrahydrofuran were added to a 25 mL reaction flask. Under nitrogen protection, the reaction solution was stirred at room temperature for 10 min, and then 50.0 mg (0.16 mmol, 1.0 eq) 5-(2-isopropylphenyl)-2,3-dihydrospiro[indene-1,3'-pyrrolidine]-3-ol was weighed and dissolved in 3 mL anhydrous tetrahydrofuran, and then was slowly added to the reaction solution. Stirring was carried out at room temperature for 1 h. A complete reaction of the raw materials was confirmed through TLC Plate (DCM : MeOH=20:1). The reaction solution was concentrated to dryness, and sent for preparation and purification (mobile phase A: 0.1% formic acid; mobile phase B: acetonitrile; gradient: 30% -70% B, 55 min; flow rate: 70mL/min), and then freeze-dried to obtain a white solid pure product (11.2 mg, yield =16%).
LC-MS: (M+H)⁺; m/z = 431.15;
¹H NMR (400 MHz, CDCl₃) δ 8.47 - 8.37 (m, 1H), 8.07 - 8.03 (m, 1H), 7.55 - 7.47 (m, 1H), 7.40 - 7.33 (m, 3H), 7.0 - 7.26 (m, 2H), 7.21 - 7.13 (m, 2H), 5.37 - 5.29 (m, 1H), 4.25 - 3.79 (m, 4H), 3.11 - 3.00 (m, 1H), 2.69 - 2.37 (m, 2H), 2.28 - 2.18 (m, 1H), 2.16 - 2.00 (m, 1H), 1.89 - 1.83 (m, 1H), 1.19 (d, *J* = 6.8 Hz, 6H).
¹⁹F NMR (376 MHz, CDCl₃) δ -123.29.

### Example 17 : (2-chlorothiazol-4-yl)(5-(2-cyclopropylphenyl)-3-hydroxy-2,3-dihydrospiro[indene-1,3'-pyrrolidine]-1'-yl) ketone

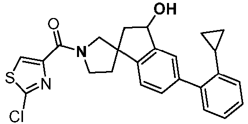

Experimental procedure referred to Example 15, but wherein 5-fluoropyridin-2-carboxylic acid was replaced with 2-chlorothiazole-4-carboxylic acid to synthesize, prepare and purify the compound of Example 17.
LC-MS: (M+H)⁺; m/z = 451.08;
¹H NMR (400 MHz, CDCl₃) δ 8.08 - 8.04 (m, 1H), 7.54 - 7.48 (m, 1H), 7.46 - 7.41 (m, 1H), 7.32 - 7.26 (m, 2H), 7.25 - 7.21 (d, *J* = 6.1 Hz, 2H), 6.92 (d, *J* = 7.9 Hz, 1H), 5.37 (s, 1H), 4.33 - 3.75 (m, 4H), 2.66 - 2.04 (m, 4H), 2.03 - 1.98 (m, 1H), 1.90 - 1.86 (m, 1H), 0.88 - 0.84 (m, 2H), 0.75 - 0.71 (m, 2H).

### Example 18 : (2-chlorothiazol-4-yl)(3-hydroxy-5-(2-isopropylphenyl)-2,3-dihydrospiro[indene-1,3'-pyrrolidine]-1'-yl) ketone

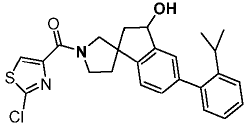

26.7 mg (0.16 mmol, 1.0 eq) 2-chlorothiazol-4-carboxylic acid, 74.2 mg (0.41 mmol, 1.2 eq) 2-(7-azobenzotriazole)-N,N,N',N'-tetramethylurea hexafluorophosphate, 63.0 mg (0.48 mmol, 3.0eq) N,N-diisopropylethylamine and 3mL tetrahydrofuran were added to a 25 mL reaction flask. Under nitrogen protection, the reaction solution was stirred at room temperature for 10 min, and then 50.0 mg (0.16 mmol, 1.0 eq) 5-(2-isopropylphenyl)-2,3-dihydrospiro[indene-1,3'-pyrrolidine]-3-ol was weighed and dissolved in 3 mL tetrahydrofuran, and then was slowly added to the reaction solution. Stirring was carried out at room temperature for 1 h. A complete reaction of the raw materials was confirmed through TLC Plate (DCM/MeOH=20/1). The reaction solution was concentrated to dryness, and sent for preparation and purification (mobile phase A: 0.1% formic acid; mobile phase B: acetonitrile; gradient: 30% -70% B, 55 min; flow rate: 70mL/min), and then freeze-dried to obtain a white solid pure product (5.11 mg, yield =7%).
LC-MS: (M-OH)⁺; m/z = 435.07;
¹H NMR (400 MHz, CDCl₃) δ 8.08 - 8.04 (m, 1H), 7.45 - 7.28 (m, 4H), 7.26 - 7.11 (m, 3H), 5.36 - 5.33 (m, 1H), 4.37 - 3.73 (m, 4H), 3.12 - 3.00 (m, 1H), 2.65 - 1.99 (m, 5H), 1.18 (d, *J* = 6.8 Hz, 6H).

### Example 19 : (5-(2-cyclopropylphenyl)-3-hydroxy-2,3-dihydrospiro[indene-1,3'-pyrrolidine]-1'-yl)(6-cyclopropylpyridin-2-yl) ketone

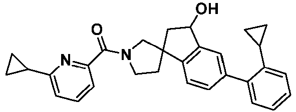

117.4 mg (0.72 mmol, 1.5 eq) 6-cyclopropylpyridine carboxylic acid, 0.24 mL *N,N-*diisopropylethylamine and 279 mg (0.72 mmol, 1.5 eq) 2-(7-azobenzotriazole)-*N*,*N*,*N*'*,N*'-tetramethylurea hexafluorophosphate were dissolved in 5 mL tetrahydrofuran, and then stirring was carried out at room temperature for 0.5 h. 150.0mg (0.48mmol, 1.0eq) 5-(2-cyclopropylphenyl)-2,3-dihydrospiro[indene-1,3'-pyrrolidine]-3-ol was added, and then stirring was carried out at room temperature for 3 h. After confirming that the product had been generated through LCMS, 5 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min) and freeze-dring, a white solid was obtained (53.0 mg, yield =24%).
LC-MS: (M+H)⁺; m/z = 451.19;
¹H NMR (400 MHz, CDCl₃) δ 7.72 - 7.59 (m, 2H), 7.52 - 7.48 (m, 1H), 7.46 - 7.40 (m, 1H), 7.34 - 7.27 (m, 1H), 7.25 - 7.15 (m, 4H), 6.92 (dd, *J* = 7.8, 4.0 Hz, 1H), 5.39 - 5.27 (m, 1H), 4.21 - 3.77 (m, 4H), 2.68 - 2.51 (m, 1H), 2.41 - 2.14 (m, 2H), 2.11 - 2.00 (m, 2H), 1.91 - 1.83 (m, 2H), 1.08 - 0.99 (m, 2H), 0.96 - 0.92 (m, 2H), 0.89 - 0.82 (m, 2H), 0.77 - 0.69 (m, 2H).

### Example 20 : (6-cyclopropylpyridin-2-yl)(3-hydroxy-5-(2-isopropylphenyl)-2,3-dihydrospiro[indene-1,3'-pyrrolidine]-1'-yl) ketone

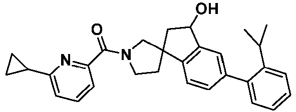

26.6 mg (0.16 mmol, 1.0 eq) 6-cyclopropylpyridine carboxylic acid, 74.2 mg (0.41 mmol, 1.2 eq) 2-(7-azobenzotriazole)-N,N,N',N'-tetramethylurea hexafluorophosphate, 63.0 mg (0.48 mmol, 3.0 eq) N,N-diisopropylethylamine and 3mL tetrahydrofuran were added to a 25 mL reaction flask. Under nitrogen protection, the reaction solution was stirred at room temperature for 10 min, and then, 50.0 mg (0.16mmol, 1.0eq) 5-(2-isopropylphenyl)-2,3-dihydrospiro[indene-1,3'-pyrrolidine]-3-ol was weighed and dissolved in 3 mL tetrahydrofuran, and then was slowly added to the reaction solution. Stirring was carried out at room temperature for 1 h. A complete reaction of the raw materials was confirmed through TLC Plate (DCM/MeOH=20/1). The reaction solution was concentrated to dryness, and sent for preparation and purification (mobile phase A: 0.1% formic acid; mobile phase B: acetonitrile; gradient: 30% -70% B, 55 min; flow rate: 70mL/min) to obtain a yellow oily pure product (7.93 mg, yield =10%).
LC-MS: (M+H)⁺; m/z = 453.16;
¹H NMR (400 MHz, CDCl₃) δ 7.84 - 7.52 (m, 3H), 7.41-7.28 (m, 4H), 7.24 - 7.13 (m, 3H), 5.42 - 5.27 (m, 1H), 4.17 - 3.80 (m, 4H), 3.07-2.97 (m, 1H), 2.49 - 2.06 (m, 6H), 1.20 (d, *J* = 6.6 Hz, 6H), 1.07 - 0.94 (m, 4H).

The stereoisomers of the compounds in Examples 15, 16, 17, 18, 19, and 20 were separated using supercritical fluid chromatography (SFC) to prepare Examples listed in Table 1.

### Analysis conditions:

instrument: WATERS 150 preparative SFC (SFC-26)
column: ChiralCel OD, 250×30mm I.D., 10µm
pressure: 100 bar
Column temperature: 38°C
wavelength: 220nm
mobile phase A: CO₂; mobile phase B: methanol; gradient: 30%B, 9.5 min; flow rate:: 150mL/min

**Table 1: Examples 21^{~}44**

| Exa mpl e No. | Structure | Compound Name | Spectral Data |
|---|---|---|---|
| 21 | 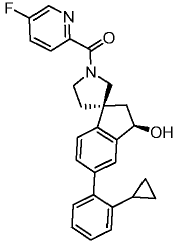 | ((1*S,*3*R*)-5-(2-cyclopropylphenyl)-3-hydroxy-2,3-dihydrospiro[indene-1, 3'-pyrrolidine]-1'-yl)(5-fluoropyridin-2-yl) ketone | LC-MS: (M+H)⁺; m/z=429. 20; |
| | | | ¹H NMR (400 MHz, CDCl₃) δ 8.42 (dd, *J* = 34.2, 2.8 Hz, 1H), 8.04 - 8.03 (m, 1H), 7.54 - 7.42 (m, 3H), 7.31 - 7.24 (m, 2H), 7.23 - 7.18 (m, 2H), 6.92 (dd, *J* = 7.6, 4. 1 Hz, 1H)., 5.38 - 5. 32 (m, 1H), 4.22 - 3.84 (m, 4H), 2.55 - 2.46 (m, 1H), 2.27 - 1.98 (m, 4H), 1.92 - 1.83 (m, 1H), 0.88 - 0.82 (m, 2H), 0.74 - 0.70 (m, 2H). |
| | | | ¹⁹F NMR (376 MHz, CDCl₃) δ -123.30 (d, *J* = 20. 9 Hz). |
| 22 | 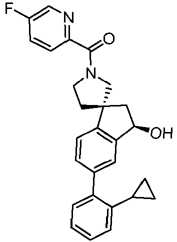 | ((1*R*,3*R*)-5-(2-cyclopropylphenyl)-3-hydroxy-2,3-dihydrospiro[indene-1, 3'-pyrrolidine]-1'-yl)(5-fluoropyridin-2-yl) ketone | LC-MS: (M+H)⁺; m/z=429. 20; |
| | | | ¹H NMR (400 MHz, CDCl₃) δ 8.42 (dd, *J* = 41.5, 2.8 Hz, 1H), 8.05 - 8.03 (m, 1H), 7.54 - 7.43 (m, 3H), 7.32 - 7.25 (m, 2H), 7.22 - 7.18 (m, 2H), 6.92 (dd, *J* = 7.7, 4.3 Hz, 1H), 5.40 - 5.32 (m, 1H), 4.25 - 3.78 (m, 4H), 2.61 - 2.59 (m, 1H), 2.41 - 2.39 (m, 1H), 2.25 - 2. 16 (m, 1H), 2.09 - 2.07 (m, 1H), 2.01 - 1.96 (m, 2H), 1.92 - 1.83 (m, 1H), 0.88 - 0.82 (m, 2H), 0.74 - 0.70 (m, 2H). |
| | | | ¹⁹F NMR (376 MHz, CDCl₃) δ -123.29 (d, *J* = 25.0 Hz). |
| 23 | 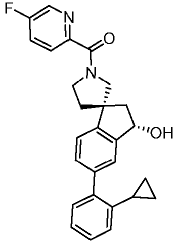 | ((1*R*,3*S*)-5-(2-cyclopropylphenyl)-3-hydroxy-2,3-dihydrospiro[indene-1,3'-pyrrolidine]-1'-yl)(5-fluoropyridin-2-yl) ketone | LC-MS: (M+H)⁺; m/z=429. 20; |
| | | | ¹H NMR (400 MHz, CDCl₃) δ 8.41 (dd, *J* = 41.6, 2.8 Hz, 1H), 8.04 (ddd, *J* = 8.8, 4.6, 2.8 Hz, 1H), 7.54 - 7.46 (m, 2H), 7.44 (d, *J* = 7.8, 1H), 7.32 - 7.25 (m, 2H), 7.22 - 7. 17 (m, 2H), 6.92 (dd, *J* = 7.6, 4.3 Hz, 1H), 5.36 - 5.32 (m, 1H), 4.24 - 3.78 (m, 4H), 2.61 - 2.59 (m, 1H), 2.41 - 2.39 (m, 1H), 2. 25 - 2. 16 (m, 1H), 2. 14 - 2.01 (m, 2H), 1. 92-1. 83 (m, 1H), 0.88 - 0.82 (m, 2H), 0.74 - 0.70 (m, 2H). ¹⁹F NMR (376 MHz, CDCl₃) δ -123.27 (d, *J* = 24. 4 Hz). |
| 24 | 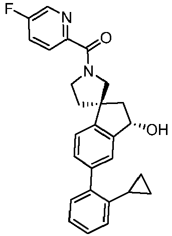 | ((1*S*,3*S*)-5-(2-cyclopropylphenyl)-3-hydroxy-2,3-dihydrospiro[indene-1,3'-pyrrolidine]-1'-yl)(5-fluoropyridin-2-yl) ketone | LC-MS: (M+H)⁺; m/z=429. 20; |
| | | | ¹H NMR (400 MHz, CDCl₃) δ 8.41 (dd, *J* = 34. 7, 2.8 Hz, 1H), 8.04 - 8.03 (m, 1H), 7. 54 - 7. 45 (m, 2H), 7.42 (d, *J* = 7.9 Hz, 1H), 7.30 - 7.24 (m, 2H), 7.23 - 7.18 (m, 2H), 6.91 (dd, *J* = 7.8, 3.7 Hz, 1H), 5.38 - 5.33 (m, 1H), 4.21 - 3.84 (m, 4H), 2.55 - 2.52 (m, 1H), 2.26 - 2.00 (m, 4H), 1.92 - 1.83 (m, 1H), 0.88 - 0.82 (m, 2H), 0.75 - 0.69 (m, 2H). |
| | | | ¹⁹F NMR (376 MHz, CDCl₃) δ -123.27 (d, *J* = 19. 4 Hz). |
| 25 | 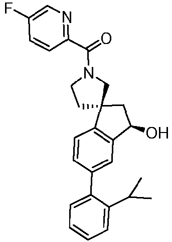 | ((1*S,*3*R*)-5-(2-cyclopropylphenyl)-3-hydroxy-2,3-dihydrospiro[indene-1,3'-pyrrolidine]-1'-yl)(5-fluoropyridin-2-yl) ketone | LC-MS: (M+H)⁺; m/z = 431. 22; |
| | | | ¹H NMR (400 MHz, CDCl₃) δ 8.42 (dd, *J* = 31.4, 2.8 Hz, 1H), 8.05 - 8.02 (m, 1H), 7.51 - 7.48 (m, 1H), 7.40 - 7.33 (m, 3H), 7.28 (d, *J* = 7.8 Hz, 1H), 7.25 (s, 1H), 7.23 - 7.10 (m, 2H), 5.36 - 5.34 (m, 1H), 4.27 - 3.80 (m, 4H), 3.05 - 3.03 (m, 1H), 2.56 - 2.47 (m, 1H), 2.29 - 2. 18 (m, 1H), 2. 18 - 2.08 (m, 1H), 2.08 - 1.91 (m, 2H), 1.17 (d, *J* = 6.8 Hz, 6H). |
| 26 | 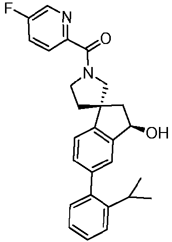 | ((1*R*,3*R*)-5-(2-cyclopropylphenyl)-3-hydroxy-2,3-dihydrospiro[indene-1,3'-pyrrolidine]-1'-yl)(5-fluoropyridin-2-yl) ketone | LC-MS: (M+H)⁺; m/z = 430. 96; |
| | | | ¹H NMR (400 MHz, CDCl₃) δ 8.41 (dd, *J* = 38.5, 2.8 Hz, 1H), 8.03 (ddd, *J* = 7.9, 4.6, 2.9 Hz, 1H), 7.51 - 7.48 (m, 1H), 7.42 - 7.31 (m, 3H), 7.31 - 7.26 (m, 2H), 7.23 - 7.11 (m, 2H), 5.33 - 5.28 (m, 1H), 4.27 - 3.71 (m, 4H), 3.05 - 3.03 (m, 1H), 2.60 - 2.57 (m, 1H), 2.42 - 2.38 (m, 1H), 2.22 - 2.19 (m, 1H), 2.15 - 1.95 (m, 2H), 1.16 (d, *J* = 6.9 Hz, 6H). |
| 27 | 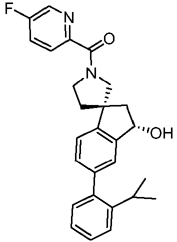 | ((1*R*,3*S*)-5-(2-cyclopropylphenyl)-3-hydroxy-2, 3-dihydrospiro [indene-1,3' - pyrrolidine]-1' -yl)(5-fluoropyridin-2-yl) ketone | LC-MS: (M+H)⁺; m/z = 431. 18 |
| 28 | 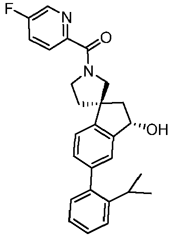 | ((1*S*,3*S*)-5-(2-cyclopropylphenyl)-3-hydroxy-2,3-dihydrospiro[indene-1, 3'-pyrrolidine]-1' -yl)(5-fluoropyridin-2-yl) ketone | LC-MS: (M+H) ⁺; m/z = 431. 18 |
| 29 | 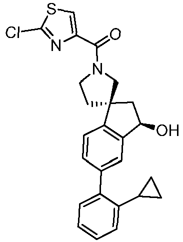 | (2-chlorothiazol-4-yl) ((1*S,3R*)-5-(2-cyclopropylphenyl)-3-hydroxy-2,3-dihydrospiro[indene-1,3'-pyrrolidine]-1'-yl) ketone | LC-MS: (M+H)⁺; m/z = 451. 10 |
| 30 | 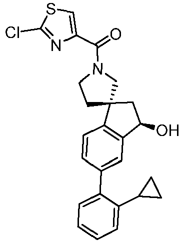 | (2-chlorothiazol-4-yl) ((1*R*,3*R*)-5-(2-cyclopropylphenyl)-3-hydroxy-2,3-dihydrospiro[indene-1,3'-pyrrolidine]-1'-yl) ketone | LC-MS: (M+H)⁺; m/z = 451. 10 |
| 31 | 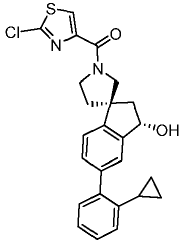 | (2-chlorothiazol-4-yl) ((1*S*,3*S*)-5-(2-cyclopropylphenyl)-3-hydroxy-2,3-dihydrospiro[indene-1,3'-pyrrolidine]-1'-yl) ketone | LC-MS: (M+H) ⁺; m/z = 451. 10 |
| 32 | 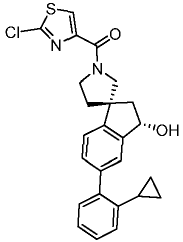 | (2-chlorothiazol-4-yl) ((1*R*,3*S*)-5-(2-cyclopropylphenyl)-3-hydroxy-2,3-dihydrospiro[indene-1,3'-pyrrolidine]-1'-yl) ketone | LC-MS: (M+H)⁺; m/z = 451. 10 |
| 33 | 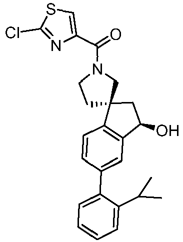 | (2-chlorothiazol-4-yl) ((1*S*,*3R*)-3-hydroxy-5-(2-isopropylphenyl)-2, 3-dihydrospiro[indene-1,3'-pyrrolidine]-1'-yl) ketone | LC-MS: (M-OH)⁺; m/z = 435.07 |
| 34 | 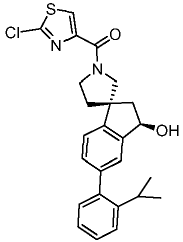 | (2-chlorothiazol-4-yl) ((1*R*,*3R*)-3-hydroxy-5-(2-isopropylphenyl)-2,3-dihydrospiro[indene-1,3'-pyrrolidine]-1'-yl) ketone | LC-MS: (M-OH)⁺; m/z = 435. 07 |
| 35 | 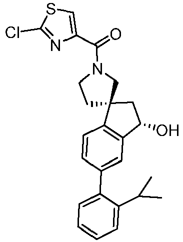 | (2-chlorothiazol-4-yl) ((1*S*, 3*S*)-3-hydroxy-5-(2-isopropylphenyl)-2,3-dihydrospiro[indene-1,3'-pyrrolidine]-1'-yl) ketone | LC-MS: (M-OH)⁺; m/z = 435.07 |
| 36 | 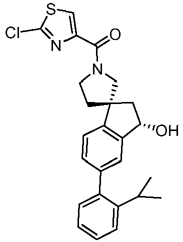 | (2-chlorothiazol-4-yl) ((1*R*,3*S*)-3-hydroxy-5-(2-isopropylphenyl)-2, 3-dihydrospiro[indene-1, 3'-pyrrolidine]-1'-yl) ketone | LC-MS: (M-OH)⁺; m/z = 435. 07 |
| 37 | 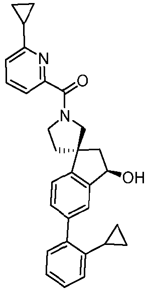 | ((1*S,*3*R*)-5-(2-cyclopropylphenyl)-3-hydroxy-2,3-dihydrospiro[indene-1, 3'-pyrrolidine]-1'-yl)(6-cyclopropylpyridin-2-yl) ketone | LC-MS: (M+H)⁺; m/z = 451. 18 |
| 38 | 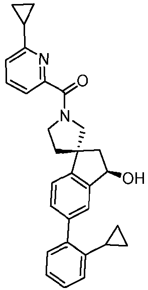 | ((1*R*,3*R*)-5-(2-cyclopropylphenyl)-3-hydroxy-2,3-dihydrospiro[indene-1, 3'-pyrrolidine]-1'-yl)(6-cyclopropylpyridin-2-yl) ketone | LC-MS: (M+H)⁺; m/z = 451. 18 |
| 39 | 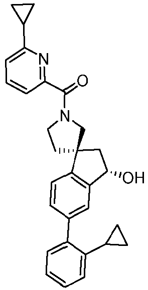 | ((1*S*,3*S*)-5-(2-cyclopropylphenyl)-3-hydroxy-2,3-dihydrospiro[indene-1,3'-pyrrolidine]-1'-yl)(6-cyclopropylpyridin-2-yl) ketone | LC-MS: (M+H)⁺; m/z = 451. 18 |
| 40 | 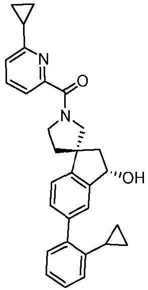 | ((1*R*,3*S*)-5-(2-cyclopropylphenyl)-3-hydroxy-2,3-dihydrospiro[indene-1,3'-pyrrolidine]-1'-yl)(6-cyclopropylpyridin-2-yl) ketone | LC-MS: (M+H)⁺; m/z = 451. 18 |
| 41 | 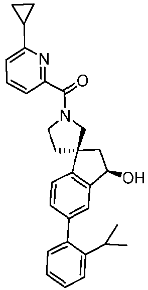 | (6-cyclopropylpyridin-2-yl)((1*S*,3*R*)-3-hydroxy-5-(2-isopropylphenyl)-2, 3-dihydrospiro[indene-1,3'-pyrrolidine]-1'-yl) ketone | LC-MS: (M+H)⁺; m/z = 453. 16 |
| 42 | 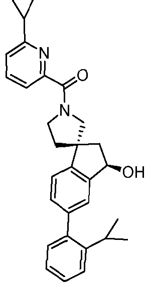 | (6-cyclopropylpyridin-2-yl)((1*R*,3*R*)-3-hydroxy-5-(2-isopropylphenyl)-2, 3-dihydrospiro[indene-1, 3'-pyrrolidine]-1'-yl) ketone | LC-MS: (M+H)⁺; m/z = 453. 16 |
| 43 | 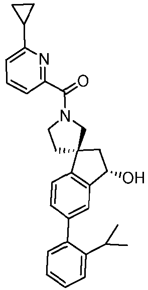 | (6-cyclopropylpyridin-2-yl)((1*S*,3*S*)-3-hydroxy-5-(2-isopropylphenyl)-2, 3-dihydrospiro[indene-1,3'-pyrrolidine]-1'-yl) ketone | LC-MS: (M+H)⁺; m/z = 453. 16 |
| 44 | 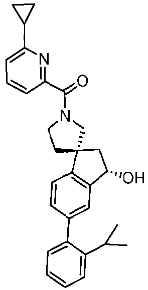 | (6-cyclopropylpyridin-2-yl)((1*R*,3*S*)-3-hydroxy-5-(2-isopropylphenyl)-2, 3-dihydrospiro[indene-1,3'-pyrrolidine]-1'-yl) ketone | LC-MS: (M+H)⁺; m/z = 453. 16 |

### Example 45 : ((3R)-5-(2-cyclopropylphenyl)-3-hydroxy-2,3-dihydrospiro[indene-1,3'-pyrrolidine]-1'-yl)(5-fluoropyridin-2-yl) ketone

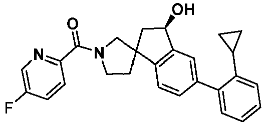

### Step A: (3R)-5-(2-cyclopropylphenyl)-3-hydroxy-2,3-dihydrospiro[indene-1,3'-pyrrolidine]-5'-one

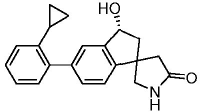

50.0 mg (0.164 mmol, 1.0 eq) 5-bromospiro[indene-1,3'-pyrrolidine]-3,5'(2*H*)-dione was dissolved in 5 mL anhydrous tetrahydrofuran, and then, 4.37 mg (0.016 mmol, 0.1 eq) (S)-3,3-diphenyl-1-methylpyrrolidone[1,2*-c*]-1,3,2-oxaborolane and 4.37 mg (0.096 mmol, 0.6 eq) borane dimethyl sulfide were added. Stirring was carried out at room temperature for 3 h. After confirming that the product had been generated through LCMS, the reaction solution was quenched using methanol, and evaporated under reduced pressure to obtain a crude product (46.0 mg, yield =91%)
LC-MS: (M+H)⁺; m/z = 320.16;

### Step B: (3R)-5-(2-cyclopropylphenyl)-2,3-dihydrospiro[indene-1,3'-pyrrolidine]-3-ol

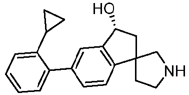

46.0 mg (0.14 mmol, 1.0 eq) (3*R*)-5-(2-cyclopropylphenyl)-3-hydroxy-2,3-dihydrospiro[indene-1,3'-pyrrolidine]-5'-one was dissolved in 5 mL anhydrous tetrahydrofuran, and 55.0 mg (1.40 mmol, 10.0 eq) lithium aluminum hydride was slowly added at 0°C. Stirring was carried out at 70°C for 2 h. After confirming that the product had been generated through LCMS, the reaction solution was filtered through diatomite and washed with tetrahydrofuran. After drying over anhydrous sodium sulfate and evaporating under reduced pressure, a crude product was obtained (39.0 mg, yield =88%).
LC-MS: (M+H)⁺; m/z = 306.17;

### Step C: ((3R)-5-(2-cyclopropylphenyl)-3-hydroxy-2,3-dihydrospiro[indene-1,3'-pyrrolidine]-1'-yl)(5-fluoropyridin-2-yl) ketone

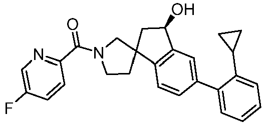

27.0 mg (0.19 mmol, 1.5eq) 5-fluoropyridin-2-carboxylic acid, 0.06 mL *N,N-*diisopropylethylamine and 73.0 mg (0.19 mmol, 1.5 eq) 2-(7-azobenzotriazole)-*N*,*N*,*N*',*N*'-tetramethylurea hexafluorophosphate were dissolved in 5 mL tetrahydrofuran, and then stirring was carried out at room temperature for 0.5 h. 39.0 mg (0.13 mmol, 1.0 eq) (3R)-5-(2-cyclopropylphenyl)-2,3-dihydrospiro[indene-1,3'-pyrrolidine]-3-ol was added, and then stirring was carried out at room temperature for 1.5 h. After confirming that the product had been generated through LCMS, 5 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min), a white solid was obtained (8.00mg, yield =13%, dr = 86:14).
LC-MS: (M+H)⁺; m/z = 429.20;
¹H NMR (400 MHz, CDCl₃) δ 8.44 (dd, *J* = 38.5, 3.2 Hz, 1H), 8.09 - 8.04 (m, 1H), 7.56 - 7.44 (m, 3H), 7.34 - 7.19 (m, 4H), 6.94 (dd, *J* = 7.9, 4.2 Hz, 1H), 5.42 - 5.30 (m, 1H), 4.27 - 3.80 (m, 4H), 2.70 - 2.03 (m, 5H), 1.94 - 1.85 (m, 1H), 0.89 - 0.84 (m, 2H), 0.76 - 0.72 (m, 2H).
¹⁹F NMR (376 MHz, CDCl₃) δ -126.50 (d, *J* = 20.5 Hz).

### Example 46 : ((3S)-5-(2-cyclopropylphenyl)-3-hydroxy-2,3-dihydrospiro[indene-1,3'-pyrrolidine]-1'-yl)(5-fluoropyridin-2-yl) ketone

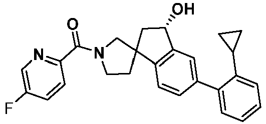

### Step A: (3S)-5-(2-cyclopropylphenyl)-3-hydroxy-2,3-dihydrospiro[indene-1,3'-pyrrolidine]-5'-one

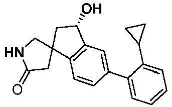

50.0 mg (0.164 mmol, 1.0 eq) 5-bromospiro[indene-1,3'-pyrrolidine]-3,5'(2*H*)-dione was dissolved in 5 mL anhydrous tetrahydrofuran, and then, 4.37 mg (0.016 mmol, 0.1 eq) (*R*)-3,3-diphenyl-1-methylpyrrolidone[1,2-*c*]-1,3,2-oxaborolane and 4.37 mg (0.096 mmol, 0.6 eq) borane dimethyl sulfide were added. Stirring was carried out at room temperature for 3 h. After confirming that the product had been generated through LCMS, the reaction solution was quenched using methanol, and evaporated under reduced pressure to obtain a crude product (48.0 mg, yield =95%)
LC-MS: (M+H)⁺; m/z = 320.14;

### Step B: (3S)-5-(2-cyclopropylphenyl)-2,3-dihydrospiro[indene-1,3'-pyrrolidine]-3-ol

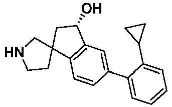

48.0 mg (0.15 mmol, 1.0 eq) (3*S*)-5-(2-cyclopropylphenyl)-3-hydroxy-2,3-dihydrospiro[indene-1,3'-pyrrolidine]-5'-one was dissolved in 5 mL anhydrous tetrahydrofuran, and 57.0 mg (1.5 mmol, 10.0 eq) lithium aluminum hydride was slowly added at 0°C. Stirring was carried out at 70°C for 2 h. After confirming that the product had been generated through LCMS, the reaction solution was filtered through diatomite and washed with tetrahydrofuran. After drying over anhydrous sodium sulfate and evaporating under reduced pressure, a crude product was obtained (43.0 mg, yield =93%).
LC-MS: (M+H)⁺; m/z = 306.16;

### Step C: ((3S)-5-(2-cyclopropylphenyl)-3-hydroxy-2,3-dihydrospiro[indene-1,3'-pyrrolidine]-1'-yl)(5-fluoropyridin-2-yl) ketone

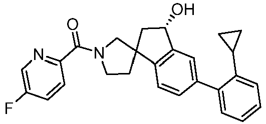

30.0 mg (0.21 mmol, 1.5eq) 5-fluoropyridin-2-carboxylic acid, 0.07 mL *N,N-*diisopropylethylamine and 80.0 mg (0.21 mmol, 1.5 eq) 2-(7-azobenzotriazole)-*N*,*N*,*N*',*N*'-tetramethylurea hexafluorophosphate were dissolved in 5 mL tetrahydrofuran, and then stirring was carried out at room temperature for 0.5 h. 43.0 mg (0.14 mmol, 1.0 eq) (3*S*)-5-(2-cyclopropylphenyl)-2,3-dihydrospiro[indene-1,3'-pyrrolidine]-3-ol was added, and then stirring was carried out at room temperature for 1.5 h. After confirming that the product had been generated through LCMS, 5 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min), a white solid was obtained (2.20 mg, yield =4.0%, dr = 75:15).
LC-MS: (M+H)⁺; m/z = 429.20;
¹H NMR (400 MHz, CDCl₃) δ 8.44 (dd, *J* = 38.5, 3.2 Hz, 1H), 8.09 - 8.04 (m, 1H), 7.56 - 7.44 (m, 3H), 7.34 - 7.19 (m, 4H), 6.94 (dd, *J* = 7.9, 4.2 Hz, 1H), 5.42 - 5.30 (m, 1H), 4.27 - 3.80 (m, 4H), 2.70 - 2.03 (m, 5H), 1.94 - 1.85 (m, 1H), 0.89 - 0.84 (m, 2H), 0.76 - 0.72 (m, 2H).
¹⁹F NMR (376 MHz, CDCl₃) δ -126.82 (d, *J* = 22.5 Hz).

### Example 47 : (5-(2-cyclopropylphenyl)-3-hydroxy-2,3-dihydrospiro[indene-1,3'-pyrrolidine]-1'-yl)(6-(methoxymethyl)pyridin-2-yl) ketone

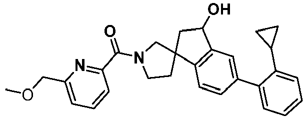

### Step A: methyl 6-(methoxymethyl)picolinate

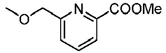

1.00 g (5.98 mmol, 1.0 eq) methyl 6-(hydroxymethyl)picolinate was dissolved in 30 mL anhydrous tetrahydrofuran, and then nitrogen replacement was carried out for three times. 0.36 g (8.97 mmol, 1.5 eq) sodium hydride was added at 0 °C, and then stirring was carried out at room temperature for 0.5 h. Then, 1.70 g (12.0 mmol, 2.0 eq) methyl iodide was added dropwise at room temperature, and stirring was carried out overnight at room temperature. After confirming that the raw materials had reacted completely, 50 mL water was added for quenching, and then extraction was carried out with dichloromethane for three times. The organic layer was washed with 50 mL saturated saline solution, dried over anhydrous sodium sulfate and evaporated under reduced pressure. The residue was subjected to column chromatography (DCM/MeOH = 0~20:1) to obtain the product (222 mg, yield =20%).
LC-MS: (M+H)⁺; m/z = 182.04;

### Step B: 6- (methoxymethyl) pyridine carboxylic acid

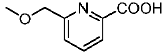

220 mg (1.21 mmol, 1.0eq) methyl 6-(methoxymethyl)picolinate was dissolved in 3 mL tetrahydrofuran, and then, 3 mL sodium hydroxide solution (0.5mol/L) was added. Stirring was carried out at room temperature for 3 h. After confirming that the product had been generated through LCMS, the reaction solution was diluted with hydrochloric acid solution (1.0 mol/L) to acidity, and evaporated under reduced pressure to abtian a crude product (0.15 g, yield = 74%).
LC-MS: (M-H)⁺; m/z = 168.05;

### Step C: (5-(2-cyclopropylphenyl)-3-hydroxy-2,3-dihydrospiro[indene-1,3'-pyrrolidine]-1'-yl)(6-(methoxymethyl)pyridin-2-yl) ketone

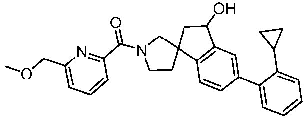

41.0 mg (0.24 mmol, 1.5 eq) 6-(methoxymethyl)pyridin-2-carboxylic acid, 0.08 mL N,N-diisopropylethylamine and 93 mg (0.24 mmol, 1.5 eq) 2-(7-azobenzotriazole)-N,N,N',N'-tetramethylurea hexafluorophosphate were dissolved in 10 mL tetrahydrofuran, and then stirring was carried out at room temperature for 0.5 h. 50.0 mg (0.16 mmol, 1.0 eq) 5-(2-cyclopropylphenyl)-2,3-dihydrospiro[indene-1,3'-pyrrolidine]-3-ol was added, and then stirring was carried out at room temperature for 3 h. After confirming that the product had been generated through LCMS, 5 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min), a white solid was obtained (11.0 mg, yield =15%).
LC-MS: (M+H)⁺; m/z = 455.16;
¹H NMR (400 MHz, CDCl₃) δ 7.86 - 7.76 (m, 2H), 7.53 - 7.41 (m, 3H), 7.32 - 7.26 (m, 2H), 7.22 - 7.17 (m, 2H), 6.93 - 6.90 (m, 1H), 5.38 - 5.27 (m, 1H), 4.63 - 4.55 (m, 2H), 4.08 - 3.78 (m, 4H), 3.50 - 3.45 (m, 3H), 2.68 - 2.35 (m, 2H), 2.26 - 2.17 (m, 1H), 2.14 - 2.00 (m, 2H), 1.91 - 1.82 (m, 1H), 0.89 - 0.82 (m, 2H), 0.74 - 0.69 (m, 2H).

### Example 48 : (5-(2-cyclopropylphenyl)-3-hydroxy-2,3-dihydrospiro[indene-1,3'-pyrrolidine]-1'-yl)(6-(methylamino)pyrazin-2-yl) ketone

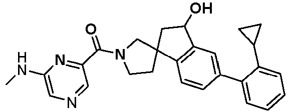

24. 5mg (0.16 mmol, 1.0 eq) 6-(methylamino)pyrazine-2-carboxylic acid, 74.2 mg (0.41 mmol, 1.2 eq) 2-(7-azobenzotriazole)-N,N,N',N'-tetramethylurea hexafluorophosphate, 63.0 mg (0.48 mmol, 3.0 eq) N,N-diisopropylethylamine and 3mL tetrahydrofuran were added to a 25 mL reaction flask. Under nitrogen protection, the reaction solution was stirred at room temperature for 10 min, and then, 50.0 mg (0.16mmol, 1.0eq) 5-(2-isopropylphenyl)-2,3-dihydrospiro[indene-1,3'-pyrrolidine]-3-ol was weighed and dissolved in 3 mL tetrahydrofuran, and then was slowly added to the reaction solution. Stirring was carried out at room temperature for 1 h. A complete reaction of the raw materials was confirmed through TLC Plate (DCM : MeOH=20:1). The reaction solution was concentrated to dryness, sent for preparation and purification (mobile phase A: 0.1% formic acid; mobile phase B: acetonitrile; gradient: 30% - 70% B, 55 min; flow rate: 70mL/min) and freeze-dried to obtain a white solid pure product (8.42 mg, yield =12%).
LC-MS: (M+H)⁺; m/z = 441.14;
¹H NMR (400 MHz, CDCl₃) δ 8.29 (d, *J* = 18.5 Hz, 1H), 8.03 - 7.96 (m, 1H), 7.53 - 7.49 (m, 1H), 7.47 - 7.42 (m, 1H), 7.32 - 7.27 (m, 1H), 7.26 - 7.23 (m, 1H), 7.22 - 7.19 (m, 2H), 6.92 (dd, *J* = 8.0, 4.1 Hz, 1H), 5.40 - 5.28 (m, 1H), 4.21 - 4.13 (m, 2H), 3.97 - 3.78 (m, 3H), 3.04 - 2.94 (m, 3H), 2.66 - 1.97 (m, 5H), 1.91 - 1.82 (m, 1H), 0.88 - 0.82 (m, 2H), 0.74 - 0.69 (m, 2H).

Examples in Table 2 were prepared by using the method described in Example 48 above, but wherein Intermediate I3 and the desired different carboxylic acids were prepared by condensation reaction.

**Table 2: Examples 49^{~}56**

| Exampl e No. | Structure | Compound Name | Spectral Data |
|---|---|---|---|
| 49 | 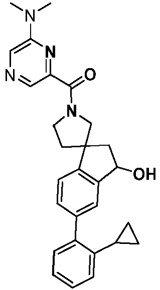 | (5-(2-cyclopropylphe ny1)-3-hydroxy-2,3-dihydrospiro[i ndene-1,3'-pyrrolidine]-1'-yl)(6-(dimethylamino ) pyrazin-2-yl)ketone | LC-MS: (M+H)⁺; m/z=455. 41; ¹H NMR (400 MHz, CDCl₃) δ 8.30 (d, *J* = 25. 9 Hz, 1H), 8.12 (d, *J* = 28. 6 Hz, 1H), 7.53 - 7.49 (m, 1H), 7.46 - 7.42 (m, 1H), 7.32 - 7.28 (m, 1H), 7.26 - 7.23 (m, 1H), 7.22 - 7. 19 (m, 2H), 6.92 (dd, *J* = 7.8, 3. 7 Hz, 1H), 5.41 - 5. 28 (m, 1H), 4. 20 - 3.98 (m, 3H), 3.92 - 3.84 (m, 1H), 3. 18 (dd, *J* = 36. 3, 2. 7 Hz, 6H), 2.67 - 2.38 (m, 2H), 2.29 - 2.01 (m, 3H), 1.86 - 1.81 (m, 1H), 0.88 - 0.82 (m, 2H), 0.73 - 0.70 (m, 2H). |
| 50 | 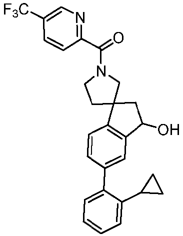 | (5-(2-cyclopropylphe nyl)-3-hydroxy-2,3-dihydrospiro[i ndene-1,3'-pyrrolidine]-1'-yl)(5-(trifluorometh yl)pyridin-2-yl)ketone | LC-MS: (M+H)⁺; m/z=479. 16; ¹H NMR (400 MHz, CDCl₃) δ 8.90 - 8.80 (m, 1H), 8.11 - 8.03 (m, 2H), 7.53 - 7.43 (m, 2H), 7.33 - 7.26 (m, 2H), 7.22 - 7.19 (m, 2H), 6.94 - 6. 90 (m, 1H), 5. 39 - 5. 30 (m, 1H), 4.12 - 3.80 (m, 4H), 2.69 - 2.39 (m, 2H), 2. 28- 2.20 (m, 1H), 2. 13 - 2.05 (m, 1H), 1.89 - 1.83 (m, 1H), 0. 89-0. 82 (m, 2H), 0.74-0.70 (m, 2H). |
| | | | ¹⁹F NMR (376 MHz, CDCl₃) δ -62.58 (d, *J* = 22. 5 Hz). |
| 51 | 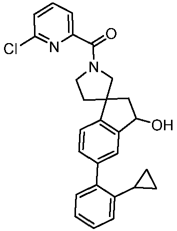 | (6-chloropyridin-2-yl)(5-(2-cyclopropylphe nyl)-3-hydroxy-2,3-dihydrospiro[i ndene-1, 3' - pyrrolidine]-1'-yl) ketone | LC-MS: (M+H)⁺; m/z=445.07; ¹H NMR (400 MHz, CDCl₃) δ 7.93 - 7.88 (m, 1H), 7.83 - 7.75 (m, 1H), 7.52 - 7.28 (m, 5H), 7.25 - 7. 19 (m, 2H), 6. 94- 6. 90 (m, 1H), 5.39 - 5. 28 (m, 1H), 4. 27 - 3. 77 (m, 4H), 2. 68 - 2. 54 (m, 1H), 2. 45-2. 36 (m, 1H), 2.27 - 2.17 (m, 1H), 2. 14 - 2. 03 (m, 1H), 1. 92 - 1. 84 (m, 1H), , 0.89- 0.83 (m, 2H), 0.75 - 0.70 (m, 2H). |
| 52 | 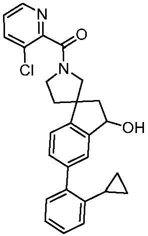 | (3-chloropyridin-2-yl) (5-(2-cyclopropylphe nyl)-3-hydroxy-2,3-dihydrospiro[i ndene-1,3'-pyrrolidine]-1'-yl) ketone | LC-MS: (M+H)⁺; m/z=445. 10; ¹H NMR (400 MHz, CDCl₃) δ 8.55 (dd, *J* = 30.8, 4.7 Hz, 1H), 7.80 (dd, J = 27.9, 8.2 Hz, 1H), 7.54 - 7.43 (m, 2H), 7.37 - 7.34 (m, 1H), 7.31 - 7.26 (m, 2H), 7.25 - 7.20 (m, 2H), 6.94 (t, *J* = 8.2 Hz, 1H), 5.43 - 5.36 (m, 1H), 4.14 - 3.87 (m, 2H), 3.59 - 3.43 (m, 2H), 2.71 - 2.59 (m, 1H), 2.54 - 2.38 (m, 1H), 2.34 - 2.18 (m, 1H), 2.16 - 2.07 (m, 1H), 1.93 - 1.84 (m, 1H), 0.90 - 0.83 (m, 2H), 0.77 - 0.71 (m, 2H). |
| 53 | 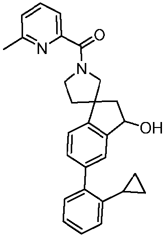 | (5-(2-cyclopropylphe nyl)-3-hydroxy-2,3-dihydrospiro[i ndene-1,3'-pyrrolidine]-1'-yl)(6-methylpyridin-2-yl) ketone | LC-MS: (M+H)⁺; m/z=425. 15; ¹H NMR (400 MHz, CDCl₃) δ 7.72 - 7.64 (m, 2H), 7.52 - 7.48 (m, 1H), 7.45 - 7.41 (m, 1H), 7.32 - 7.27 (m, 2H), 7.26- 7.17 (m, 3H), 6.92 (t, *J* = 6.9 Hz, 0H), 5.40 - 5.26 (m, 1H), 4.12 - 3.78 (m, 4H), 2.69 - 2.35 (m, 5H), 2.27 - 2.17 (m, 1H), 2.12 - 2.01 (m, 1H), 1.92-1.83 (m, 1H), 0.88- 0.82 (m, 1H), 0.74 - 0.69 (m, 1H). |
| 54 | 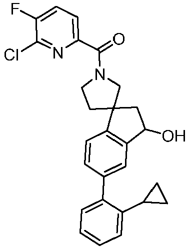 | (6-chloro-5-fluoropyridin-2-yl) (5-(2-cyclopropylphe nyl)-3-hydroxy-2,3-dihydrospiro[indene-1,3'-pyrrolidine]-1'-yl)ketone | LC-MS: (M+H)⁺; m/z=463.08; ¹H NMR (400 MHz, CDCl₃) δ 8.05 - 8.00 (m, 1H), 7.62 - 7.55 (m, 1H), 7.52 - 7. 50 (m, 1H), 7.46- 7.43 (m, 1H), 7.31 - 7.25 (m, 2H), 7.22 - 7.20 (m, 2H), 6.92 (dd, *J* = 7.7, 4.1 Hz, 1H), 5.38 - 5.31 (m, 1H), 4. 16 - 3. 77 (m, 4H), 2.67 - 2. 52 (m, 1H), 2. 47 - 2. 35 (m, 1H), 2. 29 - 2. 19 (m, 1H), 2.15 - 2.05 (m, 1H), 1. 90 - 1. 84 (m, 1H), 0.89 - 0.83 (m, 2H), 0.74 - 0.70 (m, 2H). ¹⁹F NMR (376 MHz, CDCl₃) δ -115. 42 (d, *J* = 18. 5 Hz). |
| 55 | 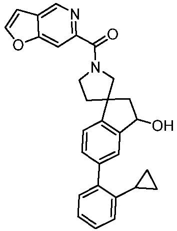 | (5-(2-cyclopropylphe nyl)-3-hydroxy-2,3-dihydrospiro[i ndene-1,3'-pyrrolidine]-1'-yl)(furan [3, 2-c]pyridin-6-yl)ketone | LC-MS: (M+H)⁺; m/z = 451. 15; ¹H NMR (400 MHz, CDCl₃) δ 8.90 - 8.75 (m, 1H), 8.29 - 8.27 (m, 1H), 7.83 - 7.79 (m, 1H), 7.55 - 7.48 (m, 1H), 7.46 - 7.41 (m, 1H), 7.37 - 7.30 (m, 1H), 7.24 - 7.18 (m, 3H), 6.96 - 6.88 (m, 2H), 5.40 - 5.27 (m, 1H), 4.23 - 4.08 (m, 2H), 4.02 - 3.80 (m, 2H), 2.74 - 2.40 (m, 1H), 2.39 - 1.90 (m, 4H), 1.89 - 1.83 (m, 1H), 0.88 - 0.80 (m, 2H), 0.74 - 0.69 (m, 2H). |
| 56 | 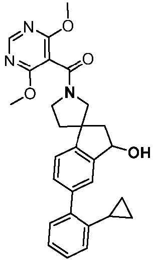 | (5-(2-cyclopropylphe nyl)-3-hydroxy-2,3-dihydrospiro[i ndene-1,3'-pyrrolidine]-1'-yl)(4, 6-dimethoxypyrim idin-5-yl) ketone | LC-MS: (M+H)⁺; m/z = 472.18; ¹H NMR (400 MHz, CDCl₃) δ 8.48 - 8.40 (m, 1H), 7.52 - 7.50 (m, 1H), 7.42 (d, *J* = 8.0 Hz, 1H), 7.38 - 7.28 (m, 2H), 7.23 - 7.17 (m, 2H), 6.94 - 6.90 (m, 1H), 5.37 - 5.25 (m, 1H), 4.08 - 3.94 (m, 6H), 3.91 - 3.28 (m, 4H), 2.66 - 2.47 (m, 1H), 2.47 - 1.98 (m, 4H), 1.86 - 1.84 (m, 1H), 0.89 - 0.81 (m, 2H), 0.73 - 0.72 (m, 2H). |

### Example 57 : (3-hydroxy-5-(2-isopropylphenyl)-2,3-dihydrospiro[indene-1,3'-pyrrolidine]-1'-yl)(pyrazolo[1,5-a]pyrimidin-5-yl) ketone

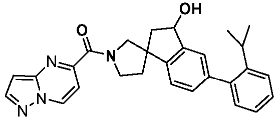

26.6 mg (0.16 mmol, 1.0 eq) pyrazolo[1,5-a]pyrimidin-5-carboxylic acid, 74.2 mg (0.41 mmol, 1.2 eq) 2-(7-azobenzotriazole)-N,N,N',N'-tetramethylurea hexafluorophosphate, 63.0 mg (0.48 mmol, 3.0 eq) N,N-diisopropylethylamine and 3mL tetrahydrofuran were added to a 25 mL reaction flask. Under nitrogen protection, the reaction solution was stirred at room temperature for 10 min, and then, 50.0 mg (0.16mmol, 1.0eq) 5-(2-isopropylphenyl)-2,3-dihydrospiro[indene-1,3'-pyrrolidine]-3-ol was weighed and dissolved in 3 mL tetrahydrofuran, and then was slowly added to the reaction solution. Stirring was carried out at room temperature for 1 h. A complete reaction of the raw materials was confirmed through TLC Plate (DCM/MeOH = 20:1). The reaction solution was concentrated to dryness, and sent for preparation and purification (mobile phase A: 0.1% formic acid; mobile phase B: acetonitrile; gradient: 30% -70% B, 55 min; flow rate: 70mL/min) to obtain a white solid pure product (8.54 mg, yield =11%).
LC-MS: (M+H)⁺; m/z = 453.37;
¹H NMR (400 MHz, CDCl₃) δ 8.79 - 8.74 (m, 1H), 8.22-8.17 (m, 1H), 7.53 - 7.48 (m, 1H), 7.41 - 7.26 (m, 5H), 7.23 - 7.13 (m, 2H), 6.81 - 6.73 (m, 1H), 5.41-5.30 (m, 1H), 4.41 - 3.81 (m, 4H), 3.09-2.99 (m, 1H), 2.70 - 2.39 (m, 2H), 2.30 - 2.00 (m, 3H), 1.17 (d, *J* = 6.8 Hz, 6H).

Examples in Table 3 were prepared by using the method described in Example 57 above, but wherein Intermediate I4 and the desired different carboxylic acids were prepared by condensation reaction.

**Table 3: Examples 58^{~}77**

| Exa mpl e No. | Structure | Compound Name | Spectral Data |
|---|---|---|---|
| 58 | 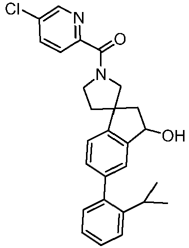 | (5-chloropyridin-2-yl)(3-hydroxy-5-(2-isopropylphenyl)-2, 3-dihydrospiro[indene-1,3'-pyrrolidine]-1'-yl)ketone | LC-MS: (M+H)⁺; m/z=447. 12; |
| | | | ¹H NMR (400 MHz, CDCl₃ δ 8. 59 - 8. 49 (m, 1H), 7.98 - 7.94 (m, 1H), 7.82 - 7. 76 (m, 1H), 7.40 - 7.33 (m, 3H), 7.31 - 7.26 (m, 2H), 7.24 - 7. 13 (m, 2H), 5.36 - 5. 29 (m, 1H), 4.25 - 3.78 (m, 4H), 3. 13 - 2. 99 (m, 1H), 2.69 - 2. 37 (m, 2H), 2. 28 - 2. 17 (m, 1H), 2. 14 - 2.00 (m, 1H), 1.87 - 1. 82 (m, 1H), 1. 19 (d, *J* = 6.6 Hz, 6H). |
| 59 | 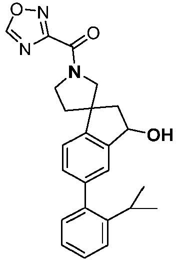 | (3-hydroxy-5-(2-isopropylphenyl)-2, 3-dihydrospiro[indene-1,3'-pyrrolidine]-1'-yl)(1, 2, 4-oxadiazol-3-yl)ketone | ¹H NMR (400 MHz, CDCl₃) δ 8.72 (s, 1H), 7.42 - 7.28 (m, 4H), 7. 25-7. 11 (m, 3H), 5.36 - 5. 24 (m, 1H), 4.08 - 3. 57 (m, 4H), 2.80 - 2.67 (m, 1H), 2.62 - 2. 50 (m, 1H), 2.22 - 2. 20 (m, 2H), 2.06 - 2.00 (m, 2H), 1. 18 (d, *J* = 6.8 Hz, 6H). |
| 60 | 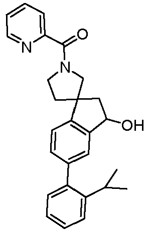 | (3-hydroxy-5-(2-isopropylphenyl)-2, 3-dihydrospiro[indene-1,3'-pyrrolidine]-1'- yl)(pyridin-2-yl) ketone | LC-MS: (M+H)⁺; m/z = 412. 90; |
| | | | ¹H NMR (400 MHz, CDCl₃ δ 8.63 - 8.55 (m, 1H), 7.94 - 7.91 (m, 1H), 7.85 - 7.78 (m, 1H), 7.41 - 7.27 (m, 6H), 7.24 - 7.12 (m, 2H), 5.38 - 5.28 (m, 1H), 4.22 - 3.79 (m, 4H), 3.13 - 2.99 (m, 1H), 2.70 - 2.37 (m, 2H), 2.28 - 2.18 (m, 1H), 2.16 - 1.98 (m, 2H), 1.16 (d, *J* = 6.8 Hz, 6H). |
| 61 | 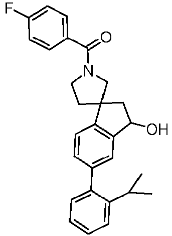 | (4-fluorophenyl) (3-hydroxy-5-(2-isopropylphenyl)-2, 3-dihydrospiro[indene-1,3'-pyrrolidine]-1'-yl)ketone | LC-MS: (M+H) ⁺; m/z = 430.39; |
| | | | ¹H NMR (400 MHz, CDCl₃) δ 7. 64 - 7. 54 (m, 2H), 7.41 - 7.26 (m, 4H), 7.25 - 7.04 (m, 5H), 5.38 - 5. 23 (m, 1H), 4.02 - 3.46 (m, 5H), 3.08 - 2.96 (m, 1H), 2. 51 - 2. 43 (m, 1H), 2. 29 - 2. 19 (m, 1H), 2. 14 - 1. 99 (m, 2H), 1. 18 (d, *J* = 7.0 Hz, 6H). |
| | | | ¹⁹F NMR (376 MHz, CDCl₃) δ -109.75 (d, *J* = 31. 2 Hz). |
| 62 | 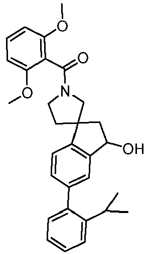 | (2, 6-dimethoxyphenyl) (3-hydroxy-5-(2-isopropylphenyl)-2, 3-dihydrospiro[indene-1,3'-pyrrolidine]-1'-yl)ketone | LC-MS: (M+H)⁺; m/z = 472. 20; |
| | | | ¹H NMR (400 MHz, CDCl₃ δ 7.42 - 7.29 (m, 4H), 7.25 - 7.17 (m, 3H), 7.16 - 7.09 (m, 1H), 6.62 - 6.50 (m, 2H), 5.35 - 5.23 (m, 1H), 4.09 - 3.91 (m, 1H), 3.91 - 3.72 (m, 7H), 3.54 - 3.43 (m, 1H), 3.28 (s, 1H), 3.03 - 3.01 (m, 1H), 2.71 - 2.51 (m, 1H), 2.49 - 2.31 (m, 1H), 2.26 - 2.18 (m, 1H), 2.13 - 2.04 (m, 1H), 2.05 - 1.96 (m, 1H), 1.18 (d, *J* = 6.8 Hz, 6H). |
| 63 | 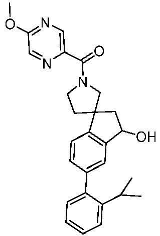 | (3-hydroxy-5-(2-isopropylphenyl)-2, 3-dihydrospiro[indene-1,3'-pyrrolidine]-1'-yl)(5-methoxypyrazin-2-yl)ketone | LC-MS: (M+H)⁺; m/z = 444. 16; |
| | | | ¹H NMR (400 MHz, CDCl₃ δ 8.82 (s, 1H), 8.18 - 8.08 (m, 1H), 7.41 - 7.26 (m, 4H), 7.25 - 7.13 (m, 3H), 5.39 - 5.28 (m, 1H), 4.25 - 3.78 (m, 8H), 3.09 - 3.00 (m, 1H), 2.68 - 2.36 (m, 2H), 2.26 - 2.18 (m, 1H), 2.17 - 2.00 (m, 2H), 1.17 (d, *J* = 6.8 Hz, 6H). |
| 64 | 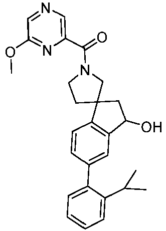 | (3-hydroxy-5-(2-isopropylphenyl)-2, 3-dihydrospiro[indene-1,3'-pyrrolidine]-1'-yl)(6-methoxypyrazin-2-yl)ketone | LC-MS: (M+H)⁺; m/z = 444. 19; |
| | | | ¹H NMR (400 MHz, CDCl₃) δ 8.85 - 8.64 (m, 1H), 8.31 (d, *J* = 24. 1 Hz, 1H), 7.42 - 7.31 (m, 3H), 7.31 - 7.27 (m, 1H), 7.25 (dd, *J* = 3.6, 1.9 Hz, 1H), 7.22 - 7.18 (m, 1H), 7. 18 - 7. 11 (m, 1H), 5.40 - 5. 27 (m, 1H), 4.28 - 4.08 (m, 1H), 4.08 - 3.80 (m, 6H), 3.09 - 2.97 (m, 1H), 2.68 - 2. 50 (m, 1H), 2.48 - 2.09 (m, 3H), 2.01 - 1.99 (m, 1H), 1.19 (d, *J* = 6.8 Hz, 6H). |
| 65 | 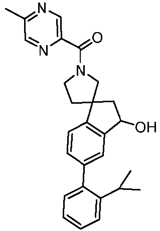 | (3-hydroxy-5-(2-isopropylphenyl)-2,3-dihydrospiro[indene-1,3'-pyrrolidine]-1'-yl)(5-methylpyrazin-2-yl) ketone | LC-MS: (M+H)⁺; m/z = 428. 18; |
| | | | ¹H NMR (400 MHz, CDCl₃) δ 9. 07 - 8. 82 (m, 1H), 8.52 (d, *J* = 18.9 Hz, 1H), 7.41 - 7.33 (m, 3H), 7.29 - 7.26 (m, 2H), 7.23 - 7.13 (m, 2H), 5.40 - 5.28 (m, 1H), 4.20 - 4.01 (m, 2H), 4.00 - 3.77 (m, 2H), 3.09 - 2.99 (m, 1H), 2.64 - 2.55 (m, 4H), 2.54 - 2. 38 (m, 1H), 2.29 - 1.98 (m, 3H), 1.16 (d, *J* = 6.6 Hz, 6H). |
| 66 | 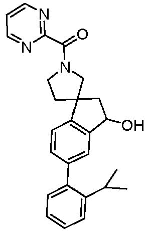 | (3-hydroxy-5-(2-isopropylphenyl)-2,3-dihydrospiro[indene-1,3'-pyrrolidine]-1'-yl)(pyrimidin-2-yl) ketone | LC-MS: (M+H)⁺; m/z = 414. 12; |
| | | | ¹H NMR (400 MHz, CDCl₃) δ 8.89 - 8.81 (m, 2H), 7.41 - 7.32 (m, 4H), 7.31 - 7.26 (m, 2H), 7.22 - 7.11 (m, 2H), 5.38 - 5.27 (m, 1H), 4.14 - 3.96 (m, 1H), 3.94 - 3.62 (m, 3H), 3.10 - 2.94 (m, 1H), 2.72 - 2.44 (m, 1H), 2.41 - 2.01 (m, 3H), 1.91 (s, 1H), 1.16 (d, *J* = 6.8 Hz, 6H). |
| 67 | 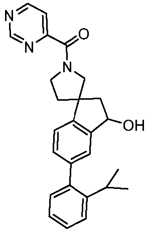 | (3-hydroxy-5-(2-isopropylphenyl)-2,3-dihydrospiro[indene-1,3'-pyrrolidine]-1'-yl)(pyrimidin-4-yl) ketone | LC-MS: (M+H)⁺; m/z = 414.13; |
| | | | ¹H NMR (400 MHz, CD₃OD) δ 9. 34 - 9. 18 (m, 1H), 9.03 - 8.93 (m, 1H), 7.88 - 7.85 (m, 1H), 7.44 - 7.28 (m, 4H), 7.26 - 7.08 (m, 3H), 5. 34 - 5. 21 (m, 1H), 4.23 - 3.99 (m, 2H), 3.94 - 3.72 (m, 2H), 3. 07 - 3. 03 (m, 1H), 2. 65 - 2. 51 (m, 1H), 2.31 - 2.00 (m, 3H), 1. 78 - 1. 55 (m, 1H), 1. 15 (d, *J* = 6.8 Hz, 6H). |
| 68 | 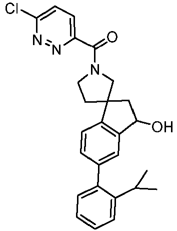 | (6-chloropyridazin-3-yl)(3-hydroxy-5-(2-isopropylphenyl)-2,3-dihydrospiro[indene-1,3'-pyrrolidine]-1'-yl) ketone | LC-MS: (M+H)⁺; m/z = 448. 15; |
| | | | ¹H NMR (400 MHz, CDCl₃) δ 8.15 - 8.10 (m, 1H), 7.68 - 7.63 (m, 1H), 7.40 - 7.32 (m, 3H), 7.31 - 7.27 (m, 1H), 7.25 - 7.13 (m, 3H), 5.39 - 5.29 (m, 1H), 4.40 - 4.10 (m, 2H), 4.08 - 3.82 (m, 2H), 3.03 - 3.01 (m, 1H), 2.66 - 2.47 (m, 1H), 2.35 - 2.22 (m, 1H), 2.22 - 2.02 (m, 2H), 2.02 - 1.82 (m, 1H), 1.17 (d, *J* = 6.1 Hz, 6H). |
| 69 | 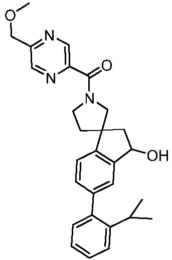 | (3-hydroxy-5-(2-isopropylphenyl)-2,3-dihydrospiro[indene-1,3'-pyrrolidine]-1'-yl)(5-(methoxymethyl)pyrazin-2-yl) ketone | LC-MS: (M+H)⁺; m/z = 458. 19 |
| 70 | 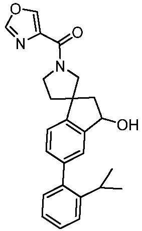 | (3-hydroxy-5-(2-isopropylphenyl)-2,3-dihydrospiro[indene-1,3'-pyrrolidine]-1'-yl)(oxazol-4-yl) ketone | LC-MS: (M+H)⁺; m/z = 403. 14; |
| | | | ¹H NMR (400 MHz, CDCl₃) δ 8.31 - 8.28 (m, 1H), 7.92 - 7.84 (m, 1H), 7.40 - 7.33 (m, 3H), 7.26 - 7.14 (m, 4H), 5.36 - 5.34 (m, 1H), 4.42 - 3.73 (m, 4H), 3.07 - 3.01 (m, 1H), 2.65 - 1. 89 (m, 5H), 1. 19 (d, *J* = 6.8 Hz, 6H). |
| 71 | 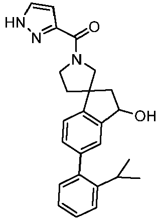 | (3-hydroxy-5-(2-isopropylphenyl)-2,3-dihydrospiro[indene-1,3'-pyrrolidine]-1'-yl)(1H-pyrazol-3-yl) ketone | LC-MS: (M+H)⁺; m/z = 402. 16; |
| | | | ¹H NMR (400 MHz, CDCl₃) δ 7.66 - 7.59 (m, 1H), 7.41 - 7.33 (m, 3H), 7.27 - 7.26 (m, 1H), 7. 24 - 7. 14 (m, 3H), 6. 77 - 6.70 (m, 1H), 5.39 - 5.32 (m, 1H), 4.42 - 3.77 (m, 4H), 3.07 - 3.00 (m, 1H), 2.65 - 1.99 (m, 6H), 1. 17 (d, *J* = 6.8 Hz, 6H). |
| 72 | 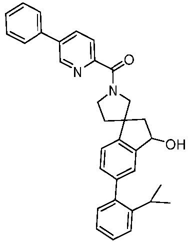 | (3-hydroxy-5-(2-isopropylphenyl)-2,3-dihydrospiro[indene-1,3'-pyrrolidine]-1'-yl)(5-phenylpyridin-2-yl) ketone | LC-MS: (M+H)⁺; m/z = 489. 16; |
| | | | ¹H NMR (400 MHz, CDCl₃) δ 8.86 - 8.76 (m, 1H), 8.05 - 7.98 (m, 2H), 7.65 - 7.27 (m, 10H), 7.22 - 7.13 (m, 2H), 5.39 - 5.29 (m, 1H), 4.28 - 3.82 (m, 4H), 3.08 - 3.00 (m, 1H), 2.72 - 2.42 (m, 2H), 2.25 - 2.20 (m, 1H), 2.09 - 2.00 (m, 1H), 1.93 - 1.88 (m, 1H), 1.18 (d, *J* = 6.6 Hz, 6H). |
| 73 | 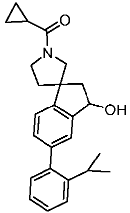 | cyclopropyl(3-hydroxy-5-(2-isopropylphenyl)-2,3-dihydrospiro[indene-1,3'-pyrrolidine]-1'-yl) ketone | LC-MS: (M+H)⁺; m/z = 376. 17; |
| | | | ¹H NMR (400 MHz, CDCl₃) δ 7.41 - 7.32 (m, 3H), 7. 27 - 7. 14 (m, 4H), 5. 38 - 5. 33 (m, 1H), 4.02 - 3. 53 (m, 4H), 3.08 - 3.01 (m, 1H), 2.65 - 1.90 (m, 6H), 1. 18 (d, *J* = 6.8 Hz, 6H), 1.08 - 1. 03 (m, 2H), 0.88 - 0.74 (m, 2H). |
| 74 | 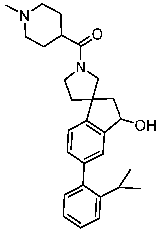 | (3-hydroxy-5-(2-isopropylphenyl)-2,3-dihydrospiro[indene-1,3'-pyrrolidine]-1'-yl)(1-methylpiperidin-4-yl) ketone | LC-MS: (M+H)⁺; m/z = 433. 27; |
| | | | ¹H NMR (400 MHz, CDCl₃) δ 7. 45 - 7. 31 (m, 4H), 7.24 - 7.14 (m, 3H), 5. 37-5. 27 (m, 1H), 3.86 - 3. 51 (m, 4H), 3. 19 - 2. 86 (m, 6H), 2.75 - 1. 86 (m, 12H), 1. 17 (d, *J* = 6.6 Hz, 6H). |
| 75 | 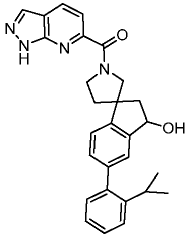 | (3-hydroxy-5-(2-isopropylphenyl)-2,3-dihydrospiro[indene-1,3'-pyrrolidine]-1'-yl)(1Hpyrazolo[3, 4-b]pyridin-6-yl) ketone | LC-MS: (M+H)⁺; m/z = 453. 35; |
| | | | ¹H NMR (400 MHz, CDCl₃) δ 10.41 (s, 1H), 8.38 - 8.31 (m, 1H), 8.09 - 8.02 (m, 1H), 7.95 - 7.89 (m, 1H), 7.41 - 7.26 (m, 5H), 7.23 - 7.13 (m, 2H), 5.41 - 5. 27 (m, 1H), 4. 36 - 3. 84 (m, 4H), 3.10 - 2.99 (m, 1H), 2. 73 - 2.41 (m, 2H), 2.30 - 2.19 - 2.05 (m, 3H), 1. 18 (d, *J* = 6.8 Hz, 6H). |
| 76 | 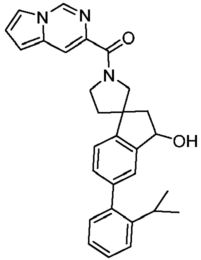 | (3-hydroxy-5-(2-isopropylphenyl)-2,3-dihydrospiro[indene-1,3'-pyrrolidine]-1'-yl)(pyrrolo[1,2-c]pyrimidin-3-yl) ketone | LC-MS: (M+H)⁺; m/z = 452. 12; |
| | | | ¹H NMR (400 MHz, CDCl₃) δ 8. 78 - 8. 69 (m, 1H), 8.08 - 8.06 (m, 1H), 7.52 - 7.26 (m, 6H), 7. 22 - 7.13 (m, 2H), 7.00 - 6.94 (m, 1H), 6.69 - 6.66 (m, 1H), 5.38 - 5.27 (m, 1H), 4. 28 - 3. 78 (m, 4H), 3.07 - 3.00 (m, 1H), 2.70 - 2.37 (m, 2H), 2.23 - 2. 18 (m, 1H), 2. 15 - 2.09 (m, 1H), 2. 06 - 1.98 (m, 1H), 1.17 (d, *J* = 6.4 Hz, 6H). |
| 77 | 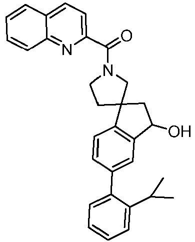 | (3-hydroxy-5-(2-isopropylphenyl)-2,3-dihydrospiro[indene-1,3'-pyrrolidine]-1'-yl)(quinolin-2-yl) ketone | LC-MS: (M+H)⁺; m/z=463. 18; |
| | | | ¹H NMR (400 MHz, CDCl₃) δ 8.40 - 8.25 (m, 2H), 8.18 - 8.05 (m, 1H), 8.03 - 7.93 (m, 1H), 7.89 - 7.80 (m, 1H), 7.78 - 7.69 (m, 1H), 7.65 - 7.54 (m, 1H), 7.41 - 7.27 (m, 4H), 7.25 - 7.12 (m, 2H), 5.40 - 5.25 (m, 1H), 4.38 - 3.86 (m, 4H), 3. 23 - 2. 98 (m, 1H), 2.74 - 2.41 (m, 2H), 2. 33 - 2. 18 (m, 1H), 2. 16 - 2.05 (m, 1H), 2. 02 - 1.86 (m, 1H), 1.26 (d, *J* = 6.8 Hz, 6H). |

### Example 78 : (5-fluoropyridin-2-yl)(3-hydroxy-5-(5,6,7,8-tetrahydroimidazo[1,2-a]pyridin-6-yl)-2,3-dihydrospiro[indene-1,3'-pyrrolidine]-1'-yl) ketone

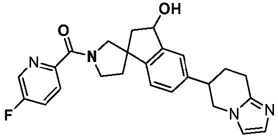

### Step A: 5-(imidazo[1,2-a]pyridin-6-yl)spiro[indene-1,3'-pyrrolidine]-3,5'(2H)-dione

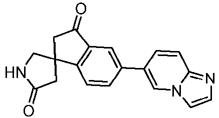

100 mg (0.36 mmol, 1.0 eq) 5-bromospiro[indene-1,3'-pyrrolidine]-3,5'(2*H*)-dione, 105 mg (0.43 mmol, 1.2 eq) 6-(4,4,5,5-tetramethyl-1,3,2-dioxaborolane-2-yl)imidazo[1,2-a] pyridine, 227 mg (1.07 mmol, 3.0 eq) potassium phosphate, 26.1 mg (0.04 mmol, 0.1 eq) [1,1'-bis(diphenylphosphine)ferrocene] palladium dichloride dichloromethane complex, and 8 mL (1,4-dioxane/water=4:1) mixed solvent were added in a 20 mL Shrek tube. Nitrogen replacement was carried out for three times, and then the reaction was carried out at 100°C for 3 h. It was confirmed that the product had been generated through LCMS, the reaction solution was added to 10 mL water for quenching, and then extracted three times with ethyl acetate (20 mL). The organic phases were combined, dried, concentrated. The crude product was purified through column chromatography (PE/EA = 50:1~1/1) to obtain 105 mg brown oily product (yield =93%).
LC-MS: (M+H)⁺; m/z = 318.09;

### Step B: 5-(5,6,7,8-tetrahydroimidazo[1,2-a]pyridin-6-yl)-2,3-dihydrospiro[indene-1,3'-pyrrolidine]-3-ol

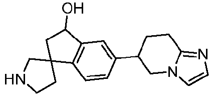

In a 50 mL Shrek tube, 120 mg (0.38 mmol, 1.0 eq) 5-(imidazo[1,2-a]pyridin-6-yl)spiro[indene-1,3'-pyrrolidine]-3,5'(2*H*)-dione was dissolved in 5 mL tetrahydrofuran. Under nitrogen protection, 144 mg (3.78 mmol, 10.0 eq) lithium aluminum tetrahydrogen was slowly added. After the addition, the system was heated up to 70 °C, and reacted for 3 h. It was confirmed that the product had been generated through LCMS. The reaction solution was cooled to -10 °C. 3 mL water was added slowly to quench the reaction, and then 20 mL tetrahydrofuran was added to the reaction system. It was filtered through diatomite. The filter cake was washed with tetrahydrofuran (20mLx3). The filtrate was concentrated and dried to obtain 158 mg yellow oily liquid crude product, which was used directly for the next reaction step.
LC-MS: (M+H)⁺; m/z = 310.12;

### Step C: (5-fluoropyridin-2-yl)(3-hydroxy-5-(5,6,7,8-tetrahydroimidazol[1,2-a]pyridin-6-yl)-2,3-dihydrospiro[indene-1,3'-pyrrolidine]-1'-yl) ketone

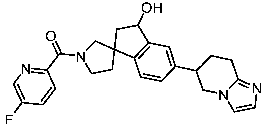

65.7 mg (0.47 mmol, 1.2 eq) 5-fluoropyridin-2-carboxylic acid., 177 mg (0.47 mmol, 1.2 eq) 2-(7-azobenzotriazole)-N,N,N',N'-tetramethylurea hexafluorophosphate, 301 mg (2.33 mmol, 6.0 eq) N,N-diisopropylethylamine and 6 mL tetrahydrofuran were added to a 25 mL reaction flask. Under nitrogen protection, the reaction solution was stirred at room temperature for 10 min, and then, 120mg (0.39mmol, 1.0eq) solution of 5-(5,6,7,8-tetrahydroimidazo[1,2-a]pyridin-6-yl)-2,3-dihydrospiro[indene-1,3'-pyrrolidine]-3-ol dissolved in 4 mL tetrahydrofuran was added to the reaction solution. Stirring was carried out at room temperature for 2 h. It was confirmed that the targent product had been generated through LCMS. The reaction solution was concentrated to dryness, and sent for preparation and purification (mobile phase A: 0.1% formic acid aqueous solution; mobile phase B: acetonitrile; gradient: 30% -70% B, 55 min; flow rate: 70mL/min) to obtain a white solid pure product (25.2 mg, yield =15%).
LC-MS: (M+H)⁺; m/z=433.15;
¹H NMR (400 MHz, CDCl₃) δ 8.46-8.31 (m, 1H), 8.02 - 7.91 (m, 1H), 7.54 - 7.44 (m, 1H), 7.22 - 6.83 (m, 5H), 5.28-5.18 (m, 2H), 4.11 - 3.56 (m, 6H), 2.60 - 2.19 (m, 2H), 2.13 - 1.94 (m, 5H), 1.32 - 1.14 (m, 3H).
¹⁹F NMR (376 MHz, CDCl₃) δ -122.93 (d, *J =* 75.2 Hz).

### Example 79 : (5-fluoropyridin-2-yl)(3-hydroxy-5-(imidazo[1,2-a]pyridin-6-yl)-2,3-dihydrospiro[indene-1,3'-pyrrolidine]-1'-yl) ketone

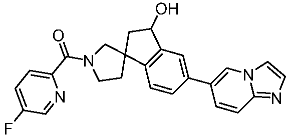

100mg (0.26mmol, 1.0eq) (5-bromo-3-hydroxy-2,3-dihydrospiro[indene-1,3'-pyrrolidine]-1'-yl)(5-fluoropyridin-2-yl) ketone, 74.9 mg (0.31 mmol, 1.2 eq) 6-(4,4,5,5-tetramethyl-1,3,2-dioxaborolane-2-yl)imidazo[1,2-a] pyridine, 163 mg (0.77 mmol, 3.0 eq) potassium phosphate, 18.7 mg (0.03 mmol, 0.1 eq) [1,1'-bis(diphenylphosphine)ferrocene] palladium dichloride dichloromethane complex, and 6 mL (1,4-dioxane/water=4:1) mixed solvent were added in a 20 mL Shrek tube. Nitrogen replacement was carried out for three times, and then the reaction was carried out at 100°C for 3 h. It was confirmed that the product had been generated through LCMS. The reaction solution was added to 10 mL water for quenching, and then extracted three times with ethyl acetate (20 mL). The organic phases were combined, dried, concentrated. The crude product was sent for preparation and purification (mobile phase A: 0.1% formic acid aqueous solution; mobile phase B: acetonitrile; gradient: 30% -70% B, 55 min; flow rate: 70mL/min) to obtain a white solid pure product.
LC-MS: (M+H)⁺; m/z = 433.15.
¹H NMR (400 MHz, CDCl₃) δ 8.47 - 8.29 (m, 1H), 8.30 (d, *J =* 8.8 Hz, 1H), 8.05 (dd, *J =* 8.4, 4.2 Hz, 1H), 7.68 - 7.47 (m, 6H), 7.42 - 7.32 (m, 2H), 5.40-5.31 (m, 1H), 4.25 - 3.75 (m, 4H), 2.70 - 2.34 (m, 2H), 2.23 - 2.01 (m, 3H).
19F NMR (376 MHz, CDCl₃) δ -123.10 (dd, *J =* 18.8, 7.5 Hz).

### Example 80: (5-(2-ethylphenyl)-3-hydroxy-2,3-dihydrospiro[indene-1,3'-pyrrolidine]-1'-yl)(5-fluoropyridin-2-yl) ketone

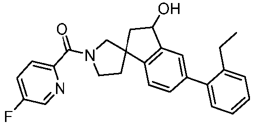

### Step A: 5-(2-ethylphenyl)spiro[indene-1,3'-pyrrolidine]-3,5'(2H)-dione

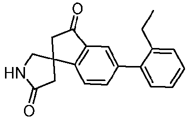

100.0 mg (0.4 mmol, 1.0 eq) 5-bromospiro[indene-1,3'-pyrrolidine]-3,5'(2*H*)-dione was dissolved in 2 mL1,4-dioxane, and then 53.5 mg (0.4 mmol, 1.0 eq) 2-ethylphenylboronic acid, 20.6mg (0.02mmol, 0.05eq) tetrakis(triphenylphosphine) palladium, 98.7mg (0.7mmol, 2.0eq) potassium carbonate and 0.4 mL water were added to the reaction solution. After replacing with nitrogen, it was heated to 100 °C, and reacted for 2 h. After confirming the complete reaction through LC-MS results, the system was returned to room temperature, and diluted with 5mL saturated sodium chloride solution, and then extracted three times with 10 mL ethyl acetate. The organic phases were taken, dried over anhydrous sodium sulfate, and evaporated under reduced pressure to obtain 5-(2-ethylphenyl)spiro[indene-1,3'-pyrrolidine]-3,5'(2*H*)-dione (109 mg, yield =100%)
LC-MS: (M+H)⁺; m/z = 306;

### Step B: 5-(2-ethylphenyl)-2,3-dihydrospiro[indene-1,3'-pyrrolidine]-3-ol

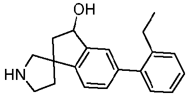

109.0 mg (0.4 mmol, 1.0 eq) 5-(2-ethylphenyl)spiro[indene-1,3'-pyrrolidine]-3,5'(2*H*)-dione was dissolved in 3 mL anhydrous tetrahydrofuran, and after replacing with nitrogen, the system was cooled to 0 °C. Then, 40.6 mg (1.1 mmol, 3.0 eq) lithium aluminum tetrahydrogen was slowly added dropwise to the former, after adding, it was heated to 75°C, and the reaction proceeded for 3 h. After confirming the complete reaction through LC-MS results, and the system was cooled to 0 °C, quenched using 10 µL water and 10µL 15% sodium hydroxide aqueous solution in sequence, and then dried over anhydrous magnesium sulfate. The filtered filtrate was concentrated by vacuum distillation to obtain 5-(2-ethylphenyl)-2,3-dihydrospiro[indene-1,3'-pyrrolidine]-3-ol (105 mg, yield =100%).
LC-MS: (M+H)⁺; m/z = 294;

### Step C: (5-(2-ethylphenyl)-3-hydroxy-2,3-dihydrospiro[indene-1,3'-pyrrolidine]-1'-yl)(5-fluoropyridin-2-yl) ketone

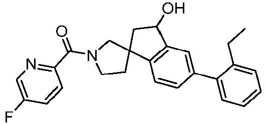

50.4 mg (0.4mmol, 1.0eq) 5-fluoro-2-pyridinecarboxylic acid was dissolved 2mL *N, N-*dimethylformamide. After replacing with nitrogen, 149.3 mg (0.4 mmol, 1.1 eq) 2-(7-azobenzotriazole)-N,N,N',N'-tetramethylurea hexafluorophosphate and 92.2 mg (0.7 mmol, 2.0 eq) N,N-diisopropylethylamine were added in sequence. Stirring was carried out at room temperature for 1 min, and then 105 mg (0.4 mmol, 1.0 eq) 5-(2-ethylphenyl)-2,3-dihydrospiro[indene-1,3'-pyrrolidine]-3-ol was added. Subsequently, stirring was proceeded for 30 min. After confirming the complete reaction through LC-MS results, 10 mL saturated sodium chloride solution was used to dilute, and then extraction was carried out with 10 mL ethyl acetate for three times. The organic phases were taken, dried over anhydrous sodium sulfate, and evaporated under reduced pressure. The residue was purified through column chromatography (EA/PE = 0~1:1) and preparative chromatography (mobile phase A: 0.1% trifluoroacetic acid; mobile phase B: acetonitrile; gradient: 30% -70% B, 55 min; flow rate: 70mL/min) in sequence to obtain (5-(2-ethylphenyl)-3-hydroxy-2,3-dihydrospiro[indene-1,3'-pyrrolidine]-1'-yl)(5-fluoropyridin-2-yl) ketone as a white solid (76.9mg, yield =52%).
LC-MS: (M+H)⁺; m/z = 417.14;
¹H NMR (400 MHz, CDCl₃) δ 8.51 - 8.33 (m, 1H), 8.05 (dd, *J =* 8.5, 4.4 Hz, 1H), 7.55 - 7.47 (m, 1H), 7.39 - 7.27 (m, 5H), 7.23 - 7.16 (m, 2H), 5.35 - 5.32 (m, 1H), 4.21 - 3.81 (m, 4H), 2.68 - 2.03 (m, 7H), 1.13 (t, *J =* 8.0 Hz, 3H).

Examples in Table 4 were prepared by using the method described in Examples 79 or 80 above, but wherein the desired different boric acid or borate ester for replacing were prepared by Suzuki reaction and other methods.

**Table 4: Examples 81^{~}102**

| Exa mpl e No. | Structure | Compound Name | Spectral Data |
|---|---|---|---|
| 81 | 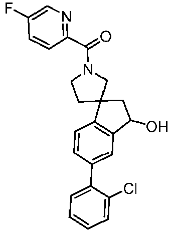 | (5-(2-chlorophenyl)-3-hydroxy-2,3-dihydrospiro[indene-1,3'-pyrrolidine]-1'-yl)(5-fluoropyridin-2-yl) ketone | LC-MS: (M+H)⁺; m/z = 423. 15; |
| | | | ¹H NMR (400 MHz, CDCl₃) δ 8.47 - 8.36 (m, 1H), 8.07-8.02 (m, 1H), 7.55 - 7.41 (m, 4H), 7.36-7.26 (m, 4H), 5.38 - 5.30 (m, 1H), 4.24 - 3.78 (m, 4H), 2.68 - 2.01 (m, 5H). |
| | | | ¹⁹F NMR (376 MHz, CDC13) δ -123.25 (dd, *J* = 18.8, 7.5 Hz). |
| 82 | 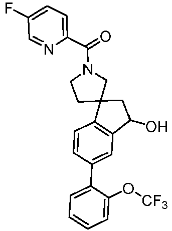 | (5-fluoropyridin-2-yl)(3-hydroxy-5-(2-(trifluoromethoxy)phenyl)-2,3-dihydrospiro[indene-1,3'-pyrrolidine]-1'-yl) ketone | LC-MS: (M+H)⁺; m/z = 473. 15; |
| | | | ¹H NMR (400 MHz, CDCl₃) δ 8. 49 - 8.35 (m, 1H), 8.08 - 8.01 (m, 1H), 7.52 (d, *J* = 4.6 Hz, 1H), 7. 48 - 7.42 (m, 2H), 7.39 (d, *J* = 7.8 Hz, 1H), 7. 38 - 7.34 (m, 3H), 7.32 - 7.29 (m, 1H), 5. 36 - 5. 31 m, 1H), 4. 26 - 4. 03 (m, 2H), 4.00 - 3. 77 (m, 2H), 2. 69 - 2.39 (m, 1H), 2.27 - 1.75 (m, 4H). |
| 83 | 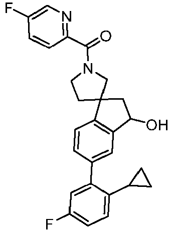 | (5-(2-cyclopropyl-5-fluorophenyl)-3-hydroxy-2,3-dihydrospiro[indene-1,3'-pyrrolidine]-1'-yl)(5-fluoropyridin-2-yl) ketone | LC-MS: (M+H)⁺; m/z = 447. 14; |
| | | | ¹H NMR (400 MHz, CDC13) δ 8.52 - 8.25 (m, 1H), 8.05 - 8.01 (m, 1H), 7.52 - 7.46 (m, 2H), 7.43 - 7.37 (m, 1H), 7.36 - 7.27 (m, 1H), 7.01 - 6.81 (m, 3H), 5.35 - 5.31 (m, 1H), 4.22 - 3.79 (m, 4H), 2.68 - 2.46 (m, 1H), 2.41 - 2.11 (m, 3H), 2.09 - 2.00 (m, 1H), 1.82 - 1.80 (m, 1H), 0.84 - 0.79 (m, 2H), 0.66 - 0.63 (m, 2H). |
| 84 | 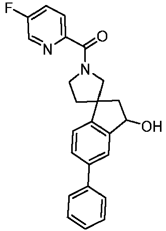 | (5-fluoropyridin-2-yl)(3-hydroxy-5-phenyl-2,3-dihydrospiro[indene-1,3'-pyrrolidine]-1'-yl) ketone | LC-MS: (M+H)⁺; m/z=389. 14; |
| | | | ¹H NMR (400 MHz, CDCl₃) δ 8.47 - 8.34 (m, 1H), 8.07 - 8.02 (m, 1H), 7.68 - 7.42 (m, 7H), 7.37-7.32 (m, 2H), 5.40 - 5.29 (m, 1H), 4.23 - 3.76 (m, 4H), 2.67 - 2.00 (m, 5H). |
| | | | ¹⁹F NMR (376 MHz, CDCl₃) δ -123. 20. |
| 85 | 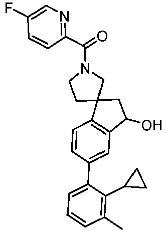 | (5-(2-cyclopropyl-3-methylphenyl)-3-hydroxy-2,3-dihydrospiro[indene-1,3'-pyrrolidine]-1'-yl)(5-fluoropyridin-2-yl) ketone | LC-MS: (M+H)⁺; m/z = 443. 11; |
| | | | ¹H NMR (400 MHz, CDCl₃) δ 8.52 - 8.36 (m, 1H), 8.08 - 8.05 (m, 1H), 7.61 - 7.44 (m, 2H), 7.42 - 7.39 (m, 1H), 7.28 - 7.25 (m, 1H), 7.21 - 7. 18 (m, 2H), 7. 16 - 7. 11 (m, 1H), 5.38 - 5.36 (m, 1H), 4.26 - 3.83 (m, 4H), 2.71 - 2.43 (m, 4H), 2.29 - 2.01 (m, 3H), 1.86 - 1.84 (m, 1H), 0.78 - 0.65 (m, 2H), 0.15 - 0.03 (m, 2H). |
| 86 | 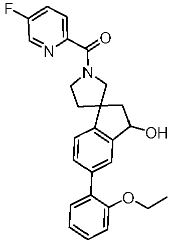 | (5-(2-ethoxyphenyl)-3-hydroxy-2,3-dihydrospiro[indene-1,3'-pyrrolidine]-1'-yl)(5-fluoropyridin-2-yl) ketone | LC-MS: (M+H-OH)⁺; m/z =4 15. 31; |
| | | | ¹H NMR (400 MHz, CDCl₃) δ 8.47 - 8.36 (m, 1H), 8.07 - 8.02 (m, 1H), 7. 65 - 7.46 (m, 3H), 7.35 - 7.26 (m, 3H), 7.04 - 6.96 (m, 2H), 5.36 - 5.28 (m, 1H), 4. 23 - 3. 78 (m, 6H), 2.66 - 2.00 (m, 4H), 1.85 - 1.83 (m, 1H), 1. 38 (t, *J* = 8.0 Hz, 3H). |
| | | | ¹⁹F NMR (376 MHz, CDCl₃) δ -123.33 (dd, *J* = 39. 5, 13.2 Hz ). |
| 87 | 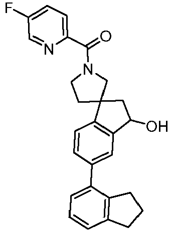 | (5-(2,3-dihydro-1*H*-*indene-*4-yl)-3-hydroxy-2,3-dihydrospiro[indene-1,3'-pyrrolidine]-1'-yl)(5-fluoropyridin-2-yl) ketone | LC-MS: (M+H)⁺; m/z=429. 12; |
| | | | ¹H NMR (400 MHz, CDCl₃) δ 8.47 - 8.35 (m, 1H), 8.07-8.02 (m, 1H), 7.54 - 7.41 (m, 3H), 7.32-7.27 (m, 1H), 7.25 - 7. 15 (m, 3H), 5.39 - 5.27 (m, 1H), 4.24 - 3. 77 (m, 4H), 3.00 - 2. 94 (m, 4H), 2.67 - 2. 46 (m, 1H), 2. 44 - 2.00 (m, 6H). |
| | | | ¹⁹F NMR (376 MHz, CDCl₃) δ -123. 25. |
| 88 | 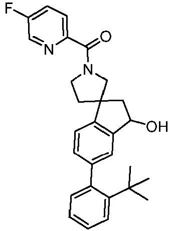 | (5-(2-(tert-butyl)phenyl)-3-hydroxy-2,3-dihydrospiro[indene-1,3'-pyrrolidine]-1'-yl)(5-fluoropyridin-2-yl) ketone | LC-MS: (M+H)⁺; m/z = 445. 18; |
| | | | ¹H NMR (400 MHz, CDCl₃) δ 8.49 - 8.36 (m, 1H), 8.07 - 8.01 (m, 1H), 7.56 - 7.47 (m, 2H), 7.34 - 7.28 (m, 2H), 7.25 - 7.13 (m, 3H), 7.03 - 6.92 (m, 1H), 5.41 - 5.27 (m, 1H), 4.20 - 3.77 (m, 4H), 2.73 - 2.05 (m, 4H), 2.04 - 2.02 (m, 1H), 1.19 (s, 9H). |
| 89 | 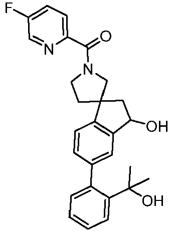 | (5-fluoropyridin-2-yl)(3-hydroxy-5-(2-(2-hydroxypropyl-2-)phenyl)-2,3-dihydrospiro[indene-1,3'-pyrrolidine]-1'-yl) ketone | LC-MS: (M+H)⁺; m/z = 447. 15 |
| 90 | 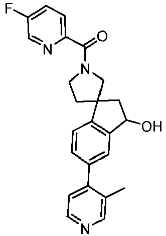 | (5-fluoropyridin-2-yl)(3-hydroxy-5-(3-methylpyridin-4-yl)-2,3-dihydrospiro[indene-1,3'-pyrrolidine]-1'-yl) ketone | LC-MS: (M+H)⁺; m/z = 404; |
| | | | ¹H NMR (400 MHz, CDCl₃) δ 8.51 - 8.36 (m, 3H), 8. 05 (dd, *J* = 9. 0, 4. 6 Hz, 1H), 7. 55 - 7. 48 (m, 1H), 7. 42 - 7. 30 (m, 3H), 7.15 (dd, *J* = 11. 5, 5.0 Hz, 1H), 5.41 - 5. 30 (m, 1H), 4.25 - 3. 77 (m, 4H), 2. 70 - 2.37 (m, 2H), 2.29 (s, 3H), 2. 25 - 2. 17 (m, 1H), 2. 15 - 1.98 (m, 2H). |
| | | | ¹⁹F NMR (376 MHz, CDCl₃) δ -123. 12. |
| 91 | 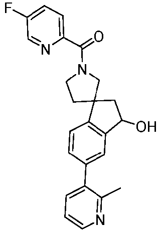 | (5-fluoropyridin-2-yl)(3-hydroxy-5-(2-methylpyridin-3-yl)-2,3-dihydrospiro[indene-1,3'-pyrrolidine]-1'-yl) ketone | LC-MS: (M+H)⁺; m/z = 404.14; |
| | | | ¹H NMR (400 MHz, CDCl₃) δ 8.51-8.49 (m, 1H), 8.47 - 8.36 (m, 1H), 8.05 (dd, *J* = 8.8, 4.3 Hz, 1H), 7. 54 - 7. 49 (m, 2H), 7. 40 - 7. 29 (m, 3H), 7. 21-7. 19 (m, 1H), 5. 41 - 5. 30 (m, 1H), 4. 25 - 3. 77 (m, 4H), 2. 70 - 2. 55 (m, 1H), 2. 52 (d, *J* = 7. 2 Hz, 3H), 2. 49 - 2. 35 (m, 1H), 2. 26 - 2. 17 (m, 1H), 2. 15 - 1. 98 (m, 2H). |
| | | | ¹⁹F NMR (376 MHz, CDCl₃) δ -123. 17 (dd, *J =* 20. 7, 4.3 Hz ). |
| 92 | 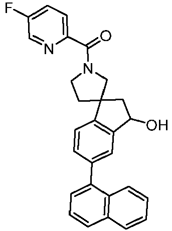 | (5-fluoropyridin-2-yl)(3-hydroxy-5-(naphthalen-1-yl)-2,3-dihydrospiro[indene-1,3'-pyrrolidine]-1'-yl) ketone | LC-MS: (M+H)⁺; m/z=439. 10; |
| | | | ¹H NMR (400 MHz, CDCl₃) δ 8.45 (dd, *J* = 34. 3, 3.2 Hz, 1H), 8.07-8.02 (m, 1H), 7. 92 - 7.86 (m, 3H), 7. 58 - 7. 34 (m, 8H), 5.44-5.33 (m, 1H), 4. 26 - 3. 83 (m, 4H), 2.71 - 2.06 (m, 5H). ¹⁹F NMR (376 MHz, CDCl₃) δ -122. 49 (dd, *J =* 11. 0, 4.7 Hz). |
| 93 | 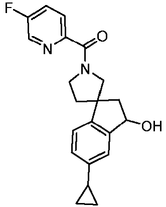 | (5-cyclopropyl-3-hydroxy-2,3-dihydrospiro[indene-1,3'-pyrrolidine]-1'-yl)(5-fluoropyridin-2-yl) ketone | LC-MS: (M+H)⁺; m/z = 353. 18; |
| | | | ¹H NMR (400 MHz, CDCl₃) δ 8.49 - 8.31 (m, 1H), 8.08 - 7.97 (m, 1H), 7.61 - 7.42 (m, 2H), 7.20 - 7.04 (m, 2H), 5.37 - 5.24 (m, 1H), 4.18 - 3.96 (m, 2H), 3.91 - 3.72 (m, 2H), 2.61 - 2.42 (m, 1H), 2.38 - 2.27 (m, 1H), 2.16 - 2.13 (m, 1H), 2.07 -1.97 (m, 2H), 1.95 - 1.88 (m, 1H), 1.04 - 0.92 (m, 2H), 0.88 - 0.82 (m, 2H). |
| 94 | 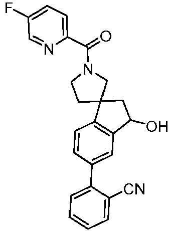 | 2-(1'-(5-fluoropyridinecarbonyl)-3-hydroxy-2,3-dihydrospiro[indene-1,3'-pyrrolidine]-5-yl)benzonitrile | LC-MS: (M+H)⁺; m/z = 414. 18; |
| | | | ¹H NMR (400 MHz, CDCl₃) δ 8.47 - 8.37 (m, 1H), 8.08 - 8.02 (m, 1H), 7.79 - 7.76 (m, 1H), 7.68 - 7.59 (m, 2H), 7.55 - 7.43 (m, 4H), 7.40 - 7.34 (m, 1H), 5.40 - 5.38 (m, 1H), 4.23 - 4.03 (m, 2H), 3.99 - 3.74 (m, 2H), 2.69 - 2.48 (m, 1H), 2.45 - 2. 11 (m, 3H), 2.09 - 2.02 (m, 1H). |
| 95 | 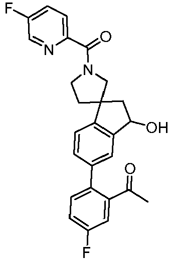 | 1-(5-fluoro-2-(1'-(5-fluoropyridoyl)-3-hydroxy-2,3-dihydrospiro[indene-1,3'-pyrrolidine]-5-yl)phenyl)ethan-1-one | LC-MS: (M+H)⁺; m/z = 449. 12 |
| 96 | 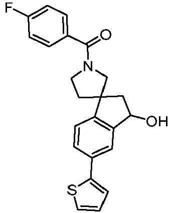 | (4-fluorophenyl)(3-hydroxy-5-(thieno-2-yl)-2,3-dihydrospiro[indene-1,3'-pyrrolidine]-1'-yl) ketone | LC-MS: (M+H)⁺; m/z = 395.09; |
| | | | ¹H NMR (400 MHz, CDCl₃) δ 8.46 - 8.35 (m, 1H), 8.06 - 8.02 (m, 1H), 7.72 - 7. 54 (m, 2H), 7. 21 - 7. 47 (m, 1H), 7.34 - 7. 27 (m, 3H), 7. 12 - 7. 05 (m, 1H), 5.37 - 5. 27 (m, 1H), 4. 23 - 3. 70 (m, 4H), 2.64 - 2. 37 (m, 1H), 2.24 - 1.99 (m, 3H), 1. 93 -1.88 (m, 1H). |
| 97 | 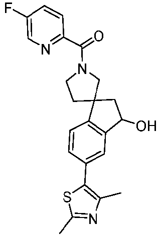 | (5-(2,4-dimethylthiazol-5-yl)-3-hydroxy-2,3-dihydrospiro[indene-1,3'-pyrrolidine]-1'-yl)(5-fluoropyridin-2-yl) ketone | LC-MS: (M+H)⁺; m/z = 424. 12 |
| 98 | 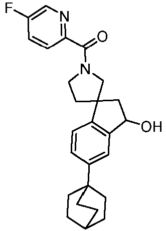 | (5-(bicyclo[2.2.2]octan-1-yl)-3-hydroxy-2,3-dihydrospiro[indene-1,3'-pyrrolidine]-1'-yl)(5-fluoropyridin-2-yl) ketone | LC-MS: (M+H)⁺; m/z = 421. 18 |
| 99 | 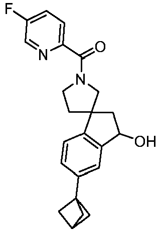 | (5-(bicyclo[1.1.1]pentan-1-yl)-3-hydroxy-2,3-dihydrospiro[indene-1,3'-pyrrolidine]-1'-yl)(5-fluoropyridin-2-yl) ketone | LC-MS: (M+H)⁺; m/z = 439. 15 |
| 100 | 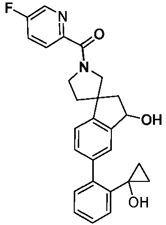 | (5-fluoropyridin-2-yl)(3-hydroxy-5-(2-(1-hydroxylcyclopropyl)phenyl )-2,3-dihydrospiro[indene-1,3'-pyrrolidine]-1'-yl) ketone | LC-MS: (M+H)⁺; m/z = 445. 14 |
| 101 | 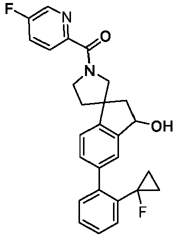 | (5-(2-(1-fluorocyclopropyl)phenyl)-3-hydroxy-2,3-dihydrospiro[indene-1,3'-pyrrolidine]-1'-yl)(5-fluoropyridin-2-yl) ketone | LC-MS: (M+H)⁺; m/z = 447. 14 |
| 102 | 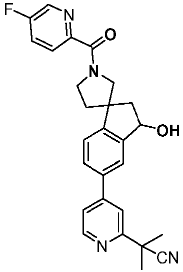 | 2-(4-(1'-(5-fluoropyridoyl)-3-hydroxy-2,3-dihydrospiro[indene-1,3'-pyrrolidine]-5-yl)pyridin-2-yl)-2-methyl propionitrile | LC-MS: (M+H)⁺; m/z = 457. 20; |
| | | | ¹H NMR (400 MHz, CDCl₃) δ 8. 65 - 8. 63 (m, 1H), 8.49 - 8.30 (m, 1H), 8. 08 - 8.03 (m, 1H), 7. 79 (d, *J* = 8.0 Hz, 1H), 7.71 (d, *J* = 11.8 Hz, 1H), 7.65 - 7. 62 (m, 1H), 7.56 - 7.48 (m, 1H), 7.46 - 7. 34 (m, 2H), 5.41 - 5. 34 (m, 1H), 4.25 - 4.01 (m, 2H), 3. 95 - 3.76 (m, 2H), 2.70 - 2. 40 (m, 1H), 2. 26 - 1. 86 (m, 4H), 1.81 (s, 6H). |

### Example 103 : (5-fluoropyridin-2-yl)(3-hydroxy-6-(2-isopropylphenyl)-2,3-dihydrospiro[indene-1,3'-pyrrolidine]-1'-yl) ketone

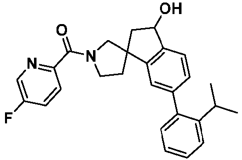

### Step A: diethyl 3-(3-bromophenyl)-3-cyanopentanedioate

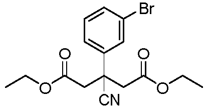

5.00 g (25.5 mmol, 1.0 eq) 2-(3-bromophenyl)acetonitrile was dissolved in 50 mL anhydrous tetrahydrofuran, and nitrogen replacement was carried out for three times. The temperature was reduced to -60 °C using dry ice. 5.00 g (56.1 mmol, 2.2 eq) lithium bis(trimethylsilyl)amine (lithium hexamethyldisilazide) was added dropwise at -60 °C, and stirring was carried out at room temperature for 3 h. Subsequently, 8.94 g (53.6 mmol, 2.0 eq) ethyl bromoacetate was added dropwise below -60 °C, and stirring was carried out overnight at room temperature. After confirming that the raw materials had reacted completely through LC-MS, 50 mL water was added for quenching, and then extraction was carried out with ethyl acetate for three times. The organic layer was washed with 50 mL saturated saline solution, dried over anhydrous sodium sulfate and evaporated under reduced pressure to obtain a crude product (10.6 g, yield =100%).
LC-MS: (M+Na+H)⁺; m/z = 391.87, 393.91.

### Step B : ethyl 2-(3-(3-bromophenyl)-5-oxopyrrolidin-3-yl)acetate

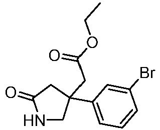

10.0 g (27.2 mmol, 1.0 eq) diethyl 3-(3-bromophenyl)-3-cyanopentanedioate was dissolved in 50 mL methanol, and 7.05 g (54.31 mmol, 2.0 eq) anhydrous cobalt chloride was added. Then, 8.22 g (217.3 mmol, 8.0 eq) sodium borohydride was slowly added at 0 °C, and stirring was carried out at room temperature for 1 h. After confirming that the product had been generated through LC-MS, 50 mL hydrochloric acid solution (3mol/L) was used to dilute, and then, extraction was carried out with dichloromethane for three times. The organic layer was washed with 50 mL saturated saline solution, dried over anhydrous sodium sulfate and evaporated under reduced pressure to obtain a crude product (7.66 g, yield =86%).
LC-MS: (M+H)⁺; m/z = 326.08, 327.95.

### Step C : 2-(3-(3-bromophenyl)-5-oxopyrrolidin-3-yl)acetic acid

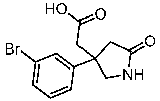

1.00 g (3.07 mmol, 1.0 eq) ethyl 2-(3(3-bromophenyl)-5-oxopyrrolidin-3-yl)acetate was dissolved in 20 mL methanol, and 4.9 mL sodium hydroxide solution (5mmol/mL) was slowly added at 0 °C, and stirring was carried out at 40 °C for 1 h. After confirming that the product had been generated through LC-MS, the reaction solution was diluted with hydrochloric acid solution (3.0 mol/L) to acidity, and then extracted three times with dichloromethane. The organic layer was washed with 10 mL saturated saline solution, dried over anhydrous sodium sulfate and evaporated under reduced pressure to obtain a crude product (0.86g, yield =94%).
LC-MS: (M-H)⁺; m/z = 297.98, 299.76.

### Step D : 6-bromopyrrole[indene-1,3'-pyrrolidine]-3,5'-2H dione

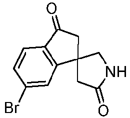

4.00 g polyphosphoric acid was heated to 150 °C, and then 0.86 g (2.88 mmol, 1.0 eq) 2-(3-(3-bromophenyl)-5-oxopyrrolidin-3-yl)acetic acid was added. Then, stirring was carried out at 150 °C for 1 h. After confirming that the product had been generated through LCMS, the mixture was slowly poured into ice water while it was still hot, and stirred while adding, and then extracted three times with dichloromethane. The organic layer was washed with 30 mL saturated saline solution, dried over anhydrous sodium sulfate and evaporated under reduced pressure. The residue was subjected to column chromatography (DCM/MeOH = 0~20:1) to obtain the product (0.39 g, yield =48%).
LC-MS: (M+H)⁺; m/z = 279.91, 281.91.

### Step E: 6-(2-isopropylphenyl)spiro[indene-1,3'-pyrrolidine]-3,5'-2H-dione

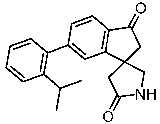

150mg (0.53mmol, 1.0eq) 6-bromopyrrole[indene-1,3'-pyrrolidine]-3,5'-2H dione and 110mg (0.64mmol, 1.2eq) (2-cyclopropylphenyl)boric acid were dissolved in 5mL dioxane/water mixed solution (V:V = 4: 1), and then 30 mg (0.05 mmol, 0.1 eq) [1,1'-bis(diphenylphosphine)ferrocene] dichloride palladium and 0.34g (1.6 mmol, 3.0eq) tripotassium phosphate were added. Nitrogen replacement was carried out for three times, and stirring was carried out at 100 °C for 1 h. After confirming that the product had been generated through LCMS, 50 mL water was added thereto, and then extraction was carried out with ethyl acetate for three times. The organic layer was washed with 5 mL saturated saline solution, dried over anhydrous sodium sulfate and evaporated under reduced pressure obtain a crude product (162 mg, yield =93%).
LC-MS: (M+H)⁺; m/z=320.10.

### Step F : 6-(2-isopropylphenyl)-2,3-dihydrospiro[indene-1,3'-pyrrolidine]-3 -ol

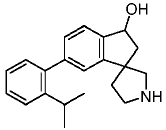

162mg (0.50mmol, 1.0eq) 5-bromopyrrole[indene-1,3'-pyrrolidine]-3,5'-2H-dione was was dissolved in 20 mL tetrahydrofuran, and then 0.29 g (7.51 mmol, 15.0 eq) lithium aluminum hydride was slowly added at 0°C. Stirring was carried out at 70 °C for 2 h. After confirming that the product had been generated through LC-MS, the reaction solution was filtered through diatomite and washed with tetrahydrofuran. After drying over anhydrous sodium sulfate and evaporating under reduced pressure, a crude product was obtained (110 mg, yield =71%).
LC-MS: (M+H)⁺; m/z = 308.20.

### Step G : (6-(2-isopropylphenyl)-3-hydroxy-2,3-dihydrospiro[indene-1,3'-pyrrolidine]-1'-yl)(5-fluoropyridin-2-yl) ketone

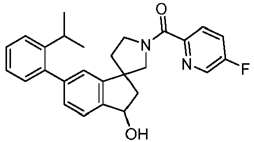

76.0 mg (0.54 mmol, 1.5 eq) 5-fluoropyridin-2-carboxylic acid, 0.17 mL *N,N-*diisopropylethylamine, and 204mg (2.06mmol, 1.5eq) 2-(7-azobenzotriazole)-*N,N,N',N'-*tetramethylurea hexafluorophosphate were dissolved in 5 mL tetrahydrofuran, and then stirring was carried out at room temperature for 0.5 h. 110 mg (0.36 mmol, 1.0 eq) 6-(2-isopropylphenyl)-2,3-dihydrospiro[indene-1,3'-pyrrolidine]-3-ol was added, and then stirring was carried out overnight at room temperature. After confirming that the product had been generated through LC-MS, 10 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min), a white solid was obtained (16.0 mg, yield =10%).
LC-MS: (M+H)⁺; m/z = 431.13;
¹H NMR (400 MHz, CDCl₃) δ 8.44 - 8.36 (m, 1H), 8.04 - 7.98 (m, 1H), 7.51 - 7.44 (m, 2H), 7.40 - 7.33 (m, 3H), 7.23 - 7.13 (m, 3H), 5.42-5.31 (m, 1H), 4.16 - 3.74 (m, 4H), 3.04 - 2.95 (m, 1H), 2.70 - 2.33 (m, 2H), 2.28 - 2.17 (m, 1H), 2.15 - 2.10 (m, 1H), 2.08 - 2.01 (m, 1H), 1.17 - 1.12 (d, *J =* 6.8 Hz, 6H).

### Example 104 : (5-fluoropyridin-2-yl)(3-hydroxy-5-(2-isopropylphenyloxy)-2,3-dihydrospiro[indene-1,3'-pyrrolidine]-1'-yl) ketone

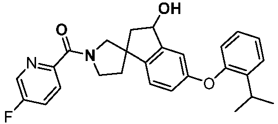

100.0 mg (0.256 mmol, 1.0 eq) (5-bromo-3-hydroxy-2,3-dihydrospiro[indene-1,3'-pyrrolidine]-1'-yl)(5-fluoropyridin-2-yl) ketone was dissolved in 2 mL 1,4-dioxane, and then, 38.3 mg (0.282 mmol, 1.1 eq) 2-isopropylphenol, 4.9 mg (0.0256 mmol, 0.1 eq) cuprous iodide, 7.3 mg (0.0512 mmol, 0.2 eq) trans-(1*R*,2*R*)- N, N'-dimethyl-1,2-cyclohexanediamine and 163.0 mg (0.768 mmol, 3.0 eq) potassium phosphate were added to the reaction system. After replacing with nitrogen, it was heated to 100 °C, and reacted for 18 h. After confirming the complete reaction through LC-MS results, the system was returned to room temperature, and diluted with 5mL saturated sodium chloride solution, and then extracted three times with 10 mL ethyl acetate. The organic phases were taken, dried over anhydrous sodium sulfate, and evaporated under reduced pressure. The residue was purified through preparative chromatography (mobile phase A: 0.1% formic acid; mobile phase B: acetonitrile; gradient: 30% -70% B, 55 min; flow rate: 70mL/min) to obtain (5-fluoropyridin-2-yl)(3-hydroxy-5-(2-isopropylphenyloxy)-2,3-dihydrospiro[indene-1,3'-pyrrolidine]-1'-yl) ketone as a yellow solid (2.0 mg, yield =2%).
LC-MS: (M+H)⁺; m/z = 447.17;
¹H NMR (400 MHz, CDCl₃) δ 8.45 - 8.40 (m, 1H), 8.03 (s, 1H), 7.56 - 7.45 (m, 1H), 7.38 - 7.33 (m, 1H), 7.23 - 7.08 (m, 3H), 7.00 - 6.80 (m, 3H), 5.37 - 5.18 (m, 1H), 4.17 - 3.97 (m, 2H), 3.92 - 3.69 (m, 2H), 3.31 - 3.20 (m, 1H), 2.62 - 2.45 (m, 1H), 2.38 - 2.21 (m, 1H), 2.18 - 2.09 (m, 1H), 2.08 - 1.95 (m, 2H), 1.22 (d, *J =* 7.0 Hz, 6H).

Examples in Table 5 were prepared by using the method described in Example 15 above, which were obtained by reacting the universal intermediate I1 with desired different phenols, thiophenols, aromatic amines, or aromatic Grignard reagents, followed by reduction and condensation.

**Table 5: Examples 105^{~}116**

| Exa mpl e No. | Structure | Compound Name | Spectral Data |
|---|---|---|---|
| 105 | 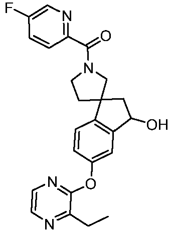 | (5-((3-ethylpyrazin-2-yl)oxy)-3-hydroxy-2,3-dihydrospiro[indene-1,3'-pyrrolidine]-1'-yl)(5-fluoropyridin-2-yl] ketone | LC-MS: (M+H)⁺; m/z = 435.14 |
| 106 | 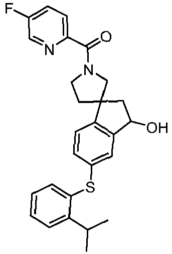 | (5-fluoropyridin-2-yl)(3-hydroxy-5-((2-isopropylphenyl)thio) -2,3-dihydrospiro[indene-1,3'-pyrrolidine]-1'-yl) ketone | LC-MS: (M+H)⁺; m/z = 463.12 |
| 107 | 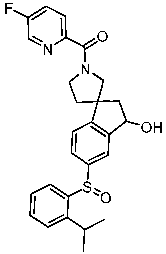 | (5-fluoropyridin-2-yl)(3-hydroxy-5-((2-isopropylphenyl)sulfo xide)-2,3-dihydrospiro[indene-1,3'-pyrrolidine]-1'-yl) ketone | LC-MS: (M+H)⁺; m/z = 479.13 |
| 108 | 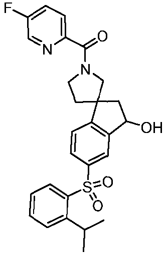 | (5-fluoropyridin-2-yl)(3-hydroxy-5-((2-isopropylphenyl)sulfo nyl)-2,3-dihydrospiro[indene-1,3'-pyrrolidine]-1'-yl) ketone | LC-MS: (M+H)⁺; m/z = 495.14 |
| 109 | 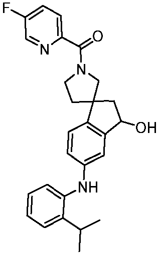 | (5-fluoropyridin-2-yl)(3-hydroxy-5-((2-isopropylphenyl)amino )-2,3-dihydrospiro[indene-1,3'-pyrrolidine]-1'-yl) ketone | LC-MS: (M+H)⁺; m/z = 446.13; |
| | | | ¹H NMR (400 MHz, CDCl₃) δ 8.45 - 8.35 (m, 1H), 8.02 (s, 1H), 7.50 (d, *J* = 8.6 Hz, 1H), 7.30 (s, 1H), 7.27 (d, *J* = 1.8 Hz, 2H), 7.15 - 7.10 (m, 2H), 6.93 - 6. 85 (m, 2H), 5.47 (s, 1H), 5.35 - 5. 24 (m, 1H), 4. 29 - 3.67 (m, 4H), 3.14 - 3. 13 (m, 1H), 2. 53 - 2.49 (m, 1H), 2.35 - 1. 95 (m, 3H), 1. 79 - 1.78 (m, 1H), 1.32 - 1.20 (d, *J* = 6.8 Hz, 6H). |
| 110 | 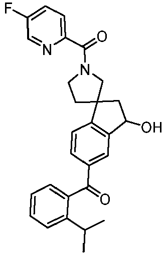 | (1'-(5-fluoropyridinecarbony l)-3-hydroxy-2,3-dihydrospiro[indene-1,3'-pyrrolidine]-5-yl)(2-isopropylphenyl) ketone | LC-MS: (M+H)⁺; m/z = 459.15 |
| 111 | 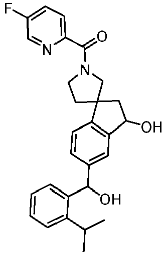 | (5-fluoropyridin-2-yl)(3-hydroxy-5-(hydroxyl(2-isopropylphenyl)methy l)-2,3-dihydrospiro[indene-1,3'-pyrrolidine]-1'-yl) ketone | LC-MS: (M+H)⁺; m/z = 461.20 |
| 112 | 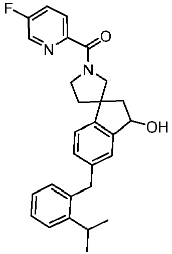 | (5-fluoropyridin-2-yl)(3-hydroxy-5-(2-isopropyl benzyl)-2,3-dihydrospiro[indene-1,3'-pyrrolidine]-1'-yl) ketone | LC-MS: (M+H)⁺; m/z = 445.19 |
| 113 | 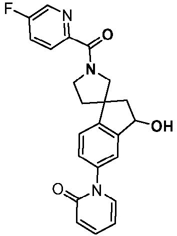 | 1-(1'-(5-fluoropyridoyl)-3-hydroxy-2,3-dihydrospiro[indene-1,3'-pyrrolidine]-5-yl)pyridin-2(1*H*) -one | LC-MS: (M+H)⁺; m/z = 406.09 |
| 114 | 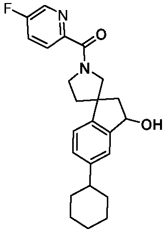 | (5-cyclohexyl-3-hydroxy-2,3-dihydrospiro[indene-1,3'-pyrrolidine]-1'-yl)(5-fluoropyridin-2-yl) ketone | LC-MS: (M+H)⁺; m/z = 395.17 |
| 115 | 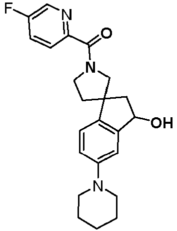 | (5-fluoropyridin-2-yl)(3-hydroxy-5-(piperidin-1-yl)-2,3-dihydrospiro[indene-1,3'-pyrrolidine]-1'-yl) ketone | LC-MS: (M+H)⁺; m/z = 396.11 |

Examples in Table 6 were prepared by using the method described in Example 14 above, which were prepared by replacing different raw materials and reagents in the reactions.

**Table 6: Examples 116^{~}133**

| Exa mpl e No. | Structure | Compound Name | Spectral Data |
|---|---|---|---|
| 116 | 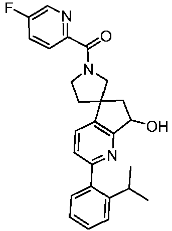 | (5-fluoropyridin-2-yl)(7-hydroxy-2-(2-isopropylphenyl)-6,7-dihydrospiro[cyclopen tyl[b]pyridin-5,3'-pyrrolidine]-1'-yl) ketone | LC-MS: (M+H)⁺; m/z = 432.15 |
| 117 | 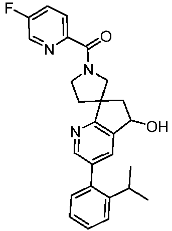 | (5-fluoropyridin-2-yl)(5-hydroxy-3-(2-isopropylphenyl)-5,6-dihydrospiro[cyclopen tyl[b]pyridin-7,3'-pyrrolidine]-1'-yl) ketone | LC-MS: (M+H)⁺; m/z = 432.17 |
| 118 | 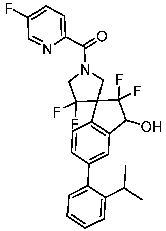 | (5-fluoropyridin-2-yl)(2,2,4',4'-tetrafluoro-3-hydroxy-5-(2-isopropylphenyl)-2,3-dihydrospiro[indene-1,3'-pyrrolidine]-1'-yl) ketone | LC-MS: (M+H)⁺; m/z = 503.15 |
| 119 | 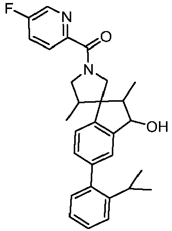 | (5-fluoropyridin-2-yl)(3-hydroxy-5-(2-isopropylphenyl)-2,4'-dimethyl-2,3-dihydrospiro[indene-1,3'-pyrrolidine]-1'-yl) ketone | LC-MS: (M+H)⁺; m/z = 459.20 |
| 120 | 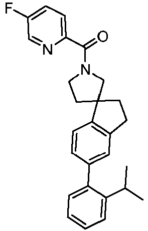 | (5-fluoropyridin-2-yl)(5-(2-isopropylphenyl)-2,3-dihydrospiro[indene-1,3'-pyrrolidine]-1'-yl) ketone | LC-MS: (M+H)⁺; m/z = 415.09 |
| 121 | 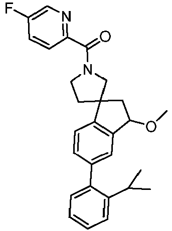 | (5-fluoropyridin-2-yl)(5-(2-isopropylphenyl)-3-methoxy-2,3-dihydrospiro[indene-1,3'-pyrrolidine]-1'-yl) ketone | LC-MS: (M+H)⁺; m/z = 445.20 |
| 122 | 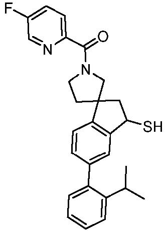 | (5-fluoropyridin-2-yl)(5-(2-isopropylphenyl)-3-mercapto-2,3-dihydrospiro[indene-1,3'-pyrrolidine]-1'-yl) ketone | LC-MS: (M+H)⁺; m/z = 447.12 |
| 123 | 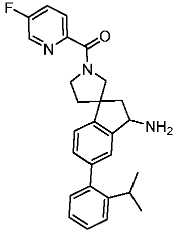 | (3-amino-5-(2-isopropylphenyl)-2,3-dihydrospiro[indene-1,3'-pyrrolidine]-1'-yl)(5-fluoropyridin-2-yl) ketone | LC-MS: (M+H)⁺; m/z = 430.19 |
| 124 | 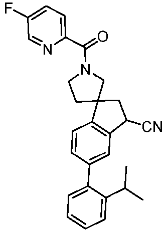 | 1'-(5-fluoropyridoyl)-5-(2-isopropylphenyl)-2,3-dihydrospiro[indene-1,3'-pyrrolidine]-3-nitrile | LC-MS: (M+H)⁺; m/z = 440.17 |
| 125 | 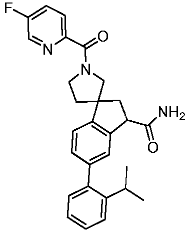 | 1'-(5-fluoropyridoyl)-5-(2-isopropylphenyl)-2,3-dihydrospiro[indene-1,3'-pyrrolidine]-3-carboxamide | LC-MS: (M+H)⁺; m/z = 458.18 |
| 126 | 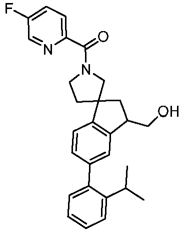 | (5-fluoropyridin-2-yl)(3-(methylol)-5-(2-isopropylphenyl)-2,3-dihydrospiro[indene-1,3'-pyrrolidine]-1'-yl) ketone | LC-MS: (M+H)⁺; m/z = 445.20 |
| 127 | 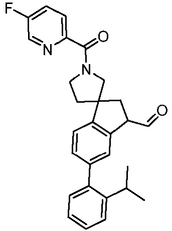 | 1'-(5-fluoropyridoyl)-5-(2-isopropylphenyl)-2,3-dihydrospiro[indene-1,3'-pyrrolidine]-3-carboxaldehyde | LC-MS: (M+H)⁺; m/z = 443.18 |
| 128 | 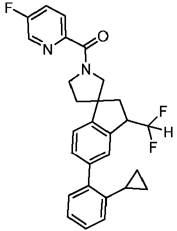 | (5-(2-cyclopropylphenyl)-3-(difluoromethyl)-2,3-dihydrospiro[indene-1,3'-pyrrolidine]-1'-yl)(5-fluoropyridin-2-yl) ketone | LC-MS: (M+H)+; m/z = 463.15 |
| 129 | 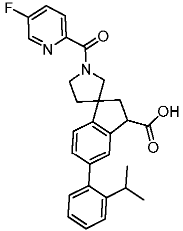 | 1'-(5-fluoropyridoyl)-5-(2-isopropylphenyl)-2,3-dihydrospiro[indene-1,3'-pyrrolidine]-3-carboxylic acid | LC-MS: (M+H)⁺; m/z = 459.16 |
| 130 | 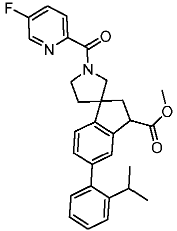 | methyl 1'-(5-fluoropyridoyl)-5-(2-isopropylphenyl)-2,3-dihydrospiro[indene-1,3'-pyrrolidine]-3-carboxylate | LC-MS: (M+H)⁺; m/z = 473.16 |
| 131 | 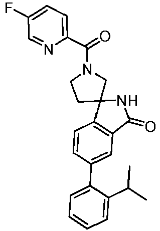 | 1'-(5-fluoropyridoyl)-5-(2-isopropylphenyl) spiro [isoindole-1,3'-pyrrolidine]-3-one | LC-MS: (M+H)⁺; m/z = 430.14 |
| 132 | 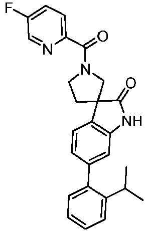 | 1'-(5-fluoropyridoyl)-6-(2-isopropylphenyl) spiro [indene-3,3'-pyrrolidine]-2-one | LC-MS: (M+H)⁺; m/z = 430.15 |
| 133 | 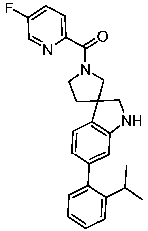 | (5-fluoropyridin-2-yl)(6-(2-isopropylphenyl) spiro [indene-3, 3'-pyrrolidine]-1'-yl) ketone | LC-MS: (M+H)⁺; m/z = 416.18 |

### Example 134 : 1'-((5-fluoropyridin-2-yl)methyl)-5-(2-isopropylphenyl)-2,3-dihydrospiro[indene-1,3'-pyrrolidine]-3-ol

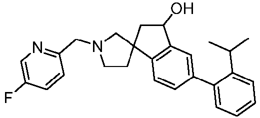

50.0 mg (0.16 mmol, 1.0 eq) 5-(2-isopropylphenyl)-2,3-dihydrospiro[indene-1,3'-pyrrolidine]-3-ol, 24.5 mg (0.19 mmol, 1.2 eq) 5-fluoropyridine aldehyde, 5 mL methanol were added to a 25 mL reaction flask, and then the temperature was reduced to 0 °C. 20.5 mg (0.32 mmol, 2.0 eq) sodium cyanoborohydride was added to the reaction solution, and the temperature was raised to room temperature. Stirring was carried out for 20 min. It was confirmed that the raw materials had reacted completely through LCMS. 2 mL water was added to the reaction solution for quenching. It was concentrated to to dryness, and sent for preparation (mobile phase A: 0.1% formic acid; mobile phase B: acetonitrile; gradient: 30%-70% B, 55 min; flow rate: 70mL/min). A white solid pure product was obtained (12.3 mg, yield =18%)
LC-MS: (M+H)⁺; m/z = 417.16;
¹H NMR (400 MHz, CDCl₃) δ 8.40 (d, *J =* 2.8 Hz, 1H), 7.42 - 7.32 (m, 4H), 7.28 - 7.26 (m, 2H), 7.23 - 7.15 (m, 3H), 5.29 - 5.21 (m, 1H), 3.88 - 3.84 (m, 2H), 3.08 - 2.89 (m, 2H), 2.76 - 2.68 (m, 2H), 2.37 - 2.33 (m, 1H), 2.24 - 2.13 (m, 2H), 2.09 - 1.99 (m, 2H), 1.17 (d, *J* = 6.8 Hz, 6H).
¹⁹F NMR (376 MHz, CDCl₃) δ -129.86.

### Example 135 : 1'-((5-chloropyridin-2-yl)sulfonyl)-5-(2-isopropylphenyl)-2,3-dihydrospiro[indene-1,3'-pyrrolidine]-3-ol

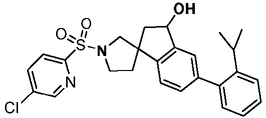

34.6 mg (0.16 mmol, 1.0 eq) 5-chloropyridine-2-sulfonyl chloride, 49.5 mg (0.48 mmol, 3.0 eq) triethylamine, 50.0 mg (0.16 mmol, 1.0 eq) 5-(2-isopropylphenyl)-2,3-dihydrospiro[indene-1,3'-pyrrolidine]-3-ol and 3 mL tetrahydrofuran were added to a 25 mL reaction flask. Under nitrogen protection, stirring was carried out at room temperature for 1 h. It was confirmed that the raw materials had reacted completely through through TLC Plate (DCM/MeOH = 20:1). The reaction solution was concentrated to dryness, and sent for preparation and purification (mobile phase A: 0.1% formic acid; mobile phase B: acetonitrile; gradient: 30% -70% B, 55 min; flow rate: 70mL/min), and freeze-dried to obtain a white solid pure product (7.19 mg, yield =9%).
LC-MS: (M+H)⁺; m/z=483.05;
¹H NMR (400 MHz, CDCl₃) δ 8.71 - 8.70 (m, 1H), 7.99 - 7.89 (m, 2H), 7.40 - 7.31 (m, 3H), 7.26 - 7.12 (m, 4H), 5.36 - 5.26 (m, 1H), 3.90 - 3.66 (m, 3H), 3.59 (m, 1H), 3.04 - 2.97 (m, 1H), 2.52 - 2.30 (m, 2H), 2.17 - 1.94 (m, 2H), 1.85 - 1.80 (m, 1H), 1.17 (d, *J =* 6.6 Hz, 6H).

Examples in Table 7 were prepared by using the method described in Example 135 above, which were obtained under alkaline conditions by replacing different raw materials in the reactions.

**Table 7: Examples 136^{~}146**

| Exa mpl e No. | Structure | Compound Name | Spectral Data |
|---|---|---|---|
| 136 | 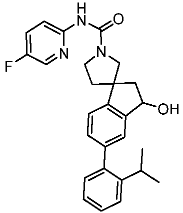 | *N*-(5-fluoropyridin-2-yl)-3-hydroxy-5-(2-isopropylphenyl)-2, 3-dihydrospiro[indene-1,3'-pyrrolidine]-1'-carboxamide | LC-MS: (M+H)⁺; m/z = 446. 20 |
| 137 | 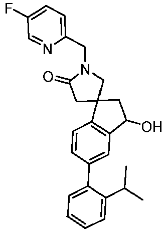 | 1'-((5-fluoropyridin-2-yl)methyl)-3-hydroxy-5-(2-isopropylphenyl)-2,3-dihydrospiro[indene-1,3'-pyrrolidine]-5'-one | LC-MS: (M+H)⁺; m/z = 431. 18 |
| 138 | 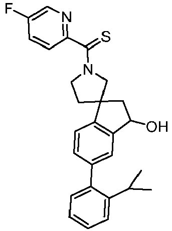 | (5-fluoropyridin-2-yl) (3-hydroxy-5-(2-isopropylphenyl)-2, 3-dihydrospiro[indene-1,3'-pyrrolidine]-1'-yl)methanethione | LC-MS: (M+H)⁺; m/z = 447. 15 |
| 139 | 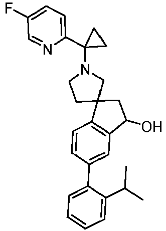 | 1'-(1-(5-fluoropyridin-2-yl)cyclopropyl)-5-(2-isopropylphenyl)-2,3-dihydrospiro[indene-1,3'-pyrrolidine]-3-ol | LC-MS: (M+H)⁺; m/z = 443.19 |
| 140 | 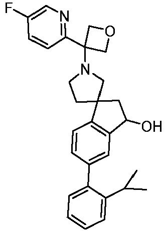 | 1'-(3-(5-fluoropyridin-2-yl)oxetane-3-yl)-5-(2-isopropylphenyl)-2,3-dihydrospiro[indene-1,3'-pyrrolidine]-3-ol | LC-MS: (M+H)⁺; m/z = 459. 21 |
| 141 | 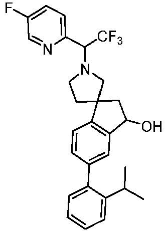 | 5-(2-isopropylphenyl)-1'-(2,2,2-trifluoro-1-(5-fluoropyridin-2-yl) ethyl)-2,3-dihydrospiro[indene-1,3'-pyrrolidine]-3-ol | LC-MS: (M+H)⁺; m/z = 485.17 |
| 142 | 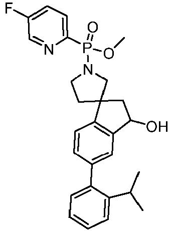 | methyl (5-fluoropyridin-2-yl)(3-hydroxy-5-(2-isopropylphenyl)-2,3-dihydrospiro[indene-1,3'-pyrrolidine]-1'-yl)phosphonate | LC-MS: (M+H)⁺; m/z = 481.03 |
| 143 | 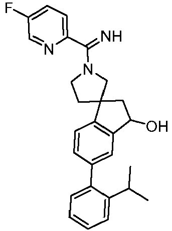 | 1'-((5-fluoropyridin-2-yl)(imino)methyl)-5-(2-isopropylphenyl)-2,3-dihydrospiro[indene-1,3'-pyrrolidine]-3-ol | LC-MS: (M+H)⁺; m/z = 430.09 |
| 144 | 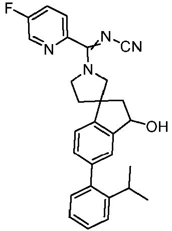 | *N*-((5-fluoropyridin-2-yl)(3-hydroxy-5-(2-isopropylphenyl)-2,3-dihydrospiro[indene-1,3'-pyrrolidine]-1'-yl)methylene)cyanamide | LC-MS: (M+H)⁺; m/z = 455. 16 |
| 145 | 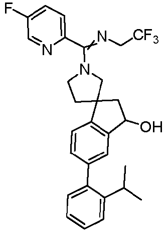 | 1'-((5-fluoropyridin-2-yl)((2,2,2-trifluoroethyl)imino)methyl)-5-(2-isopropylphenyl)-2,3-dihydrospiro[indene-1,3'-pyrrolidine]-3-ol | LC-MS: (M+H)⁺; m/z = 512.16 |
| 146 | 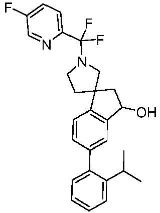 | 1'-(difluoro(5-fluoropyridin-2-yl)methyl)-5-(2-isopropylphenyl)-2,3-dihydrospiro[indene-1,3'-pyrrolidine]-3-ol | LC-MS: (M+H)⁺; m/z = 453.15 |

### Example 147 : (5-fluoropyridin-2-yl)(3-hydroxy-2,3-dihydrospiro[indene-1,3'-pyrrolidine]-1'-yl) ketone

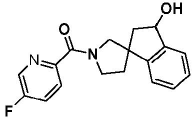

Experimental procedure referred to Example 15, but wherein the starting raw material p-bromophenylacetonitrile was replaced with phenylacetonitrile to synthesize Example 147.
LC-MS: (M+H)⁺; m/z = 313.07

### Example 148 : (5-fluoropyridin-2-yl)(5-bromo-3-hydroxy-2,3-dihydrospiro[indene-1,3'-pyrrolidine]-1'-yl) ketone

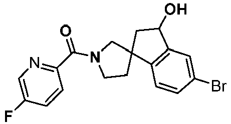

Compound I5 in Preparation Example 5 for Intermediate is Example 148.

LC-MS: (M+H)⁺; m/z = 391.01.

### Example 149: 1'-(5-chloropyridin-2-yl)-5-(2-cyclopropylphenyl)-2,3-dihydrospiro[indene-1,3'-pyrrolidine]-3-ol

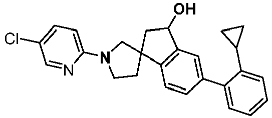

100 mg (327 µmol, 1.0 eq) 5-(2-cyclopropylphenyl)-2,3-dihydrospiro[indene-1,3'-pyrrolidine]-3-ol was dissolved in 10 mL *N*, *N*-dimethylformamide, and then 94.0 mg (491 µmol, 1.5 eq) 2-bromo-5-chloropyridine and 213 mg (654 µmol, 2.0 eq) cesium carbonate were added. Nitrogen replacement was carried out for three times, and stirring was carried out at 100 °C for 3 h. After confirming that the product had been generated through LCMS, 10 mL salt water was added thereto, and then extraction was carried out with ethyl acetate for three times. After drying over anhydrous sodium sulfate, evaporating under reduced pressure, preparation and purification (mobile phase A: 0.1% formic acid; mobile phase B: acetonitrile; gradient: 30%-70% B, 55 min; flow rate: 70mL/min), a white solid was obtained (17.0 mg, yield =12%).
LC-MS : (M+H)⁺; m/z = 417.15;
¹H NMR (400 MHz, CDCl₃) δ 8.13 (dd, *J =* 5.9, 2.5 Hz, 1H), 7.56 - 7.50 (m, 1H), 7.47 - 7.36 (m, 2H), 7.30 (dd, *J* = 6.4, 2.4 Hz, 1H), 7.27 - 7.19 (m, 3H), 6.94 (d, *J* = 7.7 Hz, 1H), 6.43 - 6.32 (m, 1H), 5.45 - 5.34 (m, 1H), 3.87 - 3.54 (m, 4H), 2.63 - 2.61 (m, 1H), 2.49 - 2.41 (m, 1H), 2.31 - 2.29 (m, 1H), 2.25 - 2.08 (m, 2H), 1.93 - 1.87 (m, 1H), 0.92 - 0.80 (m, 2H), 0.79 - 0.70 (m, 2H).

### Example 150 : 5-(2-cyclopropylphenyl)-1'-(6-fluorobenzo[c]isothiazol-3-yl)-2,3-dihydrospiro[indene-1,3'-pyrrolidine]-3-ol

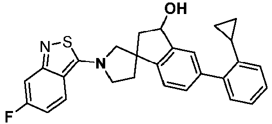

20.0mg (65.0µmol, 1.0eq) 5-(2-cyclopropylphenyl)-2,3-dihydrospiro[indene-1,3'-pyrrolidine]-3-ol, 24.5 mg (131 µmol, 2.0 eq) 3-chloro-6-fluorobenzo[c]isothiazole, 25.3 mg (196 µmol, 3.0 eq) *N,N*-diisopropylethylamine and 3 mL DMF were added to a 25 mL reaction flask. Nitrogen replacement was carried out for three times. The temperature was raised to 80 °C, and stirring was carried out overnight. It was confirmed that the raw materials had reacted completely through LC-MS. After cooling down, 20mL water was added to the reaction solution. Extraction was carried out with 20mL ethyl acetate for three times. The organic phases were combined, dried over anhydrous sodium sulfate and concentrate after filtration. The crude product was purified through preparative chromatography (mobile phase A: 0.1% formic acid; mobile phase B: acetonitrile; gradient: 30% -70% B, 55 min; flow rate: 70mL/min), and freeze-dried to ontian a white solid (1.36 mg, yield =4%).
LC-MS: (M+H)⁺; m/z = 457.12;
¹H NMR (400 MHz, CDCl₃) δ 7.76 (dd, *J* = 9.8, 5.1 Hz, 1H), 7.54 - 7.51 (m, 1H), 7.47 - 7.44 (m, 1H), 7.31 - 7.27 (m, 2H), 7.24 - 7.21 (m, 2H), 7.07 (d, *J =* 10.7 Hz, 1H), 6.93 (d, *J =* 7.7 Hz, 1H), 6.67 - 6.63 (m, 1H), 5.41 (s, 1H), 3.98 - 3.76 (m, 4H), 2.69 - 2.58 (m, 2H), 2.47 - 2.42 (m, 1H), 2.31 - 2.18 (m, 2H), 1.90 - 1.83 (m, 1H), 0.87 - 0.84 (m, 2H), 0.75 - 0.71 (m, 2H).
¹⁹F NMR (376 MHz, CDCl₃) δ -112.83.

### Example 151 : 1'-(5-chlorobenzo[d]thiazol-2-yl)-5-(2-isopropylphenyl)-2,3-dihydrospiro[indene-1,3'-pyrrolidine]-3-ol

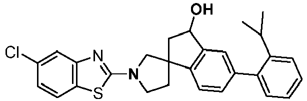

Experimental procedure referred to Example 149 in order to synthesize the compound of Example 151.
LC-MS: (M+H)⁺; m/z = 475.06

### Example 152 : 5-(2-cyclopropylphenyl)-1'-(7-chloroimidazo[1,2-a]pyridin-3-yl)-2,3-dihydrospiro[indene-1,3'-pyrrolidine]-3-ol

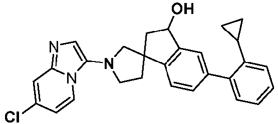

Intermediate I3 and 3-bromo-7-chloroimidazo[1,2-A] pyridine were used in prepareing by Buchwald Hartwig coupling reaction to obtain the compound of Example 152.
LC-MS: (M+H)⁺; m/z = 456.08

Examples in Table 8 were prepared by using the method described in Example 152 above, which were obtained by replacing different raw materials in the reactions and through Buchwald Hartwig coupling reaction.

**Table 8: Examples 153^{~}158**

| Exam ple No. | Structure | Compound Name | Spectral Data |
|---|---|---|---|
| 153 | 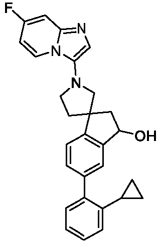 | 5-(2-cyclopropylphenyl)-1'-(7-fluoroimidazo[1,2-a]pyridin-3-yl)-2,3-dihydrospiro[indene-1,3'-pyrrolidine]-3-ol | LC-MS: (M+H) ⁺; m/z = 440.02 |
| 154 | 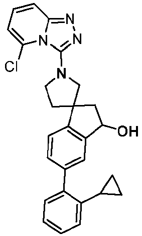 | 1'-(5-chloro-[1,2,4]triazolo[4,3-a]pyridin-3-yl)-5-(2-cyclopropylphenyl)-2,3-dihydrospiro[indene-1,3'-pyrrolidine]-3-ol | LC-MS: (M+H) ⁺; m/z = 457. 10 |
| 155 | 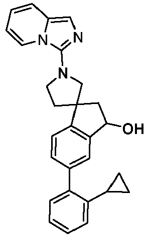 | 5-(2-cyclopropylphenyl)-1'-(imidazo[1,5-a]pyridin-3-yl)-2,3-dihydrospiro[indene-1,3'-pyrrolidine]-3-ol | LC-MS: (M+H) ⁺; m/z = 422.05 |
| 156 | 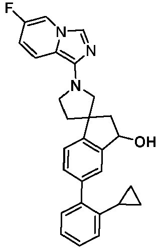 | 5-(2-cyclopropylphenyl)-1'-(6-fluoroimidazo[1,5-a]pyridin-1-yl)-2,3-dihydrospiro[indene-1,3'-pyrrolidine]-3-ol | LC-MS: (M+H) ⁺; m/z = 440. 10 |
| 157 | 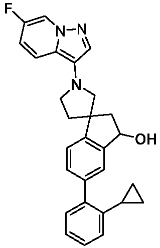 | 5-(2-cyclopropylphenyl)-1'-(6-fluoropyrazolo[1,5-a]pyridin-3-yl)-2,3-dihydrospiro[indene-1,3'-pyrrolidine]-3-ol | LC-MS: (M+H) ⁺; m/z = 440. 15 |
| 158 | 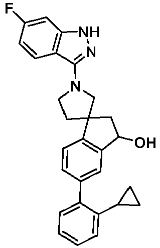 | 5-(2-cyclopropylphenyl)-1'-(6-fluoro-1*H*-*indazol-*3-yl)*-*2,3-dihydrospiro[indene-1,3'-pyrrolidine]-3-ol | LC-MS: (M+H)⁺; m/z = 440.17 |

### Example 159 : 2-(5-(2-cyclopropylphenyl)-3-hydroxy-2,3-dihydrospiro[indene-1,3'-pyrrolidine]-1'-carbonyl)-5-fluoropyridin-1-oxide

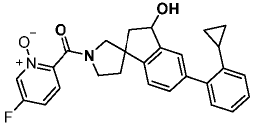

(5-(2-cyclopropylphenyl)-3-hydroxy-2,3-dihydrospiro[indene-1,3'-pyrrolidine]-1'-yl)(5-fluoropyridin-2-yl) ketone was dissolved in dichloromethane. Under an ice water bath, m-chloroperoxybenzoic acid was added to the mixture, and then Example 159 was obrianed by oxidation.
LC-MS: (M+H)⁺; m/z = 445.13

### Example 160 : 2-(3-(2'-cyclopropyl-3-(methylol)-[1,1'-biphenyl]-4-yl)pyrrolidine-1-carbonyl)-5-fluoropyridin-1-oxide

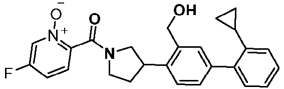

Experimental procedure referred to Example 159 in order to synthesize Example 160.
LC-MS: (M+H)⁺; m/z = 433.15

### Example 161 : (5-fluoropyridin-2-yl)(1-hydroxy-6-(2-isopropylphenyl)-1,3-dihydrospiro[indene-2,4'-piperidine]-1'-yl) ketone

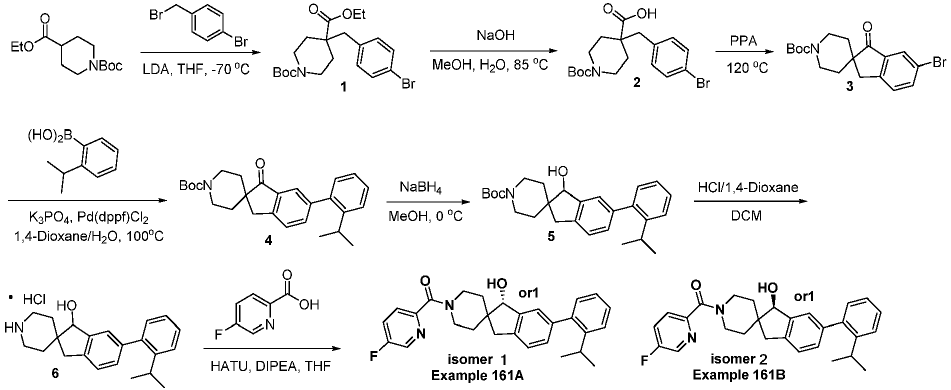

### Step A: 1-(tert butyl)4-ethyl-4-(4-bromobenzyl)piperidine-1,4-dicarboxylate

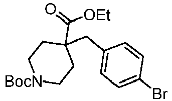

Under nitrogen protection, 10.0 g (38.9 mmol, 1.0 eq) ethyl N-Boc-piperidine-4-carboxylate and 60mL anhydrous tetrahydrofuran were added into a 250mL three necked bottle. It was controlled that 23.3mL (46.7mmol, 2M, 1.2eq) lithium diisopropylamide solution was added dropwise at -70 ~ -60°C. During the dropwise addition process, heat was released. After the dropwise addition, the reaction was thermally insulated for 1 hour. It was maintained that 9.70 g (38.9 mmol, 1.0 eq) solution of p-bromobenzyl bromide in tetrahydrofuran (25 mL) was added dropwise at -70~-60 °C. After the dropwise addition, it was let to naturally heat up. The reaction was carried out with stirring for 4 h. Sample was taken for LC-MS central control, and the reaction was considered complete when the residual amount of p-bromobenzyl bromide is less than 5%. The reaction solution was quenched by dropwise addition of saturated ammonium chloride solution (60mL) under a temperature control at 10-25 °C. Ethyl acetate (100mL) was added, and stirring was carried out for 10 min. After standing for 5 min, the liquid was separated. 50mL ethyl acetate was added to the aqueous phase, and extraction was carried out twice. The organic phases were combined, dried over anhydrous sodium sulfate, and concentrated under reduced pressure after filtration to obtain a crude product (13.8g), which was used directly in the subsequent reaction without purification.
LC-MS: (M-100+H)⁺; m/z = 325.98; 327.98.

### Step B: 4-(4-bromobenzyl)-1-( tert butoxycarbonyl)piperidin-4-carboxylic acid

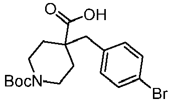

5.00 g (11.7 mmol, 1.0 eq) 1-(tert butyl)4-ethyl-4-(4-bromobenzyl)piperidin-1,4-dicarboxylate and 30mL methanol were added into a 250mL single necked flask. An aqueous solution (30mL) containing 2.80g (70.2mmol, 6.0eq) sodium hydroxide was added with stirring. Then the temperature was raised to 85 °C, and a reflux reaction was carried out for 24 h. It was detected that the raw materials had been completely converted through LC-MS. After cooling the reaction solution, methanol was evaporated by concentration under reduced pressure, and dichloromethane (150mL) was added. The lower organic phase was separated. The pH of the system was adjusted to 5-6 with 6M hydrochloric acid. After phase separation, the aqueous phase was extracted once with dichloromethane (150mL). The organic phases were combined, dried over anhydrous sodium sulfate, and concentrated under reduced pressure after filtration to obtain a light yellow oily substance (4.50 g, yield =96%).
LC-MS: (M-100+H)⁺; m/z = 297.92;299.92.

### Step C: tert-butyl 6-bromo-1-oxo-1,3-dihydrospiro[indene-2,4'-piperidine]-1'-carboxylate

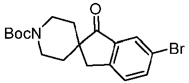

36.0 g polyphosphoric acid was added into a 250mL three necked bottle, and the temperature was raised to 120 °C. 4.50g (11.34mmol, 1.0eq) 4-(4-bromobenzyl)-1-(tert butoxycarbonyl)piperidin-4-carboxylic acid was dissolved in dichloromethane (20mL), and then the above solution was added dropwise with stirring. After the dropwise addition, reaction was carried out with stirring for 3 h. After detecting no remaining raw materials through LC-MS, the reaction was terminated. The reaction solution was slowly poured into 200g ice water while hot for quenching (quenching temperature at 25-35 °C). Then, The pH of the system was adjusted to 9-10 with sodium hydroxide aqueous solution. 3.70 g (17.0 mmol, 1.5 eq) Di-tertbutyl decarbonate was added, and stirring was carried out at room temperature for 3 h. The system was extracted twice with ethyl acetate. The organic phases were combined, dried over anhydrous sodium sulfate, and concentrated under reduced pressure after filtration to obtain a light yellow solid (4.00 g, yield =93%).
LC-MS: (M-100+H)⁺; m/z = 279.91; 281.90.

### Step D: tert-butyl 6-(2-isopropylphenyl)-1-oxo-1,3-dihydrospiro[indene-2,4'-piperidine]-1'-carboxylate

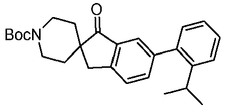

1.10 g (2.89 mmol, 1.0 eq) tert-butyl 6-bromo-1-oxo-1,3-dihydrospiro[indene-2,4'-piperidine]-1'-carboxylate and 569 mg (3.47 mmol, 1.2 eq) (2-isopropylphenyl)boric acid were dissolved in 10mL1,4-dioxane/water mixed solution (V:V = 4: 1), and then 210 mg (289 µmol, 0.1eq) [1,1'-bis(diphenylphosphine)ferrocene] dichloride palladium and 1.84 g (8.67 mmol, 3.0 eq) tripotassium phosphate were added. Nitrogen replacement was carried out for three times, and stirring was carried out at 100 °C for 1 h. After confirming that the product had been generated through LCMS, 50 mL water was added thereto, and then extraction was carried out with ethyl acetate for three times. The organic layer was washed with 30 mL saturated saline solution, dried over anhydrous sodium sulfate and evaporated under reduced pressure after filtration. The residue was subjected to column chromatography (petroleum ether: ethyl acetate=0~10:1) to obtain a light yellow solid (780 mg, yield =65%).
LC-MS: (M-100+H)⁺; m/z = 320.12;

### Step E: tert-butyl 1-hydroxy-6-(2-isopropylphenyl)-1,3-dihydrospiro[indene-2,4'-piperidine]-1'-carboxylate

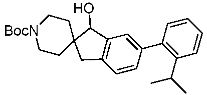

780 mg(1.86 mmol , 1.0 eq.) tert-butyl 6-(2-isopropylphenyl)-1-oxo-1,3-dihydrospiro[indene-2,4'-piperidine]-1'-carboxylate was dissolved in 10 mL anhydrous methanol, and 106 mg(2.79 mmol, 1.5 eq.) sodium borohydride was slowly added at 0 °C. After stirring under ice bath for 1 hour, it was send to LC-MS for confirmation of a complete conversion of raw materials and the generation of the product. The reaction solution was quenched with a saturated sodium carbonate aqueous solution, filtered through diatomite, and washed with ethyl acetate. The organic phase was separated, dried over anhydrous sodium sulfate and evaporated under reduced pressure after filtration to obtain a light yellow oily liquid (800 mg).
LC-MS: (M-100+H)⁺; m/z = 322.20;

### Step F: 6-(2-isopropylphenyl)-1,3-dihydrospiro[indene-2,4'-piperidine]-1-ol hydrochloride

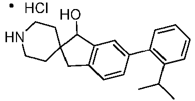

800 mg (1.89 mmol, 1.0 eq)tert-butyl 1-hydroxy-6-(2-isopropylphenyl)-1,3-dihydrospiro[indene-2,4'-piperidine]-1'-carboxylate was dissolved in 10 mL anhydrous dichloromethane, and 2.0mL HCl/1, 4-dioxane solution(4M) was added under an ice bath. After stirring under ice bath for 1 hour, it was send to LC-MS detection. The raw materials had been completely converted. After concentrating under reduced pressure, a light yellow solid was obtained (600 mg, yield =88%).
LC-MS: (M+H)⁺; m/z = 322.17;

### Step G: 6-(2-isopropylphenyl)-1,3-dihydrospiro[indene-2,4'-piperidine]-1-ol(5-fluoropyridin-2-yl)(1-hydroxy-6-(2-isopropylphenylphenyl)-1,3-dihydrospiro[indene-2,4'-piperidine]-1'-yl) ketone

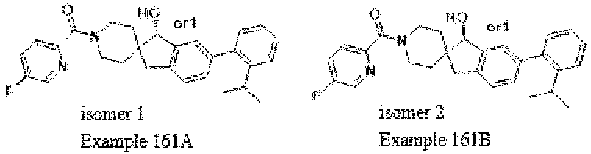

29.6 mg(209.6 µmol, 1.5 eq) 5-fluoropyridin-2-carboxylic acid, 90.1 mg (698.5 µmol, 5.0 eq) *N,N*-diisopropylethylamine and 79.7 mg (209.6 µmol, 1.5eq) 2-(7-azobenzotriazole)-*N,N,N',N'*-tetramethylurea hexafluorophosphate were dissolved in 5 mL tetrahydrofuran, and then stirring was carried out at room temperature for 10 min. 50 mg (139.7 µmol, 1.0 eq) 6-(2-isopropylphenyl)-1,3-dihydrospiro[indene-2,4'-piperidine]-1-ol hydrochloride was added, and then stirring was carried out at room temperature for 1.5 h. After confirming that the raw materials had been completely converted and the product had been generated through LC-MS, 50 mL saturated sodium chloride aqueous solution was added thereto, and then extraction was carried out with ethyl acetate for three times. After drying over anhydrous sodium sulfate, evaporating under reduced pressure after filteration, it was sent to HPLC preparation and purification (mobile phase A: 0.1% formic acid; mobile phase B: acetonitrile; gradient: 30%-70% B, 55 min; flow rate: 70mL/min), two isomers were obtained by freeze drying (Example 161A, 9.05 mg, yield =15%; Example 161A, 3.11 mg, yield =5%).

### Example 161A: LC-MS: (M+H)⁺; m/z = 427.23;

¹H NMR (400 MHz, CDCl₃) δ 8.43 (dd, *J =* 5.8, 2.8 Hz, 1H), 7.75 - 7.70 (m, 1H), 7.53 - 7.48 (m, 1H), 7.39 - 7.32 (m, 3H), 7.28 - 7.25 (m, 1H), 7.22 - 7.14 (m, 3H), 4.87 - 4.84 (m, 1H), 4.34 - 4.24 (m, 1H), 3.85 - 3.81 (m, 1H), 3.52 - 3.42 (m, 2H), 3.14 - 3.01 (m, 2H), 2.86 - 2.77 (m, 1H), 2.06 - 1.95 (m, 1H), 1.87 - 1.78 (m, 1H), 1.77 - 1.65 (m, 2H), 1.16 (d, *J =* 7.0 Hz, 6H).

¹⁹F NMR (376 MHz, CDCl₃) δ -123.95 (d, *J =* 2.9 Hz).

### Example 161B: LC-MS: (M+H)⁺; m/z=427.18;

¹H NMR (400 MHz, CDCl₃) δ 8.43 (dd, *J =* 5.8, 2.8 Hz, 1H), 7.75 - 7.70 (m, 1H), 7.56 - 7.51 (m, 1H), 7.39 - 7.32 (m, 3H), 7.27 - 7.25 (m, 1H), 7.22 - 7.14 (m, 3H), 4.87 - 4.84 (m, 1H), 4.34 - 4.24 (m, 1H), 3.85 - 3.81 (m, 1H), 3.52 - 3.42 (m, 2H), 3.14 - 2.99 (m, 2H), 2.88 - 2.83 (m, 1H), 2.05 - 1.96 (m, 1H), 1.87 - 1.77 (m, 1H), 1.74 - 1.65 (m, 2H), 1.16 (d, *J =* 7.0 Hz, 6H).

¹⁹F NMR (376 MHz, CDCl₃) δ -123.96 (d, *J =* 3.0 Hz).

### Example 162 : (S) -5-(2-cyclopropylphenyl)-1'-(5-fluoropyridoyl)spiro[indene-1,3'-pyrrolidine]-3(2H)-one

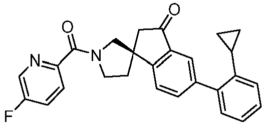

20.0mg (46.7µmol, 1.0eq) ((1*S*,3*R*)-5-(2-cyclopropylphenyl)-3-hydroxy-2,3-dihydrospiro[indene-1,3'-pyrrolidine]-1'-yl)(5-fluoropyridin-2-yl) ketone was dissolved in 1 mL dichloromethane, and then 23.8 mg (56.0 µmol, 1.2 eq.) Deiss Martin reagent was added to the reaction system. Stirring was carried out at room temperature for 2 h. After confirming the complete reaction through LC-MS results, the system was diluted with 5mL saturated sodium bicarbonate solution, and then extracted three times with 10 mL ethyl acetate. The organic phases were taken, dried over anhydrous sodium sulfate, and evaporated under reduced pressure. The residue was purified through preparative chromatography (mobile phase A: 0.1% formic acid; mobile phase B: acetonitrile; gradient: 30% -70% B, 55 min; flow rate: 70mL/min) to obtain (*S*) -5-(2-cyclopropylphenyl)-1'-(5-fluoropyridinecarbonyl)spiro[indene-1,3'-pyrrolidine]-3(2H)-one (11.7 mg, yield =58.8%) as a white solid (11.7 mg, yield =58.8%).
LC-MS: (M+H)⁺; m/z = 427.17;
¹H NMR (400 MHz, CDCl₃) δ 8.42 (dd, *J* = 44.0, 2.8 Hz, 1H), 8.09 (d, *J* = 4.1 Hz, 1H), 7.84 (d, *J =* 9.9 Hz, 1H), 7.78 (d, *J =* 7.9 Hz, 1H), 7.65 - 7.58 (m, 1H), 7.52 - 7.51 (m, 1H), 7.30 - 7.26 (m, 1H), 7.25 - 7.17 (m, 2H), 6.97 (dd, *J =* 7.7, 3.9 Hz, 1H), 4.38 - 4.10 (m, 2H), 4.09 - 3.86 (m, 2H), 2.93 - 2.69 (m, 2H), 2.45 - 2.43 (m, 1H), 2.18 - 2.17 (m, 1H), 1.84 - 1.76 (m, 1H), 0.87 - 0.83 (m, 2H), 0.73 - 0.70 (m, 2H).

### Example 163 : (5-(2-cyclopropyl-4-fluorophenyl)-3-hydroxy-2,3-dihydrospiro[indene-1,3'-pyrrolidine]-1'-yl)(5-fluoropyridin-2-yl) ketone

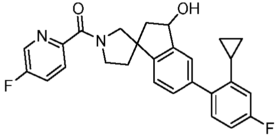

48.3mg (0.30mmol, 1.0eq) 5-fluoro-2-pyridinecarboxylic acid was dissolved in 2 mL N,N-dimethylformamide. After replacing with nitrogen, 143 mg (0.40 mmol, 1.1 eq) 2-(7-azobenzotriazole)-N,N,N',N'-tetramethylurea hexafluorophosphate and 88.5 mg (0.70 mmol, 2.0 eq) N,N-diisopropylethylamine were added in sequence. After stirring at room temperature for 1 min, 111 mg (0.30 mmol, 1.0 eq) 5- (2-cyclopropyl-4-fluorophenyl) -2,3-dihydrospiro[indene-1,3'-pyrrolidine]-3-ol was added, and stirring was proceeded for 30 min. After confirming the complete reaction through LC-MS results, 10 mL saturated sodium chloride solution was used to dilute, and extraction was carried out with 10 mL ethyl acetate for three times. The organic phases were taken, dried over anhydrous sodium sulfate, and evaporated under reduced pressure. The residue was purified through column chromatography (Methanol/dichloromethane=0~1:40) and preparative chromatography (mobile phase A: 0.1% trifluoroacetic acid; mobile phase B: acetonitrile; gradient: 30% -70% B, 55 min; flow rate: 70mL/min) in sequence to obtain (5-(2-cyclopropyl-4-fluorophenyl)-3-hydroxy-2,3-dihydrospiro[indene-1,3'-pyrrolidine]-1'-yl)(5-fluoropyridin-2-yl) ketone (86.4 mg, yield =57%) as a white solid.
LC-MS: (M+H⁺)⁺; m/z = 447.14;
¹H NMR (400 MHz, CDCl₃) δ 8.46 - 8.35 (m, 1H), 8.06 - 8.01 (m, 1H), 7.54 - 7.46 (m, 2H), 7.42 - 7.39 (m, 1H), 7.32 - 7.28 (m, 1H), 7.01 - 6.86 (m, 3H), 5.37 - 5.28 (m, 1H), 4.24 - 3.76 (m, 4H), 2.68 - 2.46 (m, 1H), 2.41 - 2.11 (m, 3H), 2.09 - 2.00 (m, 1H), 1.86 - 1.77 (m, 1H), 0.85 - 0.79 (m, 2H), 0.66 - 0.63 (m, 2H).

### Example 164 : 2-(2-(1'-(5-fluoropyridoyl)-3-hydroxy-2,3-dihydrospiro[indene-1,3'-pyrrolidine]-5-yl)phenyl)-2-methyl propionitrile

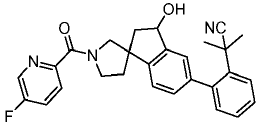

100 mg (256 µmol, 1.0 eq) (5-bromo-3-hydroxy-2,3-dihydrospiro[indene-1,3'-pyrrolidine]-1'-yl)(5-fluoropyridin-2-yl) ketone was dissolved in 2 mL 1,4-dioxane, and then 130 mg (511 µmol, 2.0 eq) bis(pinacolato)diboron, 18.7mg (25.6µmol, 0.1eq) [1,1'-bis(diphenylphosphine)ferrocene] dichloride palladium and 100 mg (1.02 mmol, 4.0 eq) potassium acetate were added to the reaction system. Under nitrogen protection, reaction was carried out at 100 °C for 3 h with stirring. After cooling the system to room temperature, 57.3 mg (256 µmol, 1.0 eq) 2-(2-bromophenyl)-2-methyl propionitrile, 23.6 mg (20.4 µmol, 0.08 eq) tetrakis(triphenylphosphine) palladium, 70.7 mg (551 mmol, 2.0 eq) potassium carbonate and 0.4 mL water were added to the reaction system. Under nitrogen protection, reaction was carried out at 100 °C for 2 h with stirring. After confirming that the raw materials had been completely converted through LC-MS results, the temperature was reduced to room temperature. 5 mL water and 10 ml ethyl acetate were added. The organic phase was separated. The aqueous phase was extracted twice with 10 mL of ethyl acetate. The organic phases were combined, washed with salt water, dryed over anhydrous sodium sulfate, and concented to obtain a crude product. The crude product was purified to column chromatography (methanol/dichloromethane=0~1:30) and preparative chromatography (mobile phase A: 0.1% formic acid; mobile phase B: acetonitrile; gradient: 30% -70% B, 55 min; flow rate: 70mL/min) to obtain a white solid (8.70 mg, yield =7%).
LC-MS: (M+Na)⁺; m/z=478.18;
¹H NMR (400 MHz, CDCl₃) δ 8.52 - 8.35 (m, 1H), 8.11 - 7.97 (m, 1H), 7.62 - 7.48 (m, 2H), 7.48 - 7.37 (m, 2H), 7.36 - 7.26 (m, 3H), 7.20 - 7.11 (m, 1H), 5.43 - 5.26 (m, 1H), 4.24 - 4.02 (m, 2H), 4.02 - 3.81 (m, 2H), 2.69 - 2.32 (m, 2H), 2.24 - 2.02 (m, 3H), 1.79 - 1.59 (m, 6H).

### Example 165 : 2-(3-(1'-(5-fluoropyridoyl)-3-hydroxy-2,3-dihydrospiro[indene-1,3'-pyrrolidine]-5-yl)phenyl)-2-methyl propionitrile

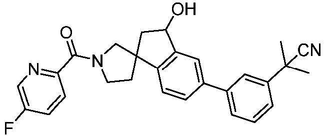

180 mg (952 µmol, 1.0 eq) (3-(2-cyanopropyl-2-yl)phenyl)boric acid was dissolved in 2mL 1,4-dioxane, and then 372 mg (952 µmol, 1.0 eq) (5-bromo-3-hydroxy-2,3-dihydrospiro[indene-1,3'-pyrrolidine]-1'-yl)(5-fluoropyridin-2-yl) ketone, 34.8 mg (0.026 mmol, 0.05 eq) [1,1'-bis(diphenylphosphine)ferrocene] palladium dichloride dichloromethane complex, 606 mg (2.85 mmol, 3.0 eq) potassium phosphate and 0.4 mL water were added to the reaction system. After repacing with nitrogen, the temperature was raised to 100 °C, and the reaction was carried out for 3 h. After confirming the complete reaction through LC-MS results, the system was cooled to room temperature, and diluted with 5mL saturated sodium chloride solution, and then extracted three times with 10 mL ethyl acetate, dried over anhydrous sodium sulfate and concentrated after filteration. The crude product was subjected to preparative chromatography (mobile phase A: 0.1% formic acid; mobile phase B: acetonitrile; gradient: 30% -70% B, 55 min; flow rate: 70mL/min), and freeze-dried to obtain a white solid (22.0 mg, yield = 5%).
LC-MS:(M+H)⁺; m/z = 456.23;
¹H NMR (400 MHz, CDCl₃) δ 8.41 (dd, *J =* 47.8, 2.8 Hz, 1H), 8.06 - 8.04 (m, 1H), 7.68 - 7.63 (m, 2H), 7.60 - 7.43 (m, 5H), 7.35 (dd, *J =* 10.3, 7.9 Hz, 1H), 5.39 - 5.32 (m, 1H), 4.08 - 4.08 (m, 1H), 3.94 - 3.93 (m, 1H), 3.92 - 3.76 (m, 2H), 2.70 - 2.53 (m, 1H), 2.46 - 2.34 (m, 1H), 2.26 - 1.99 (m, 3H), 1.78 (s, 6H).

### Inhibition rate activity testing of the compounds of the present invention

In this study, HEK-293 cells instantaneously expressing TRPV3 were used for experimental detection.

The operation steps are as follows:
The cells were cultured in DMEM medium containing 10% fetal bovine serum at a temperature of 37 °C and a carbon dioxide concentration of 5%.

Day 1: Cells were inoculated into a 6-well plate with 5 × 10⁵ cells per well.

Day 2: Transfection was performed using Lipofectamine 3000 transfection reagent, with a plasmid to transfection reagent ratio of 1 µg: 2 µL. The total amount of plasmid used per well was 3 µg. Specifically, two sterile centrifuge tubes were taken and 100 µL Opti-MEM was added to each tube. 6 µL Lipofectamine 3000 was added to one tube and mixed well; and 3 µg plasmid was added to another tube and mixed well, and then 6 µL P3000 was added and mixed well; Subsequently, the diluted plasmid DNA was addeds to diluted Lipofectamine 3000 and incubated at room temperature for 10-15 min. DNA liposome complex was droped into the cells, shaked gently and mixed well, and then cultured in an incubator. The liquid was changeed after 4 to 6 h.

Day 3: The cells were digested and seeded into 24 well plates with pre-placed cover slides, with 8 × 10³ cells per well.

Day 4: Patch clamp testing was performed.

The voltage stimulation protocol for recording TRPV3 current using whole cell patch clamp is as follows: after forming a whole cell seal, a cell membrane voltage clamp was set at -80mV. Firstly, a membrane potential was recorded at 0mV, and then a voltage was command to start from -100mV, to depolarize in the form of Ramp to 100mV within 100ms, and finally restore to 0mV. Data collection was repeated every 5 seconds to observe the inhibitory effect of pharmaceutical on peak current. Experimental data was collected by EPC 10 amplifier (HEKA) and stored in PatchMaster (HEKA) software.

A microelectrode puller was used to pull the capillary glass tube into a recording electrode. The electrode filled with intracellular fluid was inserted into a microelectrode holder, and a microelectrode manipulator was operated under an inverted microscope to immerse the electrode in extracellular fluid and an electrode resistance (Rpip) was recorded. The electrode was brought into contact with the cell surface and a negative pressure suction was applied to form a high resistance seal (GΩ). At this point, a fast capacitance compensation was performed, and negative pressure was continued to apply in order to break the cell membrane, so that a whole cell recording mode was formed. Then, a slow capacitance compensation was performed and experimental parameters such as film capacitance (Cm) and series resistance (Rs) were recorded. No leakage compensation was provided.

When the TRPV3 current recorded by the whole cell had stabilized, administration was started. Each pharmaceutical concentration acted for 5 minutes (or until the current stabilized) before detecting the next concentration. A plurality of concentrations was tested for each test compound. A cover glass covered with cells was placed in a recording bath under an inverted microscope. A blank control extracellular solution and a working fluid for the compound to be tested sequentially flowed through the recording bath from low concentration to high concentration using gravity perfusion to act on the cells. Liquid exchange was performed using a peristaltic pump during recording. The current detected by each cell in the extracellular fluid without the compound serveed as its own control group. Each concentration was independently tested twice using at least two cells. All electrophysiological tests were conducted at room temperature.

The results obtained from testing the prepared compounds using the above analysis process are shown in Table 9. The details of the inhibition rate (%) at a concentration of 0.3 µM for the selected Examples are shown in the table. Among them, "A" relates to the inhibition rate value of 70%~100%, "B" relates to the inhibition rate value within the range of 50%~69.99%, "C" relates to the inhibition rate value within the range of 25%~49.99%, "D" relates to the inhibition rate value less than 25%, and "ND" indicates that the data is undetermined.

**Table 9: Inhibition rate (%) of the compounds of the present invention on hTRPV3 at a single concentration (0.3 µM)**

| Example No. | Inhibition rate | Example No. | Inhibition rate | Example No. | Inhibition rate |
|---|---|---|---|---|---|
| 1 | D | 56 | D | 111 | ND |
| 2 | D | 57 | B | 112 | ND |
| 3 | C | 58 | A | 113 | ND |
| 4 | C | 59 | ND | 114 | ND |
| 5 | D | 60 | A | 115 | ND |
| 6 | D | 61 | A | 116 | ND |
| 7 | D | 62 | ND | 117 | ND |
| 8 | D | 63 | A | 118 | ND |
| 9 | C | 64 | ND | 119 | ND |
| 10 | C | 65 | A | 120 | D |
| 11 | A | 66 | C | 121 | ND |
| 12 | A | 67 | B | 122 | ND |
| 13 | D | 68 | ND | 123 | ND |
| 14 | A | 69 | D | 124 | ND |
| 15 | A | 70 | B | 125 | ND |
| 16 | A | 71 | B | 126 | A |
| 17 | A | 72 | ND | 127 | ND |
| 18 | A | 73 | ND | 128 | ND |
| 19 | A | 74 | D | 129 | D |
| 20 | A | 75 | C | 130 | D |
| 21 | A | 76 | B | 131 | ND |
| 22 | D | 77 | ND | 132 | ND |
| 23 | D | 78 | ND | 133 | ND |
| 24 | D | 79 | ND | 134 | D |
| 25 | A | 80 | A | 135 | D |
| 26 | D | 81 | B | 136 | ND |
| 27 | D | 82 | B | 137 | D |
| 28 | D | 83 | A | 138 | ND |
| 29 | A | 84 | C | 139 | ND |
| 30 | D | 85 | A | 140 | ND |
| 31 | D | 86 | B | 141 | ND |
| 32 | D | 87 | B | 142 | ND |
| 33 | A | 88 | A | 143 | ND |
| 34 | D | 89 | C | 144 | ND |
| 35 | D | 90 | ND | 145 | ND |
| 36 | D | 91 | D | 146 | D |
| 37 | A | 92 | A | 147 | ND |
| 38 | D | 93 | ND | 148 | ND |
| 39 | D | 94 | ND | 149 | ND |
| 40 | D | 95 | A | 150 | ND |
| 41 | A | 96 | ND | 151 | ND |
| 42 | D | 97 | ND | 152 | A |
| 43 | D | 98 | ND | 153 | A |
| 44 | D | 99 | ND | 154 | ND |
| 45 | A | 100 | ND | 155 | ND |
| 46 | D | 101 | ND | 156 | ND |
| 47 | D | 102 | ND | 157 | ND |
| 48 | A | 103 | D | 158 | ND |
| 49 | ND | 104 | A | 159 | ND |
| 50 | A | 105 | D | 160 | ND |
| 51 | A | 106 | A | 161 | ND |
| 52 | B | 107 | D | 162 | D |
| 53 | A | 108 | D | 163 | A |
| 54 | A | 109 | ND | 164 | B |
| 55 | ND | 110 | ND | | |

**Table 10: Inhibition rates of compund on different hTRPs (0.3 µ M)**

| Ion channel name | Example 21 | |
|---|---|---|
| | Inhibition rate (%) | Selectivity ratio |
| hTRPV3 | 89.64 | 1X |
| hTRPV1 | 5.62 | 16.0X |
| hTRPV4 | 29.26 | 3.1X |
| hTRPA1 | 10.65 | 8.4X |

| | | |
|---|---|---|
| Note: The selectivity ratio relates to the ratio of the inhibition rate on TRPV3 ion channels to the inhibition rate on other ion channels at the same test concentration of a compound. | | |

### Liver microsome metabolism stability test

Two separate experiments were conducted. a) NADPH: 10 µL of 20 mg/mL liver microsomes and 40 µL of 10mM NADPH were added to a culture medium. The final concentrations of microsomes and NADPH were 0.5 mg/mL and 1 mM, respectively. b) NADPH free: 10 µL of 20 mg/mL liver microsomes and 40 µL of ultrapure H₂O were added to a culture medium. The final concentration of microsomes was 0.5 mg/mL.

At the beginning of the reaction, 4 µL of 100 µM test compound solution or a control compound solution with a final concentration of 1 µM were added, and proceed at 37 °C.

50 µL aliquots were taken from the reaction solution at 0, 7, 15, 30, and 60 minutes. The reaction was stopped by adding 4 volumes of cold acetonitrile and IS (100 nM alprazolam, 200 nM labetalol, 200 nM caffeine, and 2 µM ketoprofen). The samples were centrifuged at 3220 grams for 40 minutes. 100 µL supernatant divided equally was mixed with 100 µL of ultrapure H₂O, and use for LC-MS/MS analysis.

**Table 11: Metabolic stability test results of liver microsomes**

| Compound No. | Genus | *in vitro* T_{1/2} (min) | *in vitro* Clᵢₙₜ (microliters/minute/milligrams of protein) |
|---|---|---|---|
| Example 15 | human | 19.69 | 70.41 |
| | rat | 25.82 | 53.68 |

### Balanced dialysis method for testing plasma protein binding

1. Immersing a dialysis membrane (water: 60 minutes, 20% ethanol: 20 minutes, dialysis buffer: 20 minutes). 2. Diluting the test compound in dimethyl sulfoxide (working solution) to 200 µM. 3. Thawing the plasma in a 37 °C water bath and centrifuge the plasma at 3220 g for 10 minutes to remove blood clots. 4. Transfering the supernatant to a new tube and preheating it in a 37 °C water bath for 10 minutes. 5. Assemblimng HTD dialysis equipment according to the manufacturer's guidelines. 6. Adding 3 µL of 200 µM test compound to 597 µL of plasma and rotating at 1000 rpm for 2 minutes. 7. Transfering 50 µL of spiked plasma to a 96 well plate, then adding 50 µL of dialysis buffer and 200 µL of methanol, using IS * as the T0 sample. 8. Adding 120 µL of spiked plasma and 120 µL of dialysis buffer in duplicate into the chamber of the HTD dialysis device. 9. Covering the dialysis equipment with a breathable cover and incubating the dialysis equipment and the remaining spiked plasma at 37 °C (100 rpm, 5% CO₂) for 6 hours. 10. Transfering 50 µL of dialyzed sample from the dialysis buffer and the plasma chamber to a separate 96 well plate, then adding 50 µL of blank plasma or dialysis buffer and 200 µL of methanol, with IS * as the B and P samples. 11. Transfering 50 µL of remaining spiked plasma (as described in step 9) to a 96 well plate, then adding 50 µL of dialysis buffer and 200 µL of methanol, with IS * as the T6 sample. 12. Centrifuging the sample plate at 3220g for 40 minutes. 13. Transfering 100 µL of supernatant onto an analytical plate containing an appropriate volume of water for LC-MS/MS. 14. Data analysis.

**Table 12: The results of the plasma protein binding test of the compound of the present invention**

| **Compound No.** | **Genus** | **dissociated %** | **binding %** | **remaining %** | **recover %** |
|---|---|---|---|---|---|
| Example 15 | rat | 0.22 | 99.78 | 102.46 | 92.94 |
| | human | 0.42 | 99.58 | 97.37 | 87.63 |

### Parallel artificial membrane permeability test (lipid PAMPA) experiment

1. Preparing the test compound in 10 mM DMSO and diluting 1000 fold to 10 µM with PBS. 2. Ultrasonic treating lecithin in dodecane at a rate of 18 mg/ml. 3. Adding 300 µL/well of 10 µM test compound solution into the donor chamber (bottom) in triplicate. 4. Adding 5 µL/well lecithin/dodecane to the receptor chamber (top), and then adding 300 µL/well PBS (pH=7.4) to the receptor chamber within 10 minutes. 5. Transfering 50 µL of 10 µM test compound onto a sample plate containing 200 µL of cold methanol, with IS * being C0. 6. Inserting the receptor chamber into the donor chamber and incubating at 25 °C for 16 hours. 7. After 16 hours of incubation, transfering 50 µL from the receptor and donor compartments to a sample plate containing 200 µL of cold methanol and IS *. 8. Centrifuging the sample plate at 4 °C and 3220g for 40 minutes. 9. Transfering 100 µL of supernatant to an analysis plate containing an appropriate volume of H₂O for LC-MS/MS analysis. 10. Data analysis.

**Table 13: The permeability test results of the compound of the present invention for parallel artificial membranes**

| **Compound No.** | **Test concentration (µM)** | **Effective penetration rate Pex10⁻⁶ (cm/s)** | **-LOG Pe** | **rate of recovery %** |
|---|---|---|---|---|
| Example 15 | 10 | 14.06 | 4.85 | 16.92 |
| Example 21 | 10 | 16.26 | 4.79 | 18.82 |

### Permeability experiment of Caco-2 cells

### Cell plate preparation:

1. Transwell plate preheating: before laying cells, adding 100 µL of culture medium to the upper layer of Transwell plate and 600 µL of culture medium to the lower layer of Transwell plate, Pre-incubating at 37 °C under 5% CO₂ for 1 hour. 2. Treating Caco-2 cells by adding 100 µL of cell suspension (4 x 105 cells/mL) to each well, and cultivating in a 37 °C, 5% CO₂ incubator for 14-21 days. 3. Changing the cell culture medium every other day within 7 days, and changing it daily after 7 days. 4. Using EVOM3 to measure single-layer epithelial to epithelial resistance (TEER).

### Experimental procedure:

1. Preheating HBSS (10mM HEPES, pH 7.4) buffer, cleaning the upper and lower layers of Transwell plates twice each, and incubating at 37 °C for 30 minutes. 2. Working solution: Diluting the test compound HBSS buffer with 1mM DMSO to 5 µM. 3. A-B direction: Adding 200 µL of 5 µM working solution to the upper layer and 600 µL of HBSS buffer solution to the lower layer; 4. B-A direction: Adding 600 µL of 5 µM working solution to the lower layer and 200 µL of HBSS buffer to the upper layer, and incubating for 2 hours. 5. C0 sample: Taking 100 µL of working solution to a sample plate containing 400 µL of methanol (internal standard). 6. After incubation for 2 hours, taking 100 µL from the upper and lower layers of the chamber to the sample plates containing 400 µL of methanol (internal standard). 7. Mixing and centrifuging, mixing the supernatant with a certain amount of water, and using the sample for LC-MS/MS data analysis. 8. After completing step 6, adding 200 µL of 100 µM fluorescent yellow solution to the upper chamber and 600 µL of HBSS buffer salt solution to the lower chamber. Incubating at 37 °C and 5% CO₂ for 30 minutes, and taking out 50 µL of each into a black plate to read the fluorescence value. 9. Data analysis.

**Table14: The permeability test results of the compound of the present invention for caco-2 cells**

| **Compound No.** | **Experimental concentration (µM)** | **P_{app A→B} (1E-6 cm/s)** | **P_{app B→A} (1E-6 cm/s)** | **External ratio** | **Recover A→B%** | **Recover B→A%** |
|---|---|---|---|---|---|---|
| Example 21 | 5 | 20.93 | 9.58 | 0.46 | 49.40 | 68.20 |

### Pharmacokinetic experiments

Male SD rats were grouped in groups of 3 rats each and orally administered with the compound of Example (10mg/kg) and intravenously injected with the compound of Example (2mg/kg). Animals were fasted overnight before the experiment, from 10 hours before administration to 4 hours after administration. Blood samples were collected at 0.25, 0.5, 1, 2, 4, 8, and 24 hours after administration in the oral group, and at 0.083, 0.25, 0.5, 1, 2, 4, 8, and 24 hours after injection in the intravenous group. After anesthesia with isoflurane using a small animal anesthesia machine, 0.3 mL of whole blood was collected through the retinal venous plexus, and placed in a heparin anticoagulant tube. The sample was centrifuged at 4 °C and 4000rpm for 5 minutes, and the plasma was transferred to the centrifuge tube and stored at -80 °C until analysis. The plasma samples were extracted using protein precipitation method, and the extract was analyzed by LC-MS/MS.

**Table 15: Pharmacokinetic parameters of different compounds administered by gavage to rats at a dose of 10 mg/kg**

| Compound No. | Example 15 | KM-001 |
|---|---|---|
| T_{1/2} (hr) | 1.88 | 3.29 |
| Tmax (hr) | 1.33 | 1.00 |
| Cmax (ng/mL) | 8053 | 1573 |
| AUC_{0-inf} (hr*ng/mL) | 42175 | 6163 |
| F(%) | 80.6 | 68.2 |

**Table 16: Pharmacokinetic parameters of 2mg/kg compound administered intravenously to rats**

| Compound No. | Example 15 | KM-001 |
|---|---|---|
| T_{1/2} (hr) | 1.63 | 6.54 |
| C0 (ng/mL) | 4060 | 1480 |
| Vss (L/kg) | 0.41 | 2.79 |
| Cl (mL/min/kg) | 3.3 | 18.5 |
| AUC0-inf (hr*ng/mL) | 10465 | 1808 |

From the above results, it can be seen that the injection and oral pharmacokinetic properties of the compound in Example of the present invention are superior to the existing compound KM-001 (compounds in Examples 2-72 in patent WO2021154966A1, i.e., Example KM-001), and the oral bioavailability for Example reaches 80.6%. The pharmacokinetic properties of the compounds in other Example of the present invention were tested according to the same method, and the pharmacokinetic performances obtained are also superior to KM-001.

### Stability assessment of compounds

Taking the compound of Example 21 and KM-001-E1 in WO2021154966A1 as examples, the stability thereof was compared:
Compound of Example 21 and KM-001-E1 were accurately weighed and placed in penicillin bottles, and placed at room temperature under 92.5% humidity (open) conditions for 5-10 days to investigate the stability of the compounds.

### Analysis conditions:

instrument: Thermo U3000-ISQEC
column: Xtimate UHPLC C18 1.8µm 4.6*50mm
Column temperature: 35°C
wavelength: 210nm, 254nm
mobile phase A: 0.05% formic acid aqueous solution; mobile phase B: 0.05% formic acid acetonitrile; gradient: 30%B 0-1.2 min, 95%B 1.2-3.3min, 30%B 3.3-4.5min;

Analyzing the degradation impurities of each compound under 92.5% humidity conditions using LC-MS: The main impurities in Example 21 had m/z 427.17, with a retention time of 2.55 minutes. After being left for 10 days, the peak area proportion was only 0.3%; The main impurities in KM-001-E1 has m/z=431.14 and m/z=415.16, with retention times of 2.31 minutes and 2.64 minutes, respectively. After being left for 5 days, the peak area proportions reached 2.48% and 2.43%, respectively. Based on LC-MS and NMR data analysis, it can be inferred that the main impurities produced in Example 21 are ketones, while the main impurities produced in KM-001-E1 are aldehydes and carboxylic acids. The specific structure is as follows:

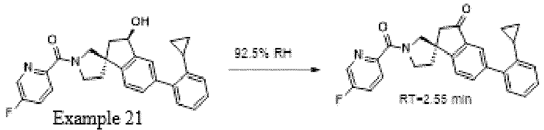

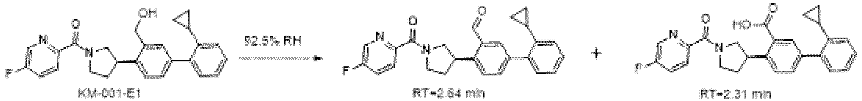

From the above results, it can be seen that the stability of the compound in Example of the present invention is superior to that of the existing compound KM-001-E1.

The compound 21 mentioned above has a secondary alcohol structure, which has better chemical stability than the primary alcohol compound in WO2021154966A1: as alcohols, the former can only be oxidized to ketones; the latter can be oxidized to aldehydes and further oxidized to carboxylic acids, with a higher quantity and level of oxidation impurities than the compound 21 mentioned in the present invention.

Similarly, based on the same structural features, the stability of the compounds in Examples of the present invention with the same secondary alcohol structure is superior to that of the compounds in WO2021154966A1.

Although preferred embodiments of the present invention have been disclosed to illustrate the present invention, those skilled in the art should understand that various modifications, additions, and substitutions can be made to the present invention without departing from the inventive concept and scope as defined in claims.

## Claims

1. A nitrogen-containing spirocyclic compound of formula (I), or stereoisomers, tautomers, solvates, hydrates, oxides, active metabolites, isotopic labels, and pharmaceutically acceptable salts thereof: 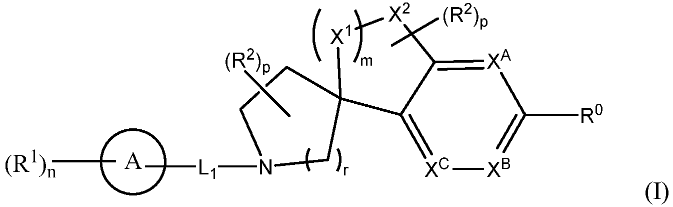
wherein ring A is selected from monocyclic or polycyclic ring systems containing 3-12 ring atoms;
R¹ is each independently selected from the group consisting of H, halogen, hydroxyl, mercapto, nitro, cyano, oxo, C₁-C₆ alkyl optionally substituted with 1 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, - C(O)N(R^{a})(R^{b}) and -SO₂N(R^{a})(R^{b}), and -SOR^{c}, or two R¹, together with the atoms of ring A to which they are connected, form a 3-10 membered ring structure;
R² is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}) and -SO₂N(R^{a})(R^{b}), and -SOR^{c}, or two R², together with the atoms of the ring to which they are connected, form a 3-10 membered ring structure;
L₁ is selected from the group consisting of a bond, and the following structural formulae: 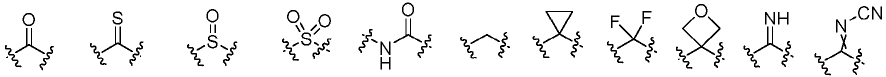 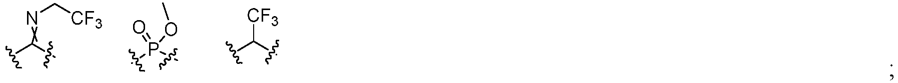
X¹ is each independently selected from the group consisting of C, O, and N at each occurrence;
X² is each independently selected from the group consisting of C, O, B, and N at each occurrence;
X^{A}, X^{B}, X^{C} are each independently CR^{x} or N;
R^{×} is each independently selected from the group consisting of H, halogen, hydroxyl, mercapto, nitro, cyano, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}) and -SO₂N(R^{a})(R^{b}), and -SOR^{c}, or two R^{x}, together with the atoms of the ring to which they are connected, form a 3-10 membered ring structure;
R⁰ is each independently selected from the group consisting of H, halogen or a structural formula of formula (II) 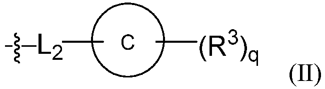
wherein,
L₂ is each independently selected from the group consisting of a bond, -O-, -S-, -N(R²⁰)-, - C(O)-, -C(R²⁰R²¹)-, -S(O)-, and -S(O₂)-;
ring C is each independently selected from monocyclic or polycyclic ring systems containing 3-12 ring atoms;
R³ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}) and -SO₂N(R^{a})(R^{b}), and -SOR^{c}, or two R³, together with the atoms of ring C to which they are connected, form a 3-10 membered ring structure;
R¹¹ is each independently selected from C₁-C₆ alkylene substituted with 0 to 2 R^{f};
R¹² is each independently selected from the group consisting of H, C₁-C₆ alkyl substituted with 0 to 2 R^{f}, C₁-C₆ haloalkyl substituted with 0 to 2 R^{f}, C₃-C₆ cycloalkyl substituted with 0 to 2 R^{f}, and C₃-C₆ halocycloalkyl substituted with 0 to 2 R^{f};
R^{a} and R^{b} are each independently selected from the group consisting of H, C₁-C₆ alkyl substituted with 0 to 2 R^{f}, aryl substituted with 0 to 2 R^{f}, aralkyl substituted with 0 to 2 R^{f}, - C(O)R^{c}, and -C(O)OR^{d};
R^{c} is each independently selected from the group consisting of H, halogen, C₁-C₆ alkyl substituted with 0 to 2 R^{f}, aryl substituted with 0 to 2 R^{f}, and aralkyl substituted with 0 to 2 R^{f},
R^{d} is each independently selected from the group consisting of H, C₁-C₆ alkyl substituted with 0 to 2 R^{f}, aryl substituted with 0 to 2 R^{f}, and aralkyl substituted with 0 to 2 R^{f},
R²⁰ and R²¹ are each independently selected from the group consisting of H, hydroxyl, C₁-C₆ alkyl, aryl, and aralkyl;
R^{f} is each independently selected from the group consisting of halogen, hydroxyl, amino, C₁-C₆ alkyl, C₁-C₆ alkoxy, C₁-C₆ haloalkyl, C₁-C₆ haloalkoxy, C₃-C₆ cycloalkyl, and C₃-C₆ halocycloalkyl;
n is 0, 1, 2 or 3;
p is 0, 1, 2 or 3;
q is 0, 1, 2 or 3;
m is 1, or 2;
r is 1, or 2.

2. The nitrogen-containing spirocyclic compound according to claim 1, wherein ring A is selected from the group consisting of benzene ring, pyridine ring, quinoline ring, piperidine ring, C₃-C₆ cycloalkyl ring, isoquinoline ring, pyrazine ring, pyrimidine ring, pyridazine ring, thiazole ring, thiophene ring, pyrrole ring, pyrazole ring, imidazole ring, isothiazole ring, indole ring, benzimidazole ring, furan ring, oxazole ring, oxadiazole ring, quinoxaline ring, and purine ring.

3. The nitrogen-containing spirocyclic compound according to claim 1, wherein ring A is selected from the following structural formulae:

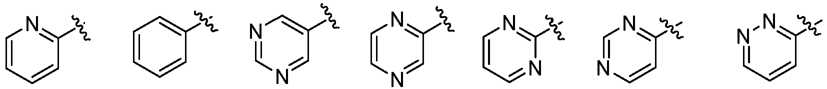

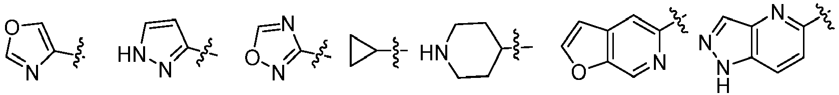

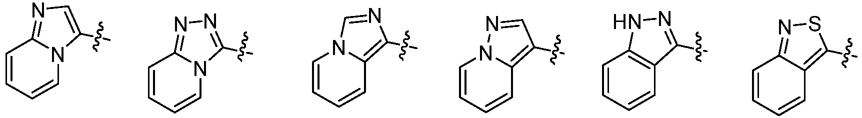

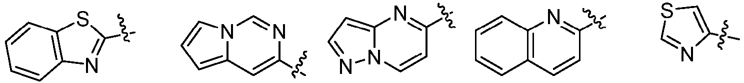

4. The nitrogen-containing spirocyclic compound according to claim 1, wherein R¹ is each independently selected from the group consisting of H, halogen, cyano, hydroxyl, C₁-C₆ alkyl, C₁-C₆ haloalkyl, C₁-C₆ alkoxy, -N(R^{a})(R^{b}) and -R¹¹OR¹², wherein R^{a} and R^{b} are each independently selected from the group consisting of H and C₁-C₆ alkyl; R¹¹ is each independently selected from C₁-C₆ alkylene; R¹² is each independently selected from the group consisting of H, and C₁-C₆ alkyl.

5. The nitrogen-containing spirocyclic compound according to claim 4, wherein R¹ is each independently selected from the group consisting of H, Cl, F, -CF₃, -CN, -CH₃, -OH, -OCH₃, and -CH₂OCH₃.

6. The nitrogen-containing spirocyclic compound according to claim 1, wherein R² is each independently selected from the group consisting of H, halogen, cyano, hydroxyl, mercapto, oxo, C₁-C₆ alkyl, C₁-C₆ haloalkyl, C₃-C₆ cycloalkyl substituted with 0 to 2 R^{f}, C₁-C₆ alkoxy, C₁-C₆ haloalkoxy, -R¹¹OR¹², -R¹¹SR¹², -CH(O), -C(O)OR^{d} and -C(O)N(R^{a})(R^{b}); wherein R¹¹ is each independently selected from C₁-C₆ alkylene; R¹² is each independently selected from the group consisting of H, C₁-C₆ alkyl, C₁-C₆ haloalkyl, C₃-C₆ cycloalkyl, and C₃-C₆ halocycloalkyl; R^{a} and R^{b} are each independently selected from the group consisting of H and C₁-C₆ alkyl; R^{d} is each independently selected from the group consisting of H and C₁-C₆ alkyl; R^{f} is each independently selected from the group consisting of halogen, hydroxyl, amino, and C₁-C₆ alkyl.

7. The nitrogen-containing spirocyclic compound according to claim 6, wherein R² is each independently selected from the group consisting of protium, deuterium, tritium, halogen, -SH, -OH, -OCF₃, -CH₃, -OCH₃, -CF₃, -NH₂, -CN, -CONH₂, -CH₂OH, -CH(O), -CHF₂, -COOH, - COOCH₃, oxo, and

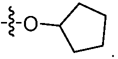

8. The nitrogen-containing spirocyclic compound according to claim 1, wherein L₂ is each independently selected from the group consisting of a bond, -O-, -S-, -N-, -C(O)-, -CH₂-, -CF₂-, -C(OH)-, -S(O)-, and -S(O₂)-.

9. The nitrogen-containing spirocyclic compound according to claim 1, wherein ring C is selected from the group consisting of C₃-C₆ cycloalkane ring, benzene ring, benzo-C₃-C₆ cycloalkane rings, C₅-C₁₂ bridged carbocyclic ring, pyridine ring, quinoline ring, isoquinoline ring, pyrazine ring, pyrimidine ring, pyridazine ring, thiazole ring, thiophene ring, pyrrole ring, pyrazole ring, imidazole ring, isothiazole ring, indole ring, benzimidazole ring, furan ring, oxazole ring, quinoxaline ring, and purine ring.

10. The nitrogen-containing spirocyclic compound according to claim 9, wherein ring C is selected from the following structural formulae:

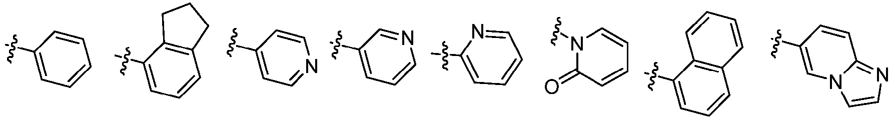

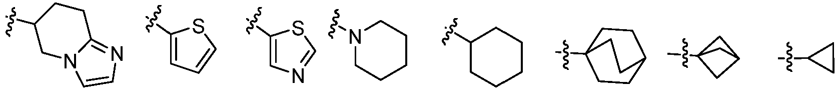

11. The nitrogen-containing spirocyclic compound according to claim 1, wherein R³ is each independently selected from the group consisting of H, cyano, hydroxyl, halogen, C₁-C₆ alkyl, C₁-C₆ haloalkyl, C₁-C₆ alkoxy, C₁-C₆ haloalkoxy, C₃-C₆ cycloalkyl, -R¹¹OR¹², -R¹¹SR¹², - C(O)R^{c}, -C(O)OR^{d}, and -C(O)N(R^{a})(R^{b}), or two R³, together with the atoms of ring C to which they are connected, form a 3-10 membered ring structure;
wherein R¹¹ is each independently selected from C₁-C₆ alkylene; R¹² is each independently selected from the group consisting of H, C₁-C₆ alkyl, C₁-C₆ haloalkyl, C₃-C₆ cycloalkyl, and C₃-C₆ halocycloalkyl; R^{a} and R^{b} are each independently selected from the group consisting of H and C₁-C₆ alkyl; R^{c} is independently selected from the group consisting of H, halogen, and C₁-C₆ alkyl; R^{d} is each independently selected from the group consisting of H and C₁-C₆ alkyl.

12. The nitrogen-containing spirocyclic compound according to claim 11, wherein R³ is each independently selected from the group consisting of H, cyclopropyl, isopropyl, tert-butyl, F, Cl, CN, ethyl, methyl, trifluoromethoxy, methylcarbonyl, methoxymethyl, and -C(CH₃)₂OH.

13. The nitrogen-containing spirocyclic compound according to claim 1, wherein 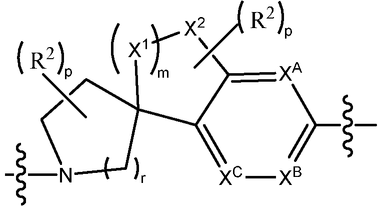 in formula (I) is selected from one of the following structural formulae: 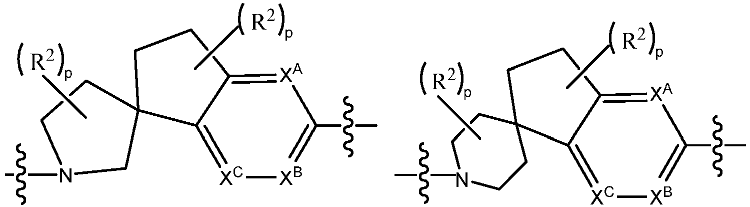 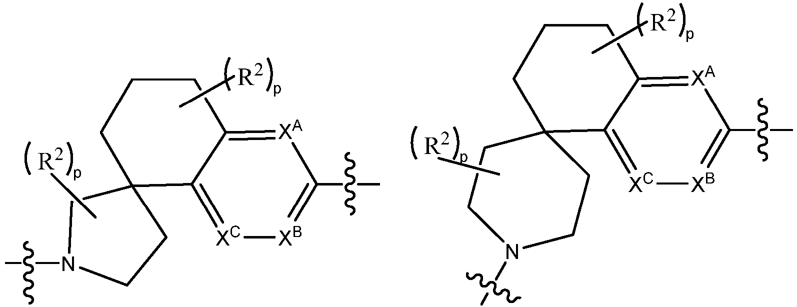 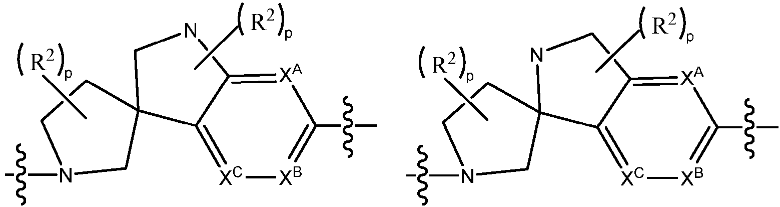 or, 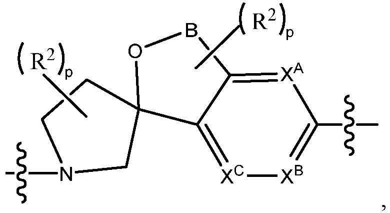 wherein R², X^{A}, X^{B}, X^{C}, and p are defined as in formula (I).

14. The nitrogen-containing spirocyclic compound according to claim 1, wherein 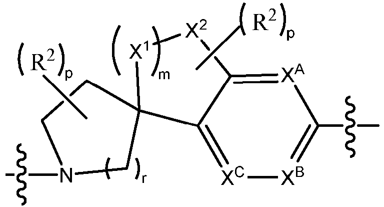 in formula (I) is selected from one of the following structural formulae: 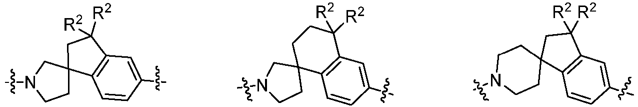 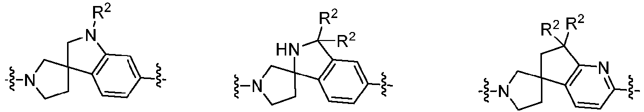 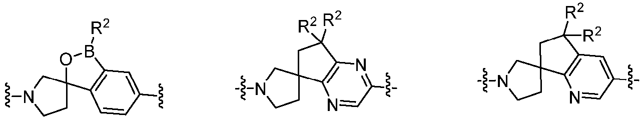 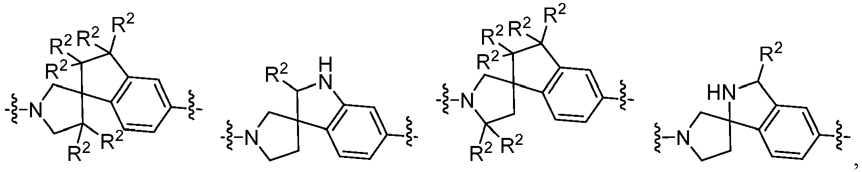 wherein R² is each independently selected from the group consisting of protium, deuterium, tritium, halogen, oxo, -SH, -OH, -CH₃, -CN, -CONH₂, -COOH, -COH, -COOCH₃, -CF₃, -OCH₃, -CH₂OH, -OCF₃, -CHF₂, and-NH₂.

15. The nitrogen-containing spirocyclic compound according to claim 14, wherein at least one R² is - OH.

16. The nitrogen-containing spirocyclic compound according to claim 14, wherein 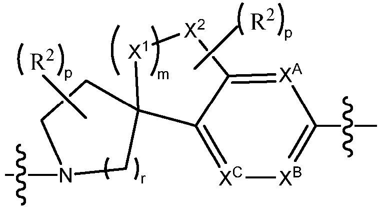 in formula (I) is selected from one of the following structural formulae: 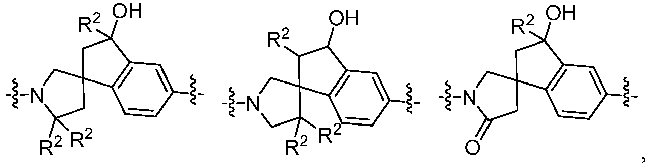 wherein R² is each independently selected from the group consisting of protium, deuterium, tritium, halogen, -CH₃ and -CF₃.

17. The nitrogen-containing spirocyclic compound according to claim 1, wherein 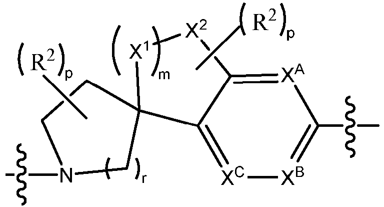 in formula (I) is selected from one of the following structural formula: 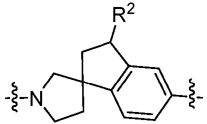 wherein R² is independently selected from the group consisting of oxo, -SH, -CN, -CONH₂, -COOH, -COH, -COOCH₃, -OCH₃, -CH₂OH, -CHF₂, -OCF₃ and -NH₂.

18. The nitrogen-containing spirocyclic compound according to claim 1, wherein 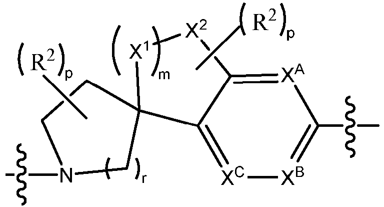 in formula (I) is selected from one of the following structural formulae:

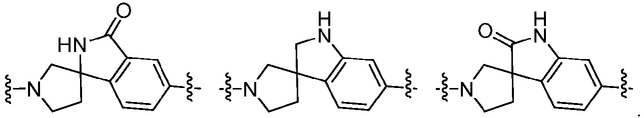

19. The nitrogen-containing spirocyclic compound according to claim 1, wherein L₁ is

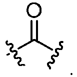

20. The nitrogen-containing spirocyclic compound according to claim 1, wherein ring A is 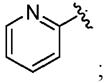 or
ring A is 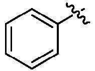 or
ring A is

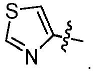

21. The nitrogen-containing spirocyclic compound according to claim 1, wherein the compound is selected from the following compounds:

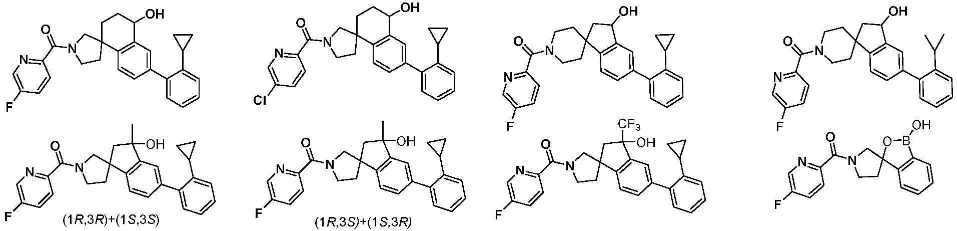

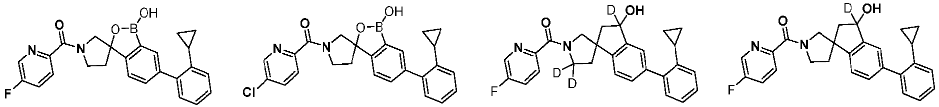

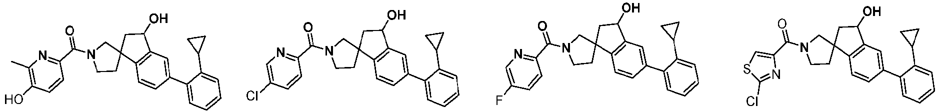

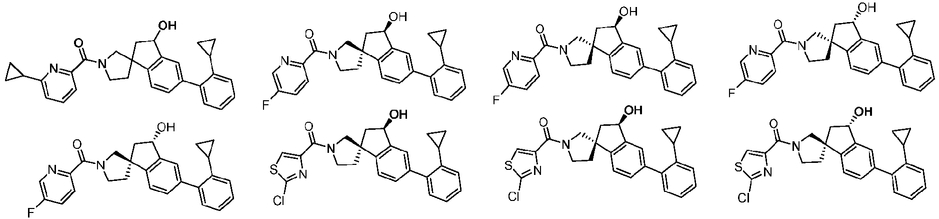

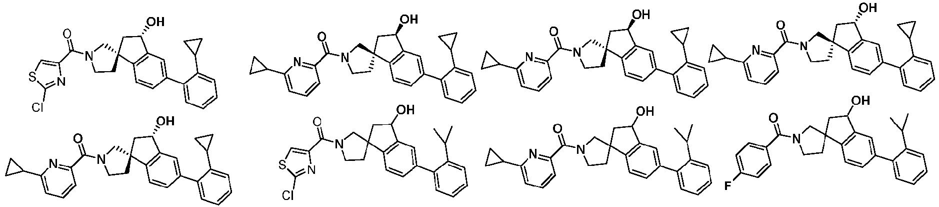

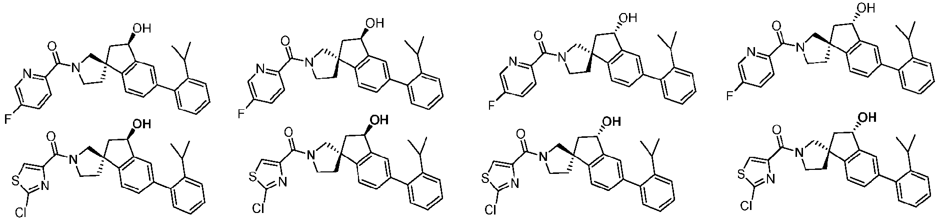

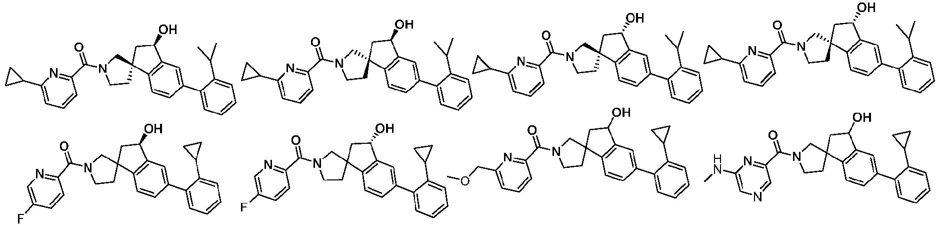

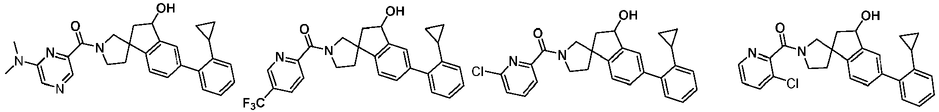

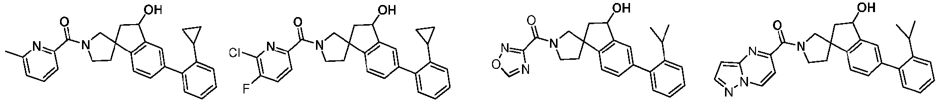

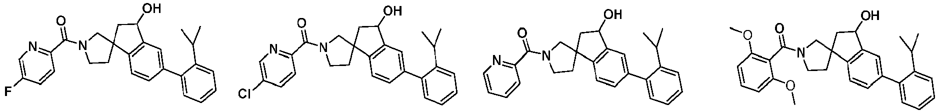

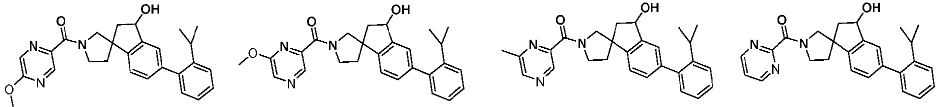

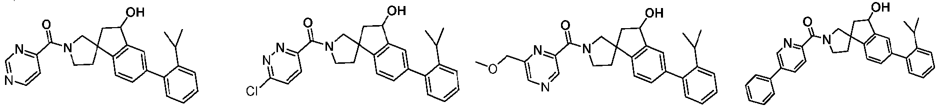

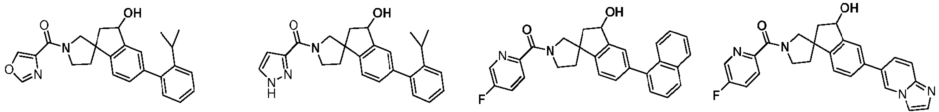

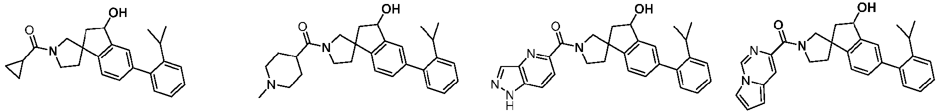

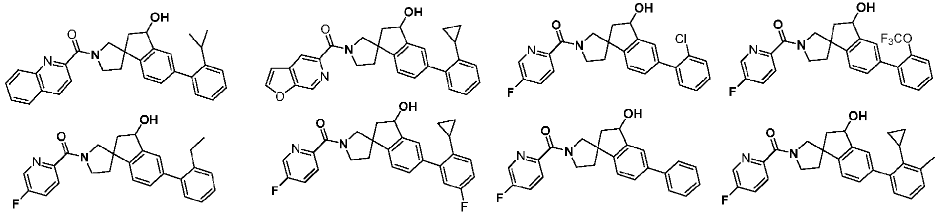

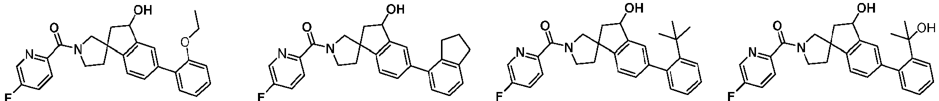

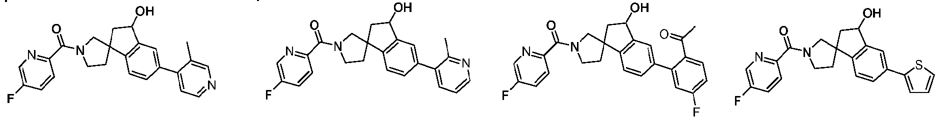

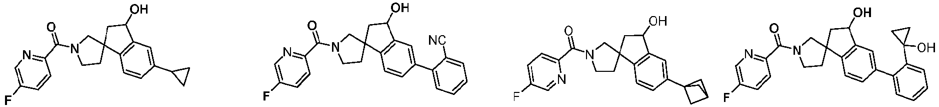

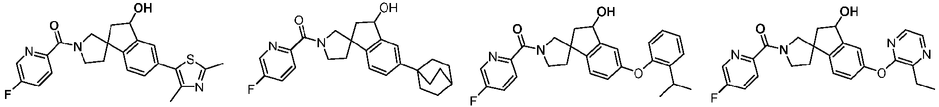

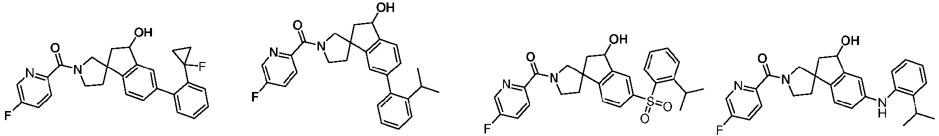

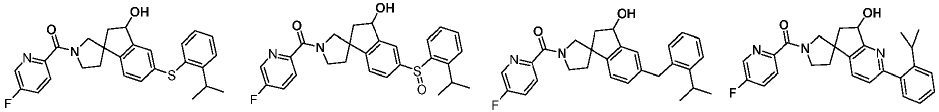

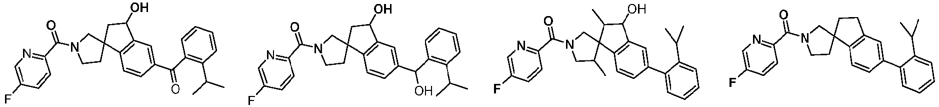

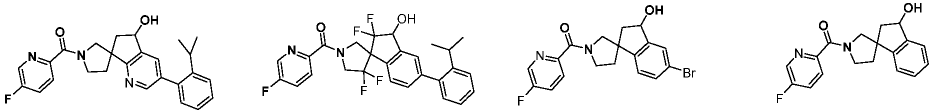

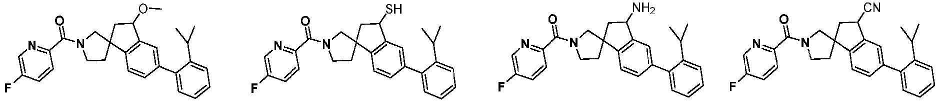

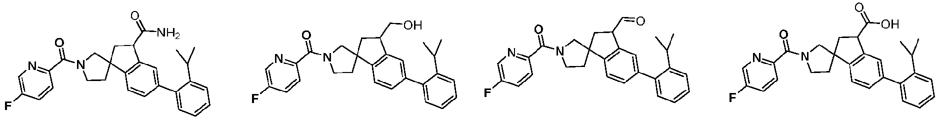

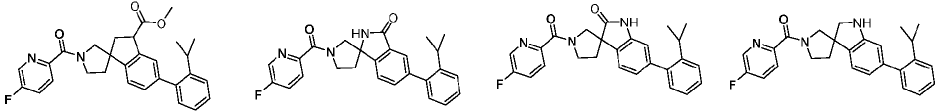

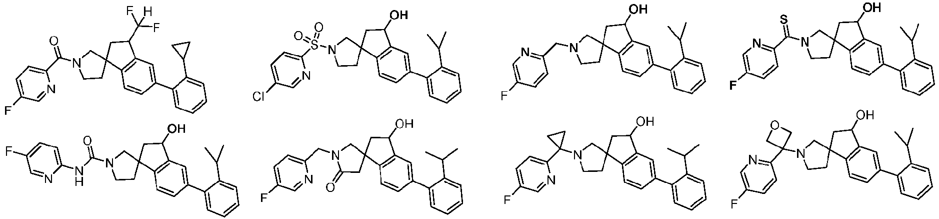

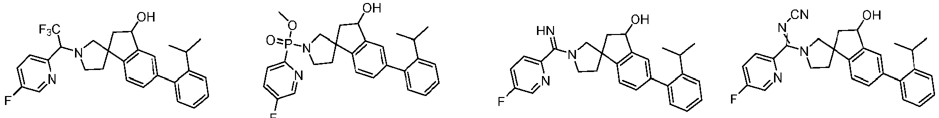

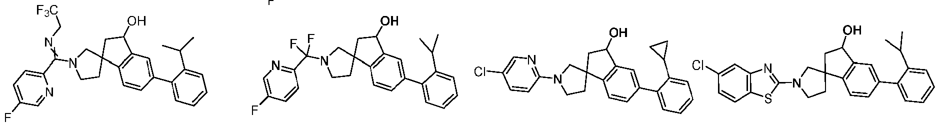

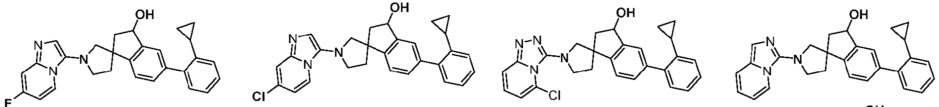

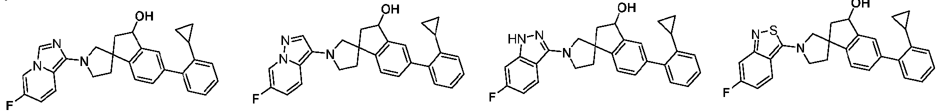

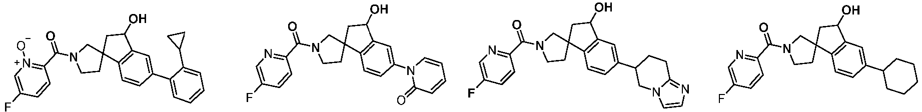

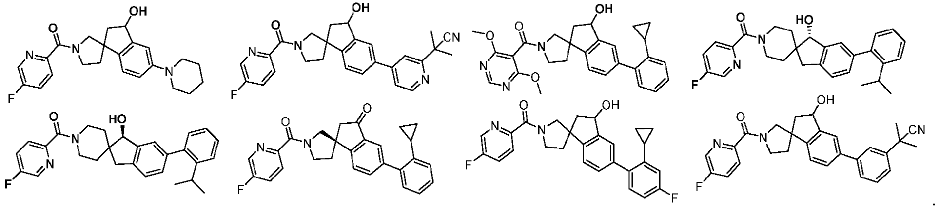

22. A pharmaceutical composition, comprising the compound according to any one of claims 1 to 21, or stereoisomers, tautomers, solvates, hydrates, active metabolites, isotopic labels, or pharmaceutically acceptable salts thereof, and a pharmaceutically acceptable carrier.

23. Use of the compound according to any one of claims 1 to 21, or stereoisomers, tautomers, solvates, hydrates, active metabolites, isotopic labels, or pharmaceutically acceptable salts thereof, and the pharmaceutical composition according to claim 22 in manufacture of pharmaceuticals for inhibiting TRPV3 activity.

24. Use of the compound according to any one of claims 1 to 21, or stereoisomers, tautomers, solvates, hydrates, active metabolites, isotopic labels, or pharmaceutically acceptable salts thereof, and the pharmaceutical composition according to claim 22 in manufacture of pharmaceuticals for treating TRPV3-mediated conditions in a subject.

25. Use according to claim 24, wherein the conditions are selected from the group consisting of pain, itching, skin conditions, inflammation, abnormal hair growth, incontinence, fever, hot flashes, cystitis, irritable bowel syndrome, and/or cough symptoms.

26. Use according to claim 24, wherein the pain is cancerous pain and skin pain.

27. Use according to claim 26, wherein used for manufacturing pharmaceuticals that inhibit proliferation, and thereby prevent, treat, or alleviat cancer symptoms.

28. Use according to claim 27, wherein the cancer is liposarcoma.

29. Use according to claim 25, wherein the abnormal hair growth is hair loss.

30. Use according to claim 25, wherein the skin conditions are selected from the group consisting of skin keratosis, ichthyosis, and pruritus.

31. Use according to claim 30, wherein the skin keratosis is olmsted syndrome.

32. Use according to claim 31, wherein the ichthyosis is harlequin ichtyosis.

33. A method for treating TRPV3 mediated conditions, comprising administering a therapeutically effective amount of the compound according to any one of claims 1 to 21, or stereoisomers, tautomers, solvates, hydrates, active metabolites, isotopic labels, or pharmaceutically acceptable salts thereof, or the pharmaceutical composition according to claim 22 to a patient in need of administration.
